# EUROPEAN PATENT SPECIFICATION

(11) **EP 1 173 610 B1**
(45) Date of publication and mention of the grant of the patent: **25.08.2010**
(21) Application number: 00914181.3
(22) Date of filing: 05.04.2000
(51) Int. Cl.: C12Q 1/68

(54) **METHOD FOR THE AMPLIFICATION OF HLA CLASS I ALLELES**
METHODE ZUR AMPLIFIZIERUNG VON HLA KLASSE I ALLELEN
TECHNIQUE D'AMPLIFICATION DES ALLELES HLA DE CLASSE I

(30) Priority: 09.04.1999 EP 99870068; 11.06.1999 US 138614 P
(43) Date of publication of application: 23.01.2002
(73) Proprietor: Innogenetics N.V., 9052 Ghent (BE)
(72) Inventor: DE CANCK, Ilse, 2020 Antwerpen (BE); ROMBOUT, Annelies, 9080 Beervelde (BE); ROSSAU, Rudi, 2180 Ekeren (BE)
(86) International application number: PCT/EP2000/002998
(87) International publication number: WO 2000/061795

(56) References cited:
- EP-A- 0 414 469
- WO-A-92/19771
- WO-A-97/20197
- WO-A-97/23645
- WO-A-97/23650
- WO-A-98/26091
- WO-A-99/07883
- WO-A-99/19509
- CEREB N ET AL: "NUCLEOTIDE SEQUENCES OF MHC CLASS I INTRONS 1,2, AND 3 IN HUMANS AND INTRON 2 IN NONHUMAN PRIMATES" TISSUE ANTIGENS,DK,MUNKSGAARD, COPENHAGEN, vol. 47, no. 6, page 498-511,ERRATUM XP002070446 ISSN: 0001-2815
- KOTSCH K ET AL: "SEQUENCING OF HLA CLASS I GENES BASED ON THE CONSERVED DIVERSITY OF THE NONCODING REGIONS: SEQUENCING-BASED TYPING OF THE HLA-A GENE" TISSUE ANTIGENS,DK,MUNKSGAARD, COPENHAGEN, vol. 50, no. 2, page 178-191 XP002070449 ISSN: 0001-2815
- BLASCZYK R ET AL: "SEQUENCE ANALYSIS OF THE 2ND INTRON REVEALED COMMON SEQUENCE MOTIFS PROVIDING THE MEANS FOR A UNIQUE SEQUENCING BASED TYPING PROTOCOL OF THE HLA-A LOCUS" TISSUE ANTIGENS,DK,MUNKSGAARD, COPENHAGEN, vol. 47, no. 2, page 102-110 XP000573588 ISSN: 0001-2815
- DELFINO L ET AL: "HLA-C high resolution typing: Analysis of exons 2 and 3 by sequence based typing and detection of polymorphisms in exons 1-5 by sequence specific primers." TISSUE ANTIGENS, vol. 52, no. 3, 1998, pages 251-259, XP000971020 ISSN: 0001-2815
- CEREB N ET AL: "LOCUS-SPECIFIC CONSERVATION OF THE HLA CLASS I INTRONS BY INTRA- LOCUS HOMOGENIZATION" IMMUNOGENETICS,DE,SPRINGER VERLAG, BERLIN, vol. 47, no. 1, page 30-36 XP002070447 ISSN: 0093-7711

## Description

### FIELD OF THE INVENTION

The present invention relates to a method for the typing or subtyping of HLA-A. More specifically, the present invention relates to a method for the locus-specific, separate amplification of exon 2, exon 3 and exon 4 of HLA-A.

### BACKGROUND OF THE INVENTION

The human major histocompatibility complex (MHC) is contained within about 4 Mbp of DNA on the short arm of chromosome 6 at 6p21.3 (Campbell and Trowsdale, 1993). The human MHC is divided into class I, class II and class III regions. The genes of class I and class II encode highly polymorphic cell-surface molecules that bind and present processed antigens in the form of peptides to T-lymphocytes, initiating both cellular and humoral immune responses.

The class I molecules of the human MHC, HLA-A, -B, and -C, are found on most nucleated cells. They are cell-surface glycoproteins that bind and present processed peptides derived from endogenously synthesized proteins to CD8+ T-cells. These heterodimers consist of an HLA-encoded α-chain associated with a non-MHC encoded monomorphic polypeptide, β-microglobulin (Townsend and Bodmer, 1989; Spencer and Parham, 1996). The class II molecules of the human MHC are encoded in the HLA-D region. These cell-surface glycoproteins consist of HLA-encoded α-, and β-chain, associated as heterodimers on the cell surface of antigen-presenting cells such as B-cells and macrophages. Class II molecules serve as receptors for processed peptides. However, these peptides are derived predominantly from membrane and extracellular proteins and are presented to CD4+ T-cells. The HLA-D region contains several class II genes and has three main subregions: HLA-DR, -DQ, and -DP. Both the HLA-DQ and -DP regions contain one functional gene for each of their α- and β-chains. The HLA-DR subregion contains one functional gene for the α-chain; the number of functional genes for the β-chain varies from one to two according to the haplotype (Andersson et al., 1987: Apple and Erlich, 1996).

Extensive polymorphism exists at most loci. In view of the biological and medical importance of these antigens, a highly sensitive and rapid technique for HLA typing is required. A variety of techniques are currently used to detect HLA polymorphism, including serological, biochemical, T-cell recognition and, most recently, molecular biological methods.

Serology remains the mainstay method for HLA typing - especially for class I - for many routine histocompatibility laboratories. The micro-lymphocytotoxicity assay (Kissmeyer et al., 1969; Terasaki and McClelland, 1964) is the standard approach: viable peripheral blood mononuclear cells (class I) or separate B-cells (class II) are mixed with antisera (polyclonal or monoclonal) of known HLA specificity.

Detection of polymorphism can be achieved by looking at the different amino acid composition of HLA molecules through biochemical techniques such as one-dimensional isoelectric focusing (IEF; Yang, 1987). This method relies on amino acid substitutions contributing to changes in charge of the HLA molecule.

Another HLA typing method is the mixed lymphocyte reaction (MLR). Concurrent to observations being made using HLA-specific antisera, it was noted that lymphocytes from two unrelated sources, when mixed in culture, would proliferate (Hirschom et al., 1963).

Analysis of HLA specificities from DNA provided a new approach to defining their polymorphic differences. Rather than looking at differences in the expressed molecule, polymorphism is characterized at the nucleotide level.

An important and powerful development in the field of molecular biology has been the polymerase chain reaction (PCR; Mullis et al., 1986; Mullis and Faloona, 1987). In tissue typing, PCR is used to amplify the polymorphic regions of HLA genes. This HLA PCR product can then be analyzed for its polymorphic differences, to establish the tissue type. A number of such approaches have been developed, including hetero duplex analysis of PCR products (Clay et al., 1994), single-stranded conformational polymorphism analysis of the PCR product (PCR-SSCP; Yoshida *et al.,* 1992), sequence-based typing (SBT; Santamaria et al.. 1992 and 1993), the use of sequence specific primers in PCR reaction (PCR-SSP; Olerup and Zetterquist, 1991), the use of PCR in combination with sequence-specific oligonucleotide probing (PCR-SSOP; Saiki et al.. 1986) or probing by reverse dot-blot (Saiki et al., 1989). These approaches, used singly or in combination, have all been applied as DNA-based methods for tissue-typing of class I and class II HLA specificities. DNA typing methods should be preferred over serological methods provided that an easy, rapid and reliable DNA typing method is available. Some differences at the subtype level which are detectable by DNA methods might go undetected by current serological typing methods, although these differences might provoke allograft rejection (Fleischhauer et al., 1990).

The HLA system is the most polymorphic human genetic system yet known. HLA class I genes share a similar structure (from 5' to 3'): a 5' untranslated flanking region, a first exon (exon 1) having a length of approximately 73 base pairs, a first intron (intron 1) having a length of approximately 130 base pairs, a second exon (exon 2), having a length of approximately 250 base pairs, a second intron (intron 2), having a length of approximately 272 base pairs, a third exon (exon 3), having a length of approximately 276 base pairs, a third intron (intron 3), having a length of approximately 588 base pairs and a fourth exon (exon 4), having a length of approximately 276 base pairs. Polymorphic substitutions within HLA class I alleles are mostly located in both exon 2 and exon 3, encoding the peptide binding groove of the class I molecule. These polymorphisms make differentiation between alleles achievable through a variety of molecular biological techniques such as sequencing or hybridization with relevant probes. In the current diagnostic kits exon 2 and exon 3 are amplified together, resulting in amplicons of about 1 kb, consisting at least of exon 2, intron 2 and exon 3. Locus-specific primers are available for the amplification of these 1 kb amplicons. However, such large amplicons are difficult to amplify and show secondary structure formation resulting in inefficient hybridization of some probes. In addition, due to the emergence of new HLA-Class I alleles, certain allele combinations cannot be distinguished anymore by the detection of polymorphism's only in exon 2 and exon 3 and additional typing in exon 4 is required. This raises the need for the additional amplification of exon 4, resulting in an even larger amplicon. Therefore, a separate amplification of exon 2, exon 3 and exon 4 would be desired resulting in amplification products that enable a more efficient typing of HLA class I alleles. However, as locus-specific primer annealing sites are scarce and cannot be found in exon 2, exon 3 or exon 4, the separate and locus-specific amplification of exon 2, exon 3 and exon 4 of HLA-A, HLA-B or HLA-C is not that evident.

Methods and Kits for HLA-A typing based on the amplification of exons 2 and 3 are for example disclosed in WO 99/07 883, WO 97/23 645, WO 98/26 091. HLA-A-specific primers are for example disclosed in EP-A-0 414 469, WO 97/23 650, WO 97/20 197.

### AIMS OF THE INVENTION

It is an aim of the present invention to provide a method for the locus-specific and separate amplification of exon 2, exon 3 and exon 4 of HLA-A alleles.

It is a more specific aim of the present invention to provide a method for a one-step, locus-specific, separate amplification of all three exons, exon 2, exon 3 and exon 4, of HLA-A.

Further disclosed herein are primers for use in a method for the locus-specific, separate amplification of exon 2 of HLA-A, HLA-B or HLA-C alleles,
primers for use in a method for the locus-specific, separate amplification of exon 3 of HLA-A, HLA-B or HLA-C alleles,
primers for use in a method for the locus-specific, separate amplification of exon 4 of HLA-A, HLA-B or HLA-C alleles,
a primer set for use in a method for the locus-specific, separate amplification of exon 2 of HLA-A, HLA-B or HLA-C alleles,
a primer set for use in a method for the locus-specific, separate amplification of exon 3 of HLA-A, HLA-B or HLA-C alleles,
a primer set for use in a method for the locus-specific, separate amplification of exon 4 of HLA-A, HLA-B or HLA-C alleles,
a multiplex primer mix for use in a method for the one step, locus-specific, separate amplification of exon 2 and exon 3 of HLA-A, HLA-B or HLA-C,
a multiplex primer mix for use in a method for the one step, locus-specific, separate amplification of exon 2 and exon 4 of HLA-A, HLA-B or HLA-C,
a multiplex primer mix for use in a method for the one step, locus-specific, separate amplification of exon 3 and exon 4 of HLA-A, HLA-B or HLA-C,
a multiplex primer mix for use in a method for the one step, locus-specific, separate amplification of exon 2, exon 3 and exon 4 of HLA-A, HLA-B or HLA-C.

It is another aim of the present invention to provide an improved method for the typing or subtyping of one or more HLA-A alleles in a sample.

It is another aim of the present invention to provide an improved diagnostic kit for the typing or subtyping of one or more HLA-A alleles in a sample.

It is a further aim of the present invention to provide an improved Line Probe Assay for the typing or subtyping of one or more HLA-A alleles in a sample.

### DETAILED DESCRIPTION OF THE INVENTION

The present invention relates to a method for the locus-specific, separate amplification of exon 2, exon 3 and exon 4 of HLA-A alleles, making use of at least one primer set wherein:
- for the amplification of exon 2, the reverse primer specifically hybridizes to a locus-specific target sequence in intron 2 of HLA-A;
- for the amplification of exon 3, the forward primer specifically hybridizes to a locus-specific target sequence in intron 2 of HLA-A, and/or the reverse primer specifically hybridizes to a locus-specific target sequence in intron 3 of HLA-A;
- for the amplification of exon 4, the forward primer specifically hybridizes to a locus-specific target sequence in intron 3 of HLA-A.

The amplification method disclosed herein thus, makes use of a primer set of which at least one of the primers is hybridizing to a locus-specific target sequence in intron 2 or in intron 3 of the HLA Class I gene in order to obtain the separate amplification of exon 2 (without exon 3 or exon 4), exon 3 (without exon 2 or exon 4) and/or exon 4 (without exon 2 or exon 3).

In the case where exon 2 is amplified, the reverse primer hybridizes to a locus-specific target sequence in intron 2 while the forward primer might be chosen to hybridize to a region upstream of exon 2 (e.g. in intron 1 or in exon 1) or to the initial nucleotides of exon 2. In a preferred embodiment, the forward primer hybridizes to a locus-specific region upstream of exon 2 (e.g. in intron 1 or in exon 1) or to locus-specific initial nucleotides of exon 2 and the reverse primer hybridizes to a locus-specific target sequence in intron 2.

In the case where exon 3 is amplified, the forward primer is hybridizing to a locus-specific target sequence in intron 2 while the reverse primer might be chosen to hybridize to the final nucleotides of exon 3 or downstream of exon 3 (e.g. in intron 3) or the reverse primer is hybridizing to a locus-specific target sequence in intron 3 while the forward primer might be chosen to hybridize to a region upstream to exon 3 (e.g. intron 2) or to the initial nucleotides of exon 3. In a preferred embodiment, exon 3 is amplified by use of a primer set of which the forward primer is hybridizing to a locus-specific target sequence in intron 2 and the reverse primer is hybridizing to a locus-specific target sequence in intron 3.

In the case where exon 4 is amplified, the forward primer is hybridizing to a locus-specific target sequence in intron 3 while the reverse primer might be chosen to hybridize to the final nucleotides of exon 4 or downstream of exon 4 (e.g. in intron 4). In a preferred embodiment, the forward primer is hybridizing to a locus-specific target sequence in intron 3 while the reverse primer hybridizes to locus-specific target sequences at the end of exon 4 or downstream of exon 4 (e.g. in intron 4).

This new amplification method will result in the amplification of shorter DNA fragments, containing only exon 2, only exon 3 or only exon 4, which are much easier to amplify and much easier for use in different typing methods such as sequencing or hybridization with different allele specific probes. From the example section it is clear that primer sets containing one primer hybridizing to a target sequence in intron 2 or intron 3 provide a much better and easier amplification of exon 2, exon 3 or exon 4 of HLA Class I alleles and a more clear and pronounced hybridization pattern with the allele typing probes. It has been the merit of the present inventors to define specific primers that enable this separate amplification of exon 2, exon 3 or exon 4 of only one HLA locus, while said exons of other HLA loci (i.e. other classical HLA genes, non-classical HLA genes or pseudogenes) are not co-amplified. As α-specific amplicons in the amplification product might result in a fault typing, the amplification method of the invention will certainly improve the security of the present typing methods.

The term "locus-specific" thus means that exon 2, exon 3 and/or exon 4 of only one locus (i.e. HLA-A, HLA-B or HLA-C) is amplified while exon 2, exon 3 and/or exon 4 of the other HLA loci (i.e. other classical HLA genes, non-classical HLA genes or pseudogenes) is not amplified. Thus, when primers specific for the amplification of exon 2, exon 3 and/or exon 4 of HLA-A are used, exon 2, exon 3 and/or exon 4 of HLA-B, HLA-C or of other HLA loci are not amplified. Similarly, when primers specific for the amplification of exon 2, exon 3 and/or exon 4 of HLA-B are used, exon 2, exon 3 and/or exon 4 of HLA-A, HLA-C or of other HLA loci are not amplified. Similarly, when primers specific for the amplification of exon 2, exon 3 and/or exon 4 of HLA-C are used, exon 2, exon 3 and/or exon 4 of HLA-A, HLA-B or of other HLA loci are not amplified. From this it is clear that exon 2, exon 3 and/or exon 4 of 2, 3 or more different HLA loci can never be amplified together. Thus, exon 2, exon 3 and/or exon 4 of HLA-A can never be amplified in the same reaction tube with exon 2, exon 3 and/or exon 4 of HLA-B. Similarly, exon 2, exon 3 and/or exon 4 of HLA-A can never be amplified in the same reaction tube with exon 2, exon 3 and/or exon 4 of HLA-C. Similarly, exon 2, exon 3 and/or exon 4 of HLA-B can never be amplified in the same reaction tube with exon 2, exon 3 and/or exon 4 of HLA-C. Similarly, exon 2, exon 3 and/or exon 4 of HLA-A can never be amplified in the same reaction tube with exon 2, exon 3 and/or exon 4 of HLA-B and exon 2, exon 3 and/or exon 4 of HLA-C. As a consequence, the present invention relates to a method for the locus-specific, separate amplification of exon 2, exon 3 and exon 4 of HLA-A alleles. This means that the invention relates to a method for the separate amplification of exon 2, exon 3 and exon 4 of HLA-A.

The term "primer" refers to a single stranded oligonucleotide sequence capable of acting as a point of initiation for the synthesis of a primer extension product that is complementary to the nucleic acid strand to be copied. The length and the sequence of the primer must be such that they allow to prime the synthesis of the extension products. In a specific embodiment, the length of the primer is about 5-50 nucleotides. In another specific embodiment, the length of the primer is about 10-30 nucleotides. In another specific embodiment, the length of the primers is about 20-25 nucleotides. Specific length and sequence will depend on the complexity of the required DNA or RNA target, as well as on the conditions at which the primer is used, such as temperature and ionic strength.

The expression "primer set" or "primer pair" refers to a pair of primers allowing the amplification of part or all of exon 2, exon 3 or exon 4 of HLA-A, HLA-B or HLA-C. A primer set always consists of a forward primer (or 5' primer) and a reverse primer (or 3' primer).

The terms "to hybridize specifically" and "specifically hybridizes" mean that, during the amplification step, said primer forms a duplex with part of its target sequence or with the entire target sequence under the experimental conditions used, and that under those conditions said primer does not form a duplex with other sequences of the polynucleic acids present in the sample to be analysed. It should be understood that primers that are designed to specifically hybridize to a target sequence of a nucleic acid, may fall within said target sequence or may to a large extent overlap with said target sequence (i.e. form a duplex with nucleotides outside as well as within said target sequence).

The term "target sequence" of a primer according to the present invention is a sequence within intron 2 or intron 3 of the HLA Class I alleles to which the primer is completely complementary or partially complementary (i.e. with up to 20%, 15%, 10% or 5% mismatches). It is to be understood that the complement of said target sequence is also a suitable target sequence in some cases. The fact that amplification primers do not have to match exactly with the corresponding target sequence in the template to warrant proper amplification is amply documented in the literature (Kwok et al., 1990). However, when the primers are not completely complementary to their target sequence, it should be taken into account that the amplified fragments will have the sequence of the primers and not of the target sequence.

In one embodiment, the amplification is performed in a reaction tube with a primer set for the amplification of exon 2 as described above (reverse primer hybridizes with a locus-specific target sequence in intron 2) and consequently only exon 2 is amplified. In another embodiment, the amplification is performed in a reaction tube with a primer set for the amplification of exon 3 as described above (forward primer hybridizes with a locus-specific target sequence in intron 2) and consequently only exon 3 is amplified.

In another embodiment, the amplification is performed in a reaction tube with a primer set for the amplification of exon 3 as described above (reverse primer hybridizes with a locus-specific target sequence in intron 3) and consequently only exon 3 is amplified.

In another embodiment, the amplification is performed in a reaction tube with a primer set for the amplification of exon 3 as described above (forward primer hybridizes with a locus-specific target sequence in intron 2 and reverse primer hybridizes with a locus-specific target sequence in intron 3) and consequently only exon 3 is amplified.

In another embodiment, the amplification is performed in a reaction tube with a primer set for the amplification of exon 4 as described above (forward primer hybridizes with a locus-specific target sequence in intron 3) and consequently only exon 4 is amplified.

Accordingly, disclosed herein is a method for the separate amplification of exon 2, exon 3 or exon 4 of HLA Class I alleles.

In another embodiment, the different primer sets involved in the amplification of exon 2 and the amplification of exon 3 are mixed and the separate amplification of both exon 2 and exon 3 is performed in a single reaction tube. Thus, further disclosed herein is a method as described above further characterized that both exon 2 and exon 3 of HLA-A, HLA-B or HLA-C are amplified by use of a multiplex primer mix containing at least one primer pair for amplification of exon 2 and at least one primer pair for amplification of exon 3. Accordingly, disclosed is a method for the separate amplification of exon 2 and exon 3 of HLA Class I alleles.

In another embodiment, the different primer sets involved in the amplification of exon 2 and the amplification of exon 4 are mixed and the separate amplification of both exon 2 and exon 4 is performed in a single reaction tube. Thus, disclosed is a method as described above further characterized that both exon 2 and exon 4 of HLA-A, HLA-B or HLA-C are amplified by use of a multiplex primer mix containing at least one primer pair for amplification of exon 2 and at least one primer pair for amplification of exon 4. Accordingly, disclosed is a method for the separate amplification of exon 2 and exon 4 of HLA Class I alleles.

In another embodiment, the different primer sets involved in the amplification of exon 3 and the amplification of exon 4 are mixed and the separate amplification of both exon 3 and exon 4 is performed in a single reaction tube. Thus, disclosed is a method as described above further characterized that both exon 3 and exon 4 of HLA-A, HLA-B or HLA-C are amplified by use of a multiplex primer mix containing at least one primer pair for amplification of exon 3 and at least one primer pair for amplification of exon 4. Accordingly, disclosed is method for the separate amplification of exon 3 and exon 4 of HLA Class I alleles.

In another embodiment, the different primer sets involved in the amplification of exon 2, the amplification of exon 3 and the amplification of exon 4 are mixed and the separate amplification of all three exons, exon 2, exon 3 and exon 4, is performed in a single reaction tube. Thus, the present invention also relates to a method as described above further characterized that all three exons, exon 2, exon 3 and exon 4, of HLA-A, HLA-B or HLA-C are amplified by use of a multiplex primer mix containing at least one primer pair for amplification of exon 2, at least one primer pair for the amplification of exon 3 and at least one primer pair for amplification of exon 4.

Accordingly, the present invention relates to a method for the separate amplification of exon 2, exon 3 and exon 4 of HLA Class I alleles.

The amplification method used can be either polymerase chain reaction (PCR; Saiki et al., 1988), ligase chain reaction (LCR; Landgren et al., 1988; Wu and Wallace, 1989; Barany, 1991), nucleic acid sequence-based amplification (NASBA; Guatelli et al., 1990; Compton, 1991), transcription-based amplification system (TAS; Kwoh et al., 1989), strand displacement amplification (SDA; Duck, 1990) or amplification by means of Qß replicase (Lomeli et al., 1989) or any other suitable method to amplify nucleic acid molecules known in the art. Also TMA (Guatelli et al., 1990) or bDNA (Sanchez-Pescador et al., 1988; Urdea et al., 1991) techniques can be used in the method of the present invention. In a specific embodiment, exon 2, exon 3 and exon 4 of HLA-A are amplified by PCR.

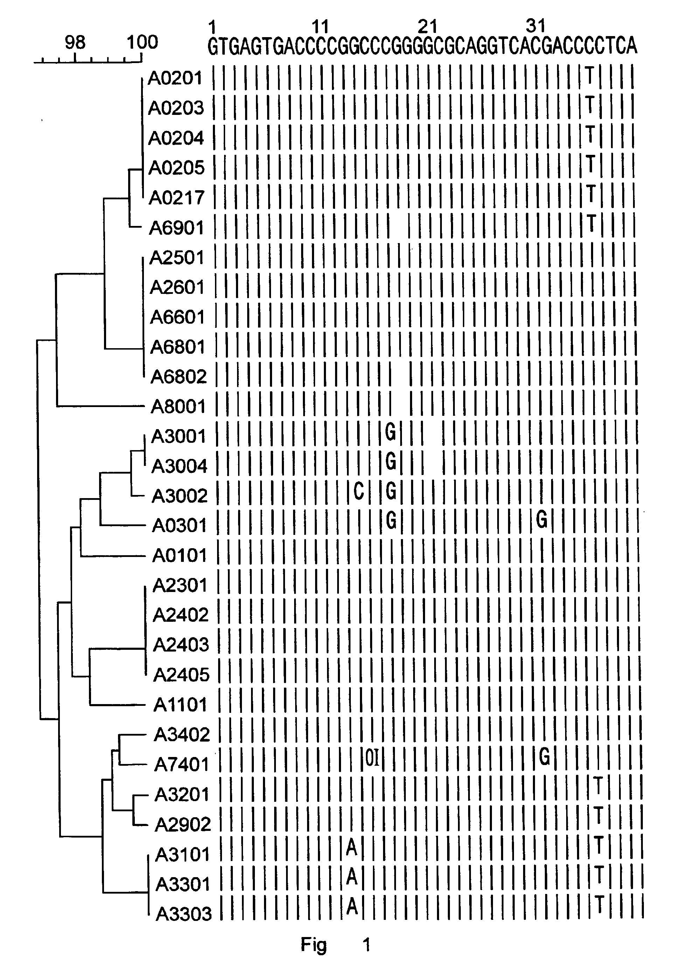

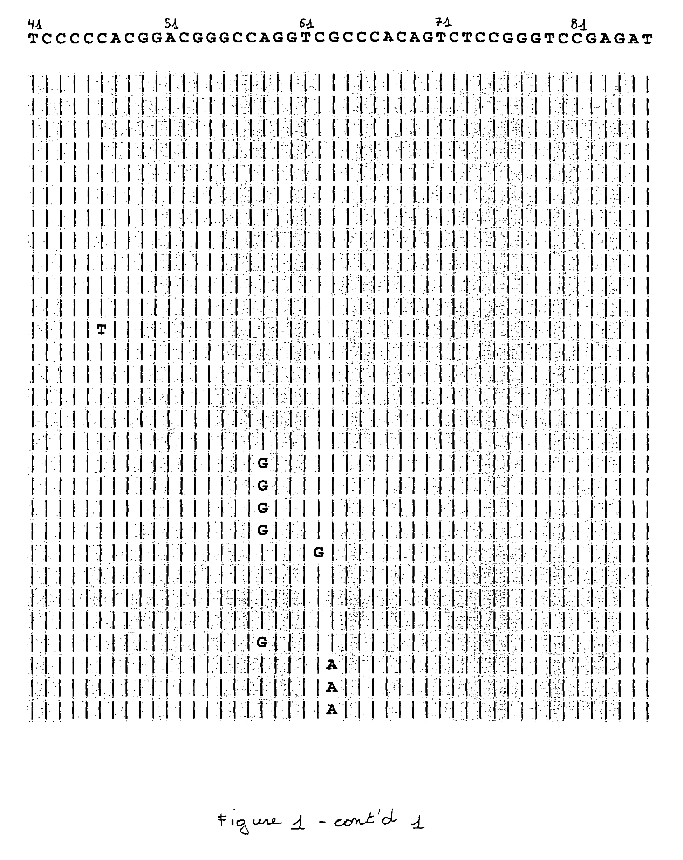

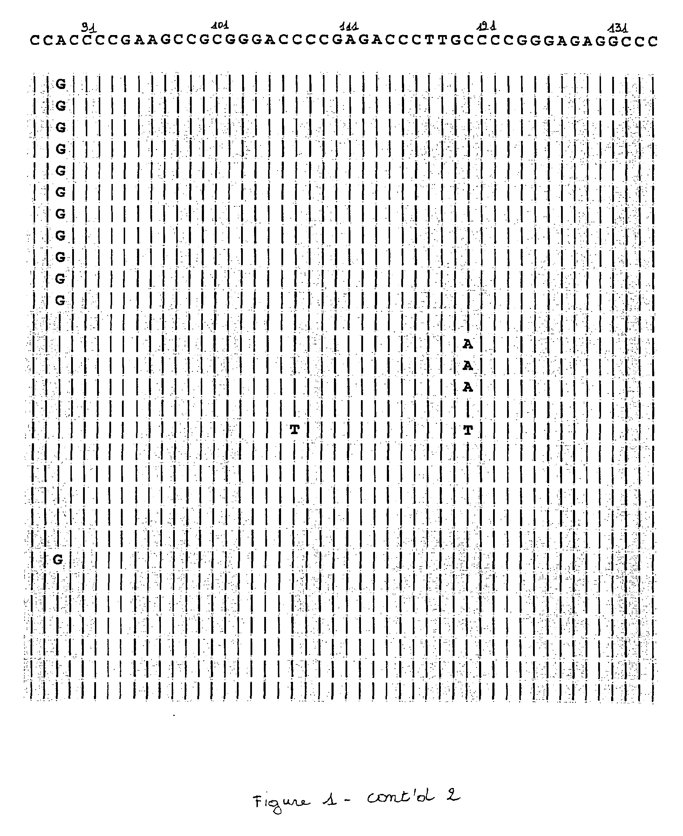

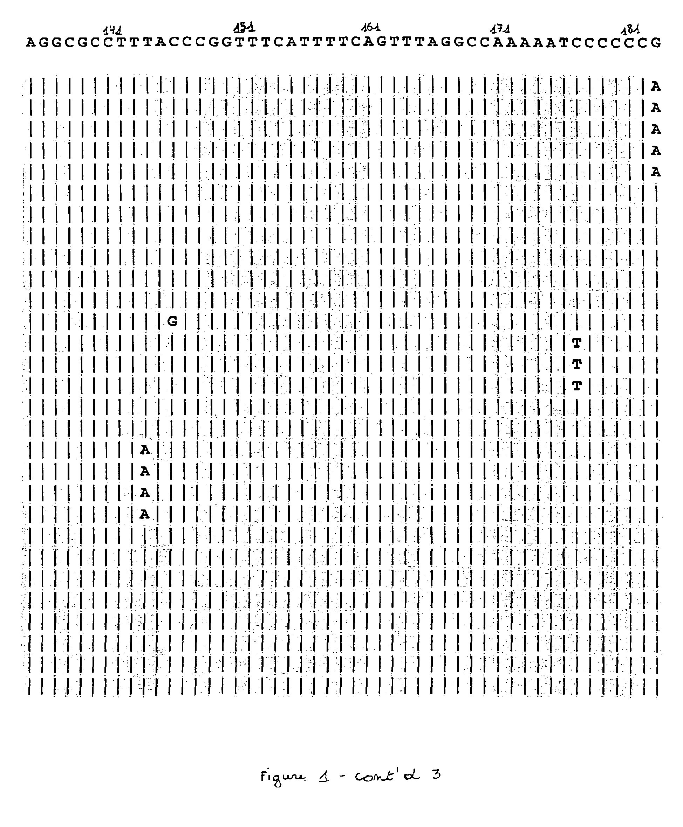

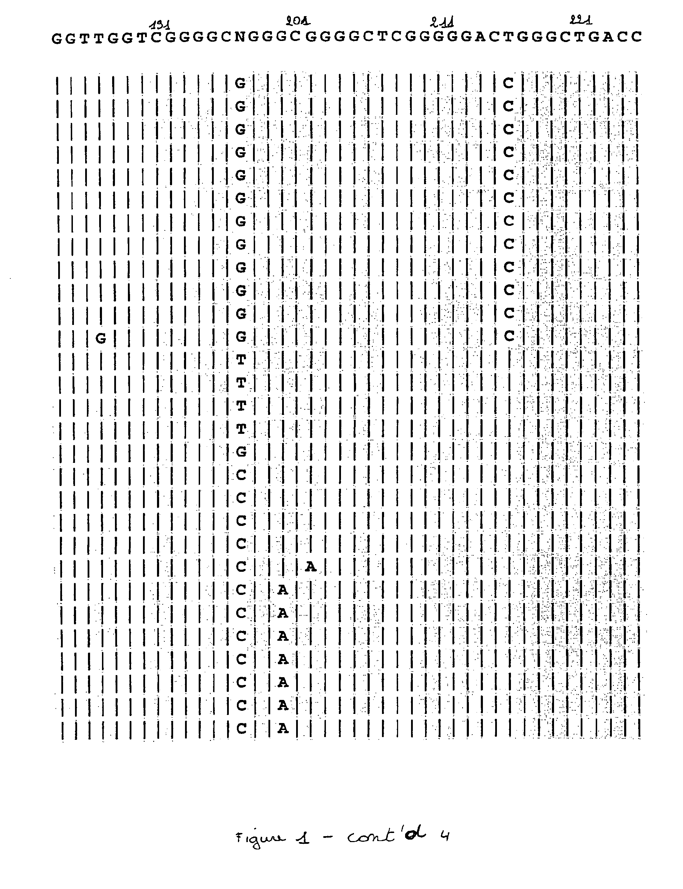

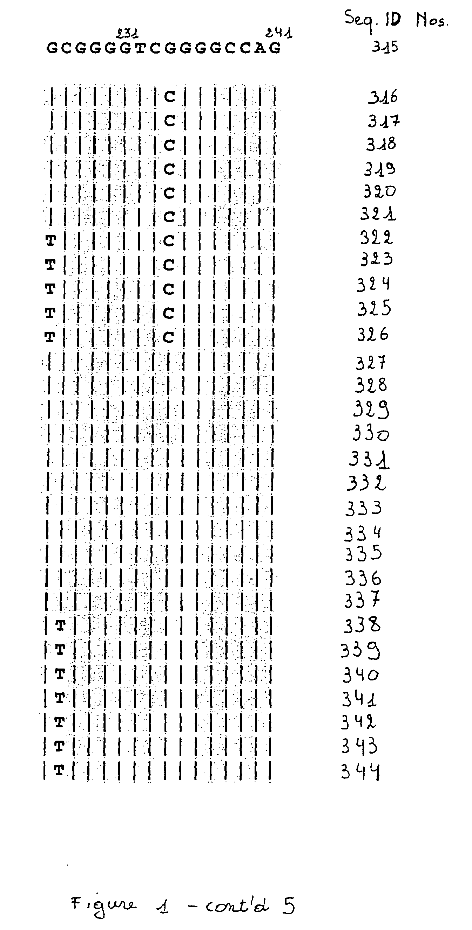

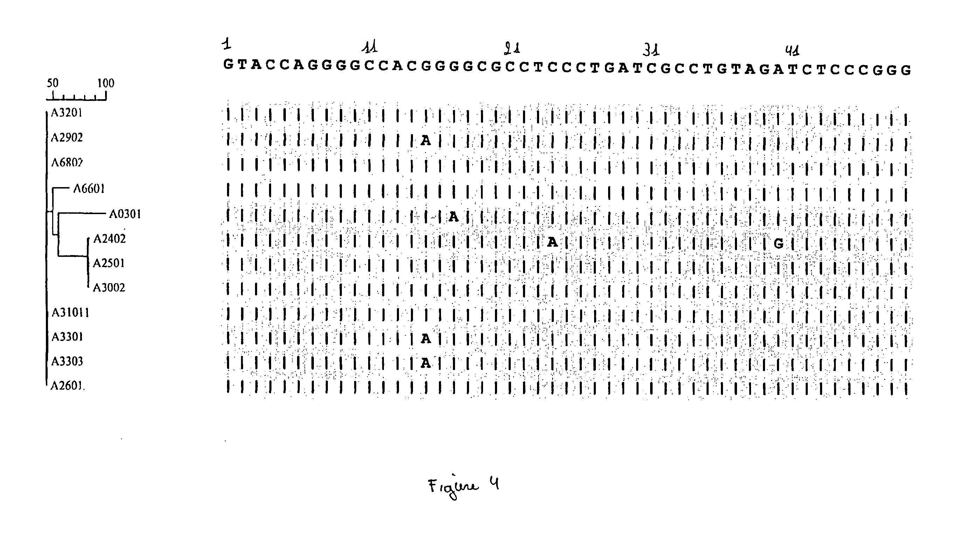

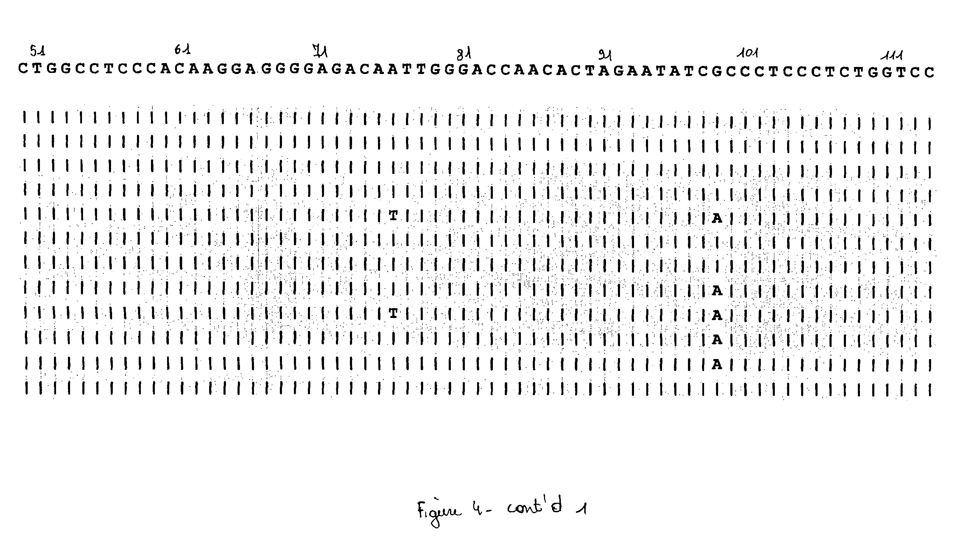

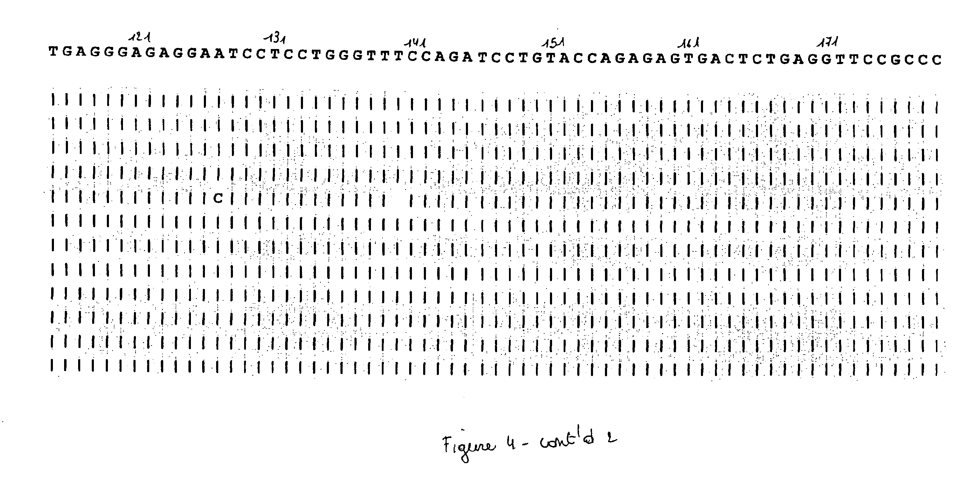

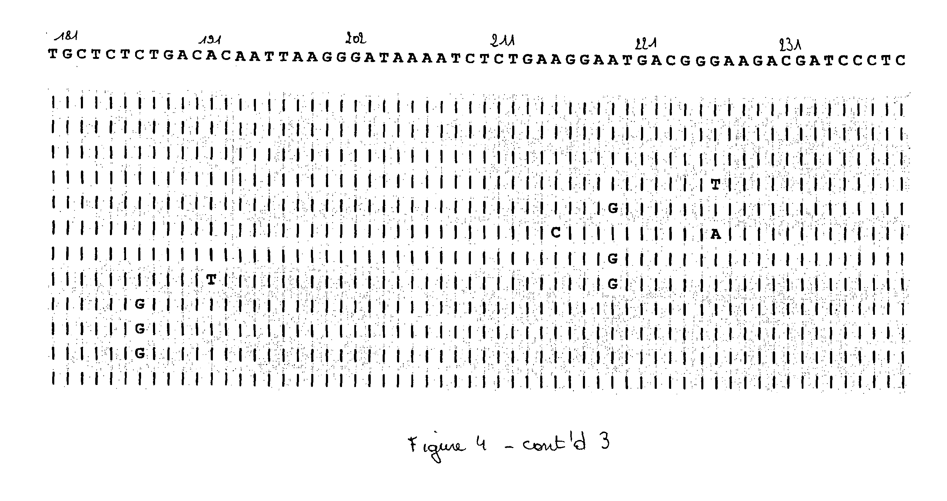

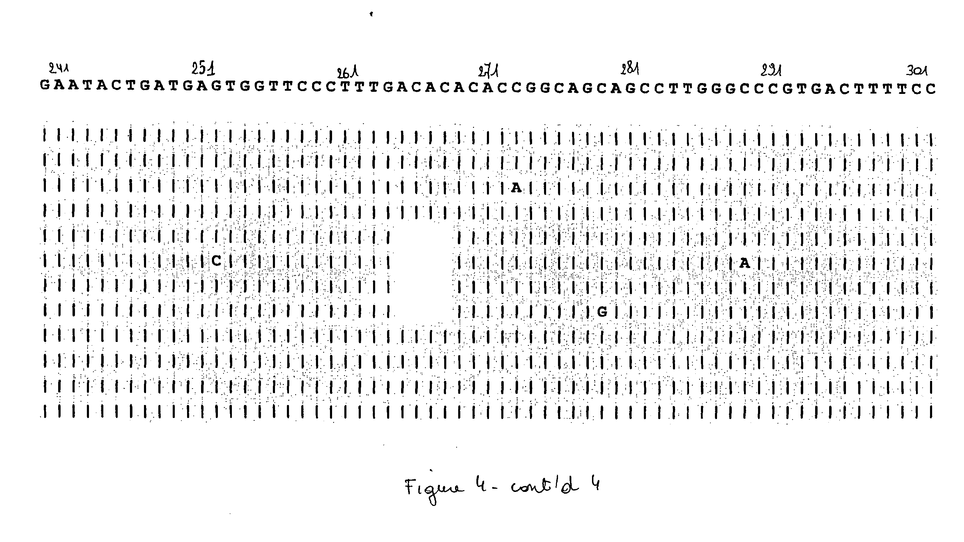

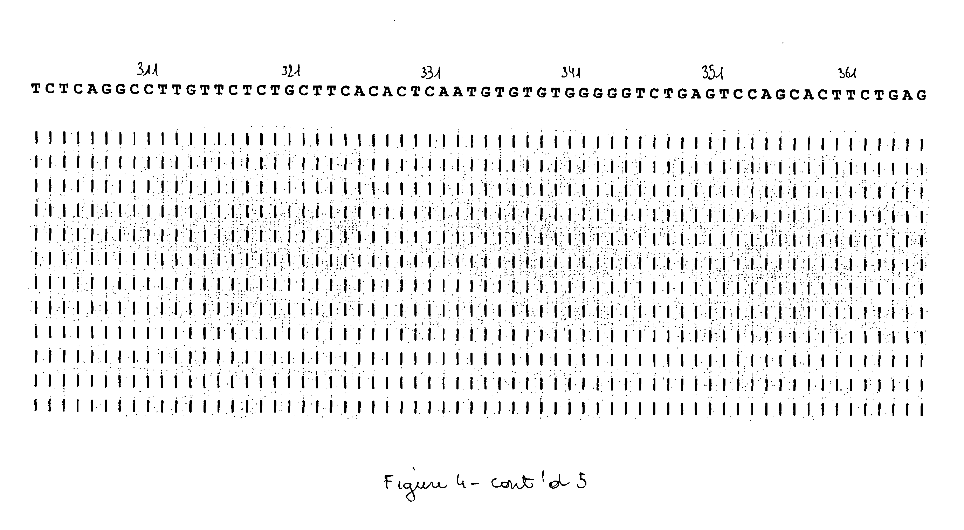

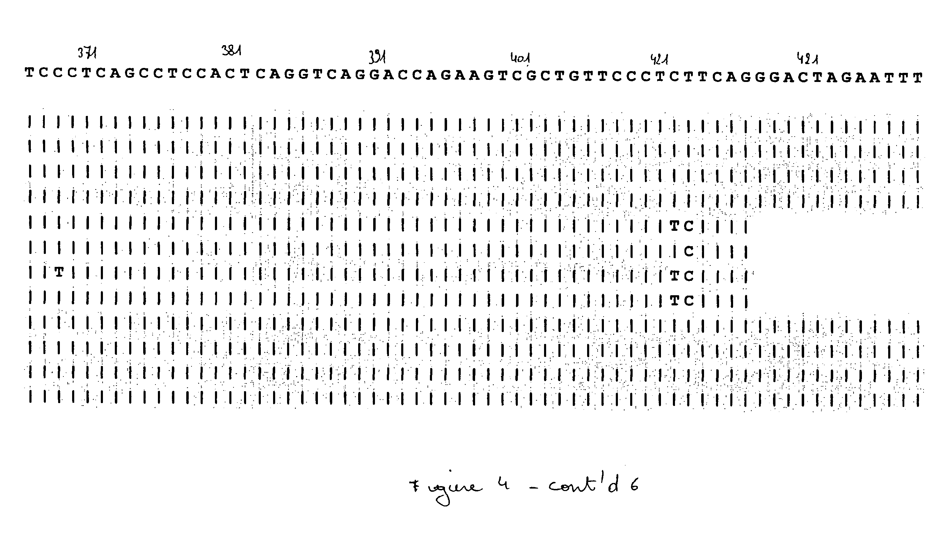

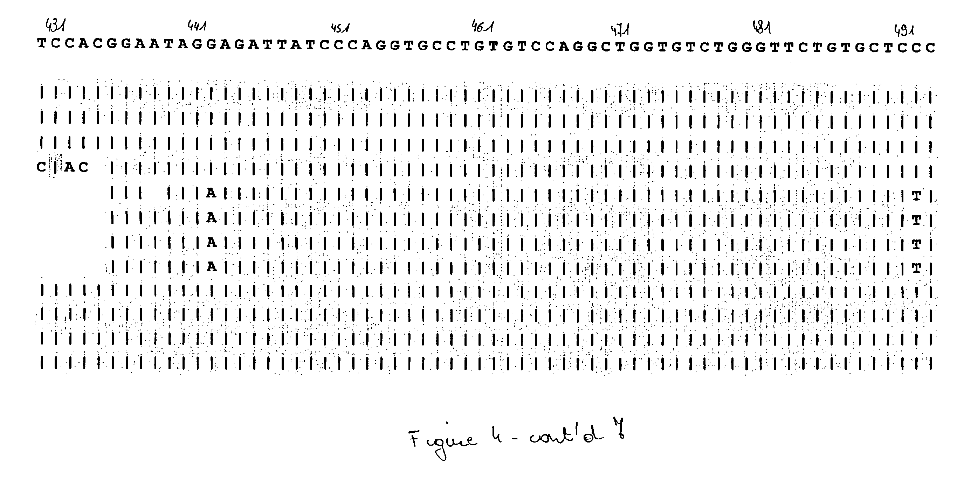

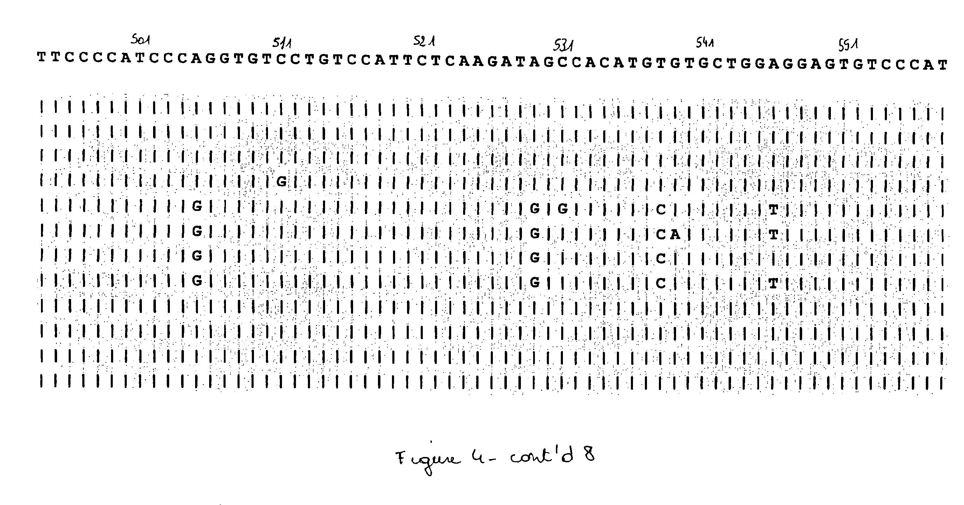

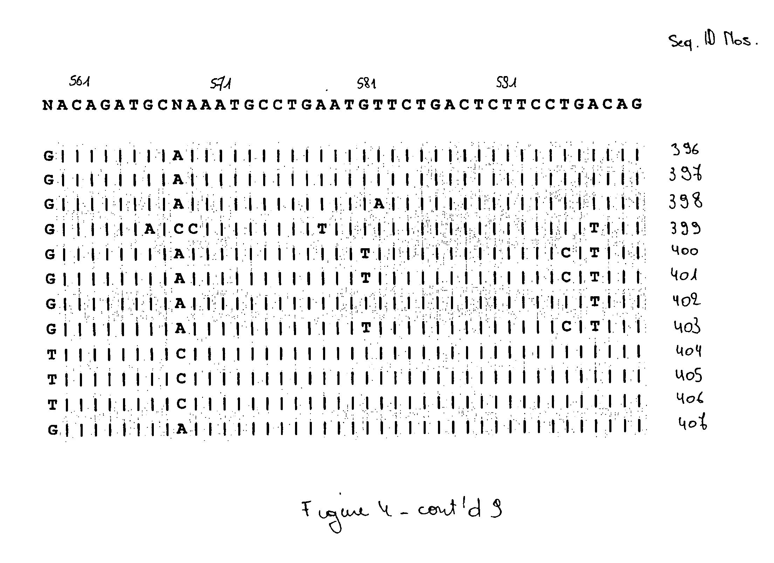

In a specific embodiment, the present invention relates to a method as described above, further characterized that the locus-specific target sequence is situated at one of the following positions:
- 67, 96, 109, 110, 118, 123, 131 or 181 of the HLA-A intron 2 (Fig. 1) and/or 32, 50, 62, 73, 83, 86, 118, 130, 150, 501, 525, 561 or 571 (Fig. 4) of the HLA-A intron 3.

As these positions all contain locus-specific nucleotides, these position are particularly suitable for designing an efficient primer for the locus-specific amplification of exon 2, exon 3 or exon 4. The primers can be of different length. In a specific embodiment, the length of the primer is about 5-50 nucleotides. In another specific embodiment, the length of the primer is about 10-30 nucleotides. In another specific embodiment, the length of the primers is about 20-25 nucleotides. Specific length and sequence will depend on the complexity of the required DNA or RNA target, as well as on the conditions at which the primer is used, such as temperature and ionic strength.

In a specific embodiment, the present invention relates to a method as described above further characterized that said positions constitute the 3' end of the primer that is used for the amplification of exon 2, exon 3 or exon 4. Thus, from the above described positions, forward as well as reverse primers can be designed that have their 3' end in these specific positions. The reverse primer having its 3' end in one of the above-mentioned specific positions of intron 2 will enable the locus-specific amplification of exon 2 of the HLA-A allele. The forward primer having its 3' end in one of the above-mentioned specific positions of intron 2 will enable the locus-specific amplification of exon 3 of the HLA-A allele. The reverse primer having its 3' end in one of the above-mentioned specific positions of intron 3 will enable the locus-specific amplification of exon 3 of the HLA-A allele. The forward primer having its 3' end in one of the above-mentioned specific positions of intron 3 will enable the locus-specific amplification of exon 4 of the HLA-A allele. The primers can be of different length. In a specific embodiment, the length of the primer is about 5-50 nucleotides. In another specific embodiment, the length of the primer is about 10-30 nucleotides. In another specific embodiment, the length of the primers is about 20-25 nucleotides. Specific length and sequence will depend on the complexity of the required DNA or RNA target, as well as on the conditions at which the primer is used, such as temperature and ionic strength.

In another embodiment, the present invention relates to a method as described above, further characterized that the primer is chosen from the following list:
- for the amplification of exon 2 of HLA-A (table 1):
   5'ATCTCGGACCCGGAGACTGT3' (SEQ ID NO 1)
   5'GATCTCGGACCCGGAGACTGT3' (SEQ ID NO 2)
   5'GGATCTCGGACCCGGAGACTGT3' (SEQ ID NO 3)
   5'YGGATCTCGGACCCGGAGACTGT3' (SEQ ID NO 4)
   5'GYGGATCTCGGACCCGGAGACTGT3' (SEQ ID NO 5)
   5'GGYGGATCTCGGACCCGGAGACTGT3' (SEQ ID NO 6)
   5'GGTCTCGGRGTCCCGCGGCT3' (SEQ ID NO 7)
   5'GGGTCTCGGRGTCCCGCGGCT3' (SEQ ID NO 8)
   5'AGGGTCTCGGRGTCCCGCGGCT3' (SEQ ID NO 9)
   5'AAGGGTCTCGGRGTCCCGCGGCT3' (SEQ ID NO 10)
   5'CAAGGGTCTCGGRGTCCCGCGGCT3' (SEQ ID NO 11)
   5'CTCCCGGGDCAAGGGTCTCG3' (SEQ ID NO 12)
   5'TCTCCCGGGDCAAGGGTCTCG3' (SEQ ID NO 13)
   5'CTCTCCCGGGDCAAGGGTCTCG3' (SEQ ID NO 14)
   5'CCTCTCCCGGGDCAAGGGTCTCG3' (SEQ ID NO 15)
   5'GCCTCTCCCGGGDCAAGGGTCTCG3' (SEQ ID NO 16)
   5'GGCCTCTCCCGGGDCAAGGGTCTCG3' (SEQ ID NO 17)
   5'TCTCCCGGGDCAAGGGTCTC3' (SEQ ID NO 18)
   5'CTCTCCCGGGDCAAGGGTCTC3' (SEQ ID NO 19)
   5'CCTCTCCCGGGDCAAGGGTCTC3' (SEQ ID NO 20)
   5'GCCTCTCCCGGGDCAAGGGTCTC3' (SEQ ID NO 21)
   5'GGCCTCTCCCGGGDCAAGGGTCTC3' (SEQ ID NO 22)
   5'GGGCCTCTCCCGGGDCAAGGGTCTC3' (SEQ ID NO 23)
   5'CCTGGGCCTCTCCCGGGDCA3' (SEQ ID NO 30)
   5'GCCTGGGCCTCTCCCGGGDCA3' (SEQ ID NO 31)
   5'CGCCTGGGCCTCTCCCGGGDCA3' (SEQ ID NO 32)
   5'GCGCCTGGGCCTCTCCCGGGDCA3' (SEQ ID NO 33)
   5'GGCGCCTGGGCCTCTCCCGGGDCA3' (SEQ ID NO 34)
   5'AGGCGCCTGGGCCTCTCCCGGGDCA3' (SEQ ID NO 35)
   5'AGGCGCCTGGGCCTCTCCCG3' (SEQ ID NO 36)
   5'AAGGCGCCTGGGCCTCTCCCG3' (SEQ ID NO 37)
   5' WAAGGCGCCTGGGCCTCTCCCG3' (SEQ ID NO 38)
   5'TWAAGGCGCCTGGGCCTCTCCCG3' (SEQ ID NO 39)
   5'GTWAAGGCGCCTGGGCCTCTCCCG3' (SEQ ID NO 40)
   5'GGTWAAGGCGCCTGGGCCTCTCCCG3' (SEQ ID NO 41)
   5'CCGGGTWAAGGCGCCTGGGC3' (SEQ ID NO 42)
   5'ACCGGGTWAAGGCGCCTGGGC3' (SEQ ID NO 43)
   5'AACCGGGTWAAGGCGCCTGGGC3' (SEQ ID NO 44)
   5'AAACCGGGTWAAGGCGCCTGGGC3' (SEQ ID NO 45)
   5'GAAACCGGGTWAAGGCGCCTGGGC3' (SEQ ID NO 46)
   5'TGAAACCGGGTWAAGGCGCCTGGGC3' (SEQ ID NO 47)
   5'YCCVGCCCCGACCAACCYGG3' (SEQ ID NO 48)
   5'GYCCVGCCCCGACCAACCYGG3' (SEQ ID NO 49)
   5'YGYCCVGCCCCGACCAACCYGG3' (SEQ ID NO 50)
   5'CYGYCCVGCCCCGACCAACCYGG3' (SEQ ID NO 51)
   5'CCYGYCCVGCCCCGACCAACCYGG3' (SEQ ID NO 52)
   5'CCCYGYCCVGCCCCGACCAACCYGG3' (SEQ ID NO 53)
- for the amplification of exon 3 of HLA-A (table 2; table 3):
   5'CGGACGGGCCRGGTSRCCCA3' (SEQ ID NO 54)
   5'ACGGACGGGCCRGGTSRCCCA3' (SEQ ID NO 55)
   5'CACGGACGGGCCRGGTSRCCCA3' (SEQ ID NO 56)
   5'CCACGGACGGGCCRGGTSRCCCA3' (SEQ ID NO 57)
   5'CCCACGGACGGGCCRGGTSRCCCA3' (SEQ ID NO 58)
   5'CCCCACGGACGGGCCRGGTSRCCCA3' (SEQ ID NO 59)
   5'GGTCCGAGATCCRCCCCGAA3' (SEQ ID NO 60)
   5'GGGTCCGAGATCCRCCCCGAA3' (SEQ ID NO 61)
   5'CGGGTCCGAGATCCRCCCCGAA3' (SEQ ID NO 62)
   5'CCGGGTCCGAGATCCRCCCCGAA3' (SEQ ID NO 63)
   5'TCCGGGTCCGAGATCCRCCCCGAA3' (SEQ ID NO 64)
   5'CTCCGGGTCCGAGATCCRCCCCGAA3' (SEQ ID NO 65)
   5'CCCCGAAGCCGCGGGACYCC3' (SEQ ID NO 66)
   5'RCCCCGAAGCCGCGGGACYCC3' (SEQ ID NO 67)
   5'CRCCCCGAAGCCGCGGGACYCC3' (SEQ ID NO 68)
   5'CCRCCCCGAAGCCGCGGGACYCC3' (SEQ ID NO 69)
   5'TCCRCCCCGAAGCCGCGGGACYCC3' (SEQ ID NO 70)
   5'ATCCRCCCCGAAGCCGCGGGACYCC3' (SEQ ID NO 71)
   5'CCCGAAGCCGCGGGACYCCG3' (SEQ ID NO 72)
   5'CCCCGAAGCCGCGGGACYCCG3' (SEQ ID NO 73)
   5'RCCCCGAAGCCGCGGGACYCCG3' (SEQ ID NO 74)
   5'CRCCCCGAAGCCGCGGGACYCCG3' (SEQ ID NO 75)
   5'CCRCCCCGAAGCCGCGGGACYCCG3' (SEQ ID NO 76)
   5'TCCRCCCCGAAGCCGCGGGACYCCG3' (SEQ ID NO 77)
   5'CGCGGGACYCCGAGACCCTT3' (SEQ ID NO 84)
   5'CCGCGGGACYCCGAGACCCTT3' (SEQ ID NO 85)
   5'GCCGCGGGACYCCGAGACCCTT3'(SEQ ID NO 86)
   5'AGCCGCGGGACYCCGAGACCCTT3' (SEQ ID NO 87)
   5'AAGCCGCGGGACYCCGAGACCCTT3' (SEQ ID NO 88)
   5'GAAGCCGCGGGACYCCGAGACCCTT3' (SEQ ID NO 89)
   5'GACYCCGAGACCCTTGDCCC3' (SEQ ID NO 90)
   5'GGACYCCGAGACCCTTGDCCC3' (SEQ ID NO 91)
   5'GGGACYCCGAGACCCTTGDCCC3' (SEQ ID NO 92)
   5'CGGGACYCCGAGACCCTTGDCCC3' (SEQ ID NO 93)
   5'GCGGGACYCCGAGACCCTTGDCCC3' (SEQ ID NO 94)
   5'CGCGGGACYCCGAGACCCTTGDCCC3' (SEQ ID NO 95)
   5'GACCCTTGDCCCGGGAGAGG3' (SEQ ID NO 96)
   5'AGACCCTTGDCCCGGGAGAGG3' (SEQ ID NO 97)
   5'GAGACCCTTGDCCCGGGAGAGG3' (SEQ ID NO 98)
   5'CGAGACCCTTGDCCCGGGAGAGG3' (SEQ ID NO 99)
   5'CCGAGACCCTTGDCCCGGGAGAGG3' (SEQ ID NO 100)
   5'YCCGAGACCCTTGDCCCGGGAGAGG3' (SEQ ID NO 101)
   5'GTTTAGGCCAAAAATCCCCC3' (SEQ ID NO 102)
   5'AGTTTAGGCCAAAAATCCCCC3' (SEQ ID NO 103)
   5'CAGTTTAGGCCAAAAATCCCCC3' (SEQ ID NO 104)
   5'TCAGTTTAGGCCAAAAATCCCCC3' (SEQ ID NO 105)
   5'TTCAGTTTAGGCCAAAAATCCCCC3' (SEQ ID NO 106)
   5'TTTCAGTTTAGGCCAAAAATCCCCC3' (SEQ ID NO 107)
   5'AGCCCGGGAGATCTAYAGGC3' (SEQ ID NO 150)
   5'CAGCCCGGGAGATCTAYAGGC3' (SEQ ID NO 151)
   5'CCAGCCCGGGAGATCTAYAGGC3' (SEQ ID NO 152)
   S'GCCAGCCCGGGAGATCTAYAGGC3' (SEQ ID NO 153)
   5'GGCCAGCCCGGGAGATCTAYAGGC3' (SEQ ID NO 154)
   5'AGGCCAGCCCGGGAGATCTAYAGGC3' (SEQ ID NO 155)
   5'CCCTCCTTGTGGGAGGCCAG3' (SEQ ID NO 156)
   5'CCCCTCCTTGTGGGAGGCCAG3' (SEQ ID NO 157)
   5'TCCCCTCCTTGTGGGAGGCCAG3' (SEQ ID NO 158)
   5'CTCCCCTCCTTGTGGGAGGCCAG3' (SEQ ID NO 159)
   5'TCTCCCCTCCTTGTGGGAGGCCAG3' (SEQ ID NO 160)
   5'GTCTCCCCTCCTTGTGGGAGGCCAG3' (SEQ ID NO 161)
   5'CCCAAWTGTCTCCCCTCCTT3' (SEQ ID NO 162)
   5'TCCCAAWTGTCTCCCCTCCTT3' (SEQ ID NO 163)
   5'GTCCCAAWTGTCTCCCCTCCTT3' (SEQ ID NO 164)
   5'GGTCCCAAWTGTCTCCCCTCCTT3' (SEQ ID NO 165)
   5'TGGTCCCAAWTGTCTCCCCTCCTT3' (SEQ ID NO 166)
   5'TTGGTCCCAAWTGTCTCCCCTCCTT3' (SEQ ID NO 167)
   5'CTAGTGTTGGTCCCAAWTGT3' (SEQ ID NO 168)
   5'TCTAGTGTTGGTCCCAAWTGT3' (SEQ ID NO 169)
   5'TTCTAGTGTTGGTCCCAAWTGT3' (SEQ ID NO 170)
   5'ATTCTAGTGTTGGTCCCAAWTGT3' (SEQ ID NO 171)
   5'TATTCTAGTGTTGGTCCCAAWTGT3' (SEQ ID NO 172)
   5'ATATTCTAGTGTTGGTCCCAAWTGT3' (SEQ ID NO 173)
   5'GGGYGATATTCTAGTGTTGG3' (SEQ ID NO 174)
   5'AGGGYGATATTCTAGTGTTGG3' (SEQ ID NO 175)
   5'GAGGGYGATATTCTAGTGTTGG3' (SEQ ID NO 176)
   5'GGAGGGYGATATTCTAGTGTTGG3' (SEQ ID NO 177)
   5'GGGAGGGYGATATTCTAGTGTTGG3' (SEQ ID NO 178)
   5'AGGGAGGGYGATATTCTAGTGTTGG3' (SEQ ID NO 179)
   5'GGAGGGYGATATTCTAGTGT3' (SEQ ID NO 180)
   5'GGGAGGGYGATATTCTAGTGT3' (SEQ ID NO 181)
   5'AGGGAGGGYGATATTCTAGTGT3' (SEQ ID NO 182)
   5'GAGGGAGGGYGATATTCTAGTGT3' (SEQ ID NO 183)
   5'AGAGGGAGGGYGATATTCTAGTGT3' (SEQ ID NO 184)
   5'CAGAGGGAGGGYGATATTCTAGTGT3' (SEQ ID NO 185)
   5'CCCAGGAGGAKTCCTCTCCC3' (SEQ ID NO 186)
   5'ACCCAGGAGGAKTCCTCTCCC3' (SEQ ID NO 187)
   5'AACCCAGGAGGAKTCCTCTCCC3' (SEQ ID NO 188)
   5'AAACCCAGGAGGAKTCCTCTCCC3' (SEQ ID NO 189)
   5'GAAACCCAGGAGGAKTCCTCTCCC3' (SEQ ID NO 190)
   5'GGAAACCCAGGAGGAKTCCTCTCCC3' (SEQ ID NO 191)
   5'AGGATCTGGAAACCCAGGAG3' (SEQ ID NO 192)
   5'CAGGATCTGGAAACCCAGGAG3' (SEQ ID NO 193)
   5'ACAGGATCTGGAAACCCAGGAG3' (SEQ ID NO 194)
   5'TACAGGATCTGGAAACCCAGGAG3' (SEQ ID NO 195)
   5'GTACAGGATCTGGAAACCCAGGAG3' (SEQ ID NO 196)
   5'GGTACAGGATCTGGAAACCCAGGAG3' (SEQ ID NO 197)
   5'TCAGAGTCACTCTCTGGTAC3' (SEQ ID NO 198)
   5'CTCAGAGTCACTCTCTGGTAC3' (SEQ ID NO 199)
   5'CCTCAGAGTCACTCTCTGGTAC3' (SEQ ID NO 200)
   5'ACCTCAGAGTCACTCTCTGGTAC3' (SEQ ID NO 201)
   5'AACCTCAGAGTCACTCTCTGGTAC3' (SEQ ID NO 202)
   5'GAACCTCAGAGTCACTCTCTGGTAC3' (SEQ ID NO 203)
- for the amplification of exon 4 of HLA-A (table 4):
   5'TTCTGTGCTCYCTTCCCCAT3' (SEQ ID NO 204)
   5'GTTCTGTGCTCYCTTCCCCAT3'(SEQ ID NO 205)
   5'GGTTCTGTGCTCYCTTCCCCAT3' (SEQ ID NO 206)
   5'GGGTTCTGTGCTCYCTTCCCCAT3' (SEQ ID NO 207)
   5'TGGGTTCTGTGCTCYCTTCCCCAT3' (SEQ ID NO 208)
   5'CTGGGTTCTGTGCTCYCTTCCCCAT3' (SEQ ID NO 209)
   5'GGTGTCCTGTCCATTCTCAA3' (SEQ ID NO 24)
   5'RGGTGTCCTGTCCATTCTCAA3' (SEQ ID NO 25)
   5'CRGGTGTCCTGTCCATTCTCAA3' (SEQ ID NO 26)
   5'CCRGGTGTCCTGTCCATTCTCAA3' (SEQ ID NO 27)
   5'CCCRGGTGTCCTGTCCATTCTCAA3' (SEQ ID NO 28)
   5'TCCCRGGTGTCCTGTCCATTCTCAA3' (SEQ ID NO 29)
   5'CTGGWGGAGTGTCCCATKAC3' (SEQ ID NO 210)
   5'GCTGGWGGAGTGTCCCATKAC3' (SEQ ID NO 211)
   5'TGCTGGWGGAGTGTCCCATKAC3' (SEQ ID NO 212)
   5'RTGCTGGWGGAGTGTCCCATKAC3' (SEQ ID NO 213)
   5'YRTGCTGGWGGAGTGTCCCATKAC3' (SEQ ID NO 214)
   5'GYRTGCTGGWGGAGTGTCCCATKAC3' (SEQ ID NO 215)
   5'GTCCCATKACAGATRCMMAA3' (SEQ ID NO 216)
   5'TGTCCCATKACAGATRCMMAA3' (SEQ ID NO 217)
   5'GTGTCCCATKACAGATRCMMAA3' (SEQ ID NO 218)
   5'AGTGTCCCATKACAGATRCMMAA3' (SEQ ID NO 219)
   5'GAGTGTCCCATKACAGATRCMMAA3' (SEQ ID NO 220)
   5'GGAGTGTCCCATKACAGATRCMMAA3' (SEQ ID NO 221).

Further disclosed herein are the following primers for the amplification of exon 2 of HLA-B (table 5):
5'ACCCGCGGGGATTTTGGCCTC3' (SEQ ID NO 108)
   5'AACCCGCGGGGATTTTGGCCTC3' (SEQ ID NO 109)
   5'CAACCCGCGGGGATTTTGGCCTC3' (SEQ ID NO 110)
   5'CCAACCCGCGGGGATTTTGGCCTC3' (SEQ ID NO 111)
   5'MCCAACCCGCGGGGATTTTGGCCTC3' (SEQ ID NO 112)
   5'GMCCAACCCGCGGGGATTTTGGCCTC3' (SEQ ID NO 113)
   5'YGMCCAACCCGCGGGGATTTTGGCCTC3' (SEQ ID NO 314)
- for the amplification of exon 3 of HLA-B (table 6; table 7):
   5'CYGGGGCGSAGGTCACGACT3' (SEQ ID NO 114)
   5'CCYGGGGCGSAGGTCACGACT3' (SEQ ID NO 115)
   5'GCCYGGGGCGSAGGTCACGACT3' (SEQ ID NO 116)
   5'GGCCYGGGGCGSAGGTCACGACT3' (SEQ ID NO 117)
   5'CGGCCYGGGGCGSAGGTCACGACT3' (SEQ ID NO 118)
   5'CCGGCCYGGGGCGSAGGTCACGACT3' (SEQ ID NO 119)
   5'CCCGGTTTCATTTTCAGTTG3' (SEQ ID NO 120)
   5'ACCCGGTTTCATTTTCAGTTG3' (SEQ ID NO 121)
   5'TACCCGGTTTCATTTTCAGTTG3' (SEQ ID NO 122)
   5'TTACCCGGTTTCATTTTCAGTTG3' (SEQ ID NO 123)
   5'TTTACCCGGTTTCATTTTCAGTTG3' (SEQ ID NO 124)
   5'GTTTACCCGGTTTCATTTTCAGTTG3' (SEQ ID NO 125)
   5'CGTTTACCCGGTTTCATTTTCAGTTG3' (SEQ ID NO 222)
   5'GCGTTTACCCGGTTTCATTTTCAGTTG3' (SEQ ID NO 223)
   5'CGCGTTTACCCGGTTTCATTTTCAGTTG3' (SEQ ID NO 224)
   5'CGTKGGAGSCCATCCCCGSC3' (SEQ ID NO 225)
   5'TCGTKGGAGSCCATCCCCGSC3' (SEQ ID NO 226)
   5'CTCGTKGGAGSCCATCCCCGSC3' (SEQ ID NO 227)
   5'TCTCGTKGGAGSCCATCCCCGSC3' (SEQ ID NO 228)
   5'TTCTCGTKGGAGSCCATCCCCGSC3' (SEQ ID NO 229)
   5'CTTCTCGTKGGAGSCCATCCCCGSC3' (SEQ ID NO 230)
   5'TCTCGTKGGAGSCCATCCCC3' (SEQ ID NO 231)
   5'TTCTCGTKGGAGSCCATCCCC3' (SEQ ID NO 232)
   5'CTTCTCGTKGGAGSCCATCCCC3' (SEQ ID NO 233)
   5'TCTTCTCGTKGGAGSCCATCCCC3' (SEQ ID NO 234)
   5'YTCTTCTCGTKGGAGSCCATCCCC3' (SEQ ID NO 235)
   S'CYTCTTCTCGTKGGAGSCCATCCCC3' (SEQ ID NO 236)
   5'GATCCCATTTTCCTCYTCTT3' (SEQ ID NO 237)
   5'TGATCCCATTTTCCTCYTCTT3' (SEQ ID NO 238)
   5'CTGATCCCATTTTCCTCYTCTT3' (SEQ ID NO 239)
   5'GCTGATCCCATTTTCCTCYTCTT3' (SEQ ID NO 240)
   5'CGCTGATCCCATTTTCCTCYTCTT3' (SEQ ID NO 241)
   5'GCGCTGATCCCATTTTCCTCYTCTT3' (SEQ ID NO 242)
   5'GCTGATCCCATTTTCCTCYT3' (SEQ ID NO 243)
   5'CGCTGATCCCATTTTCCTCYT3' (SEQ ID NO 244)
   5'GCGCTGATCCCATTTTCCTCYT3' (SEQ ID NO 245)
   5'AGCGCTGATCCCATTTTCCTCYT3' (SEQ ID NO 246)
   5'TAGCGCTGATCCCATTTTCCTCYT3' (SEQ ID NO 247)
   5'CTAGCGCTGATCCCATTTTCCTCYT3' (SEQ ID NO 248)
   5'TCCATTCAAGGGAGGGCGAC3' (SEQ ID NO 249)
   5'CTCCATTCAAGGGAGGGCGAC3' (SEQ ID NO 250)
   5'TCTCCATTCAAGGGAGGGCGAC3' (SEQ ID NO 251)
   5'TTCTCCATTCAAGGGAGGGCGAC3' (SEQ ID NO 252)
   5'ATTCTCCATTCAAGGGAGGGCGAC3' (SEQ ID NO 253)
   5'CATTCTCCATTCAAGGGAGGGCGAC3' (SEQ ID NO 254)
- for the amplification of exon 4 of HLA-B (table 8):
   5'AGATTATCCCAGGTGCCTGC3' (SEQ ID NO 255)
   5'GAGATTATCCCAGGTGCCTGC3' (SEQ ID NO 256)
   5'GGAGATTATCCCAGGTGCCTGC3' (SEQ NO 257)
   5'AGGAGATTATCCCAGGTGCCTGC3' (SEQ ID NO 258)
   5'TAGGAGATTATCCCAGGTGCCTGC3' (SEQ ID NO 259)
   5'ATAGGAGATTATCCCAGGTGCCTGC3' (SEQ ID NO 260)
   5'TGTCCTGYCCATTCTCAGKC3' (SEQ ID NO 261)
   5'GTGTCCTGYCCATTCTCAGKC3' (SEQ ID NO 262)
   5'GGTGTCCTGYCCATTCTCAGKC3' (SEQ ID NO 263)
   5'AGGTGTCCTGYCCATTCTCAGKC3' (SEQ ID NO 264)
   5'CAGGTGTCCTGYCCATTCTCAGKC3' (SEQ ID NO 265)
   5'CCAGGTGTCCTGYCCATTCTCAGKC3' (SEQ ID NO 266)
   5'TCACATGGGTGGTCCTAGG3' (SEQ ID NO 267)
   5'GTCACATGGGTGGTCCTAGG3' (SEQ ID NO 268)
   5'GGTCACATGGGTGGTCCTAGG3' (SEQ ID NO 269)
   5'TGGTCACATGGGTGGTCCTAGG3' (SEQ ID NO 270)
   5'CTGGTCACATGGGTGGTCCTAGG3' (SEQ ID NO 271)
   5'KCTGGTCACATGGGTGGTCCTAGG3' (SEQ ID NO 272)
   5'GKCTGGTCACATGGGTGGTCCTAGG3' (SEQ ID NO 273)
   5'TSCCATGARAGATGCMAAGC3' (SEQ ID NO 274)
   5'GTSCCATGARAGATGCMAAGC3' (SEQ ID NO 275)
   5'TGTSCCATGARAGATGCMAAGC3' (SEQ ID NO 276)
   5'GTGTSCCATGARAGATGCMAAGC3' (SEQ ID NO 277)
   5'GGTGTSCCATGARAGATGCMAAGC3' (SEQ ID NO 278)
   5'GGGTGTSCCATGARAGATGCMAAGC3' (SEQ ID NO 279)
   5'GWAWTTTCTGACTCTTCCCA3' (SEQ ID NO 280)
   5'TGWAWTTTCTGACTCTTCCCA3' (SEQ ID NO 281)
   5'CTGWAWTTTCTGACTCTTCCCA3' (SEQ ID NO 282)
   5'CCTGWAWTTTCTGACTCTTCCCA3' (SEQ ID NO 283)
   5'GCCTGWAWTTTCTGACTCTTCCCA3' (SEQ ID NO 284)
   5'CGCCTGWAWTTTCTGACTCTTCCCA3' (SEQ ID NO 285)
- for the amplification of exon 2 of HLA-C (table 9):
   5'GTCGAGGGTCTGGGCGGGTT3' (SEQ ID NO 126)
   5'GGTCGAGGGTCTGGGCGGGTT3' (SEQ ID NO 127)
   5'CGGTCGAGGGTCTGGGCGGGTT3' (SEQ ID NO 128)
   5'CCGGTCGAGGGTCTGGGCGGGTT3' (SEQ ID NO 129)
   5'YCCGGTCGAGGGTCTGGGCGGGTT3' (SEQ ID NO 130)
   5'CYCCGGTCGAGGGTCTGGGCGGGTT3' (SEQ ID NO 131)
- for the amplification of exon 3 of HLA-C (table 10):
   5'CGCCCCRAGTCTCCSSGTCT3' (SEQ ID NO 132)
   5'TCGCCCCRAGTCTCCSSGTCT3' (SEQ ID NO 133)
   5'GTCGCCCCRAGTCTCCSSGTCT3' (SEQ ID NO 134)
   5'GGTCGCCCCRAGTCTCCSSGTCT3' (SEQ ID NO 135)
   5'GGGTCGCCCCRAGTCTCCSSGTCT3' (SEQ ID NO 136)
   5'CGGGTCGCCCCRAGTCTCCSSGTCT3' (SEQ ID NO 137)
   5'CGRCCGGRGAGAGCCCCAGT3' (SEQ ID NO 138)
   5'TCGRCCGGRGAGAGCCCCAGT3' (SEQ ID NO 139)
   5'CTCGRCCGGRGAGAGCCCCAGT3' (SEQ ID NO 140)
   5'CCTCGRCCGGRGAGAGCCCCAGT3' (SEQ ID NO 141)
   5'CCCTCGRCCGGRGAGAGCCCCAGT3' (SEQ ID NO 142)
   5'ACCCTCGRCCGGRGAGAGCCCCAGT3' (SEQ ID NO 143)
- for the amplification of exon 4 of HLA-C (table 11):
   5'GTGCCTGTGTCCAGGCTGGC3' (SEQ ID NO 286)
   5'GGTGCCTGTGTCCAGGCTGGC3' (SEQ ID NO 287)
   5'AGGTGCCTGTGTCCAGGCTGGC3' (SEQ ID NO 288)
   5'CAGGTGCCTGTGTCCAGGCTGGC3' (SEQ ID NO 289)
   5'CCAGGTGCCTGTGTCCAGGCTGGC3' (SEQ ID NO 290)
   5'CCCAGGTGCCTGTGTCCAGGCTGGC3' (SEQ ID NO 291)
   5'TGGCGTCTGGGTTCTGTGCC3' (SEQ ID NO 292)
   5'CTGGCGTCTGGGTTCTGTGCC3' (SEQ ID NO 293)
   5'GCTGGCGTCTGGGTTCTGTGCC3' (SEQ ID NO 294)
   5'GGCTGGCGTCTGGGTTCTGTGCC3' (SEQ ID NO 295)
   5'AGGCTGGCGTCTGGGTTCTGTGCC3' (SEQ ID NO 296)
   5'CAGGCTGGCGTCTGGGTTCTGTGCC3' (SEQ ID NO 297)
   5'CTCAGGATRGTCACATGGSC3' (SEQ ID NO 298)
   5'TCTCAGGATRGTCACATGGSC3' (SEQ ID NO 299)
   5'TTCTCAGGATRGTCACATGGSC3' (SEQ ID NO 300)
   5'RTTCTCAGGATRGTCACATGGSC3' (SEQ ID NO 301)
   5'CRTTCTCAGGATRGTCACATGGSC3' (SEQ ID NO 302)
   5'CCRTTCTCAGGATRGTCACATGGSC3' (SEQ NO 303)
   5'GCGCTGTTGGAGTGTCGCAA3' (SEQ ID NO 78)
   5'GGCGCTGTTGGAGTGTCGCAA3' (SEQ ID NO 79)
   5'GGGCGCTGTTGGAGTGTCGCAA3' (SEQ ID NO 80)
   5'TGGGCGCTGTTGGAGTGTCGCAA3' (SEQ ID NO 81)
   5'ATGGGCGCTGTTGGAGTGTCGCAA3' (SEQ ID NO 82)
   5'CATGGGCGCTGTTGGAGTGTCGCAA3' (SEQ ID NO 83)
   5'SCAAGAGAGAWRCAAAGTGT3' (SEQ ID NO 304)
   5'CSCAAGAGAGAWRCAAAGTGT3' (SEQ ID NO 305)
   5'TCSCAAGAGAGAWRCAAAGTGT3' (SEQ ID NO 306)
   5'GTCSCAAGAGAGAWRCAAAGTGT3' (SEQ ID NO 307)
   5'TGTCSCAAGAGAGAWRCAAAGTGT3' (SEQ ID NO 308)
   5'GTGTCSCAAGAGAGAWRCAAAGTGT3' (SEQ ID NO 309)

In another preferred embodiment, the present invention relates to any method as described above, further characterized that the amplification of exon 2 is carried out with at least one of the following forward primers:
- for HLA-A: 5APBio: B-TTCTCCCCAGACGCCGAGGATGGCC (SEQ ID NO 144).

In another preferred embodiment, the present invention relates to any method as described above, further characterized that the amplification of exon 4 is carried out with at least one of the following reverse primers:
- for HLA-A: 3ex4APbio: B-TTGGGCAGACCCTCATGCTGC (SEQ ID NO 311).

In a very specific embodiment, the present invention relates to any method as described above, further characterized that:
- the amplification of exon 2 is carried out with at least one of the following primers sets:
   - for HLA-A: 5APbio (B-TTCTCCCCAGACGCCGAGGATGGCC; SEQ ID NO 144) and 3ex2APbio (B-ATCTCGGACCCGGAGACTGT; SEQ ID NO 1).
- the amplification of exon 3 is carried out with at least one of the following primer sets:
   - for HLA-A: 5ex3APbio (B-CAGTTTAGGCCAAAAATCCCCC: SEQ ID NO 104) and 3ex3APbio (B-CCCTCCTTGTGGGAGGCCAG; SEQ ID NO 156);
- the amplification of exon 4 is carried out with at least one of the following primer sets:
   - for HLA-A: 5ex4APbio (B-GTTCTGTGCTCYCTTCCCCAT; SEQ ID NO 205) and 3ex4APbio (B-TTGGGCAGACCCTCATGCTGC ; SEQ ID NO 311).

The skilled man will understand that these primers (SEQ ID NOs 1 to 314) may be adapted by addition or deletion of one or more nucleotides at their extremities. Such adaptations may be required, for instance, if the conditions of amplification are changed, if the amplified material is RNA instead of DNA, as is the case, for example, in the NASBA system.

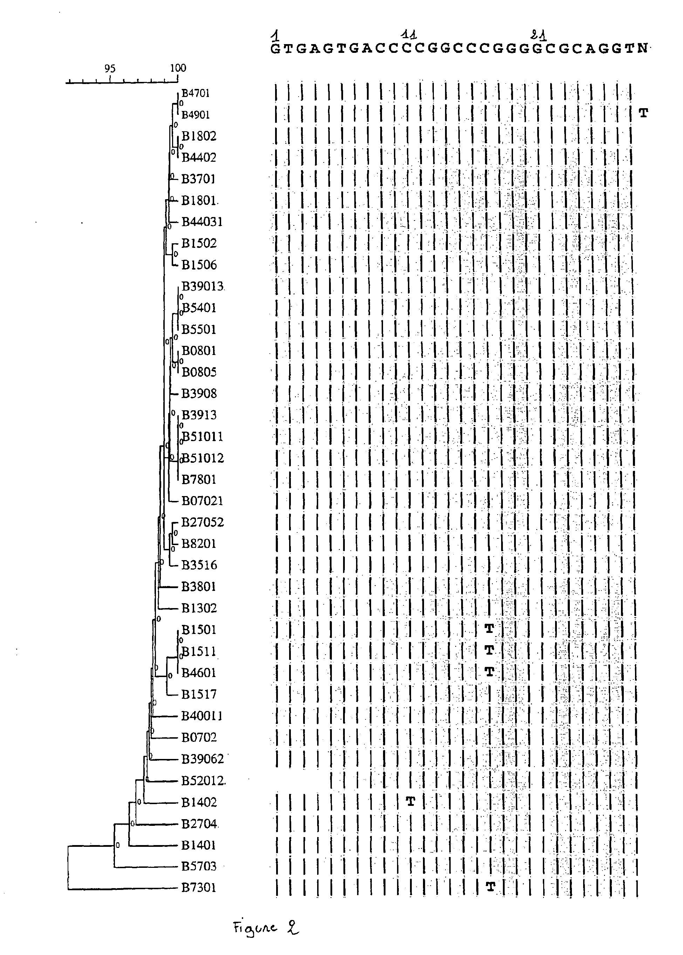

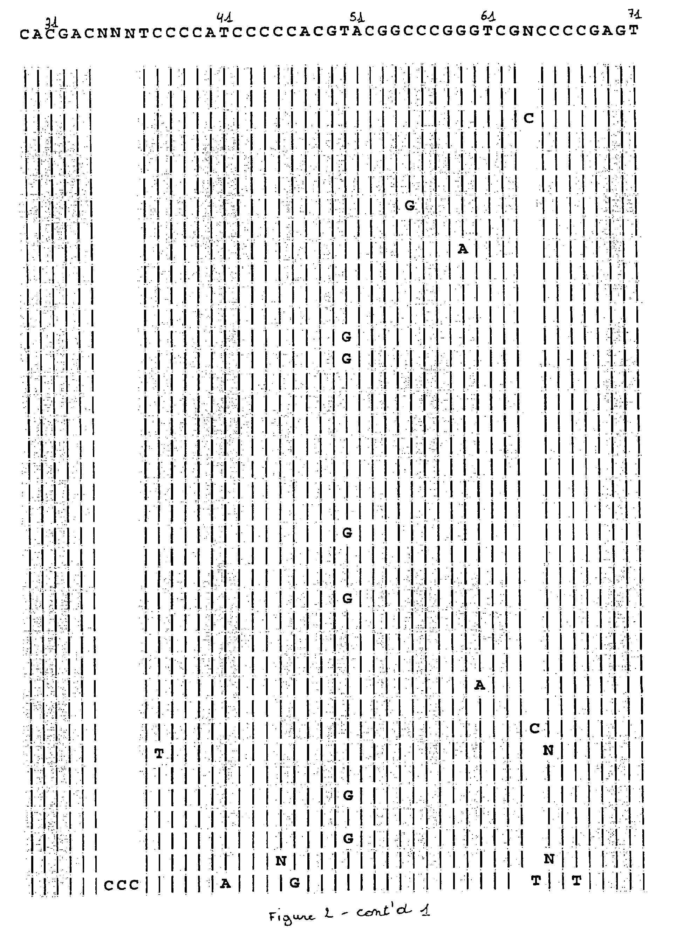

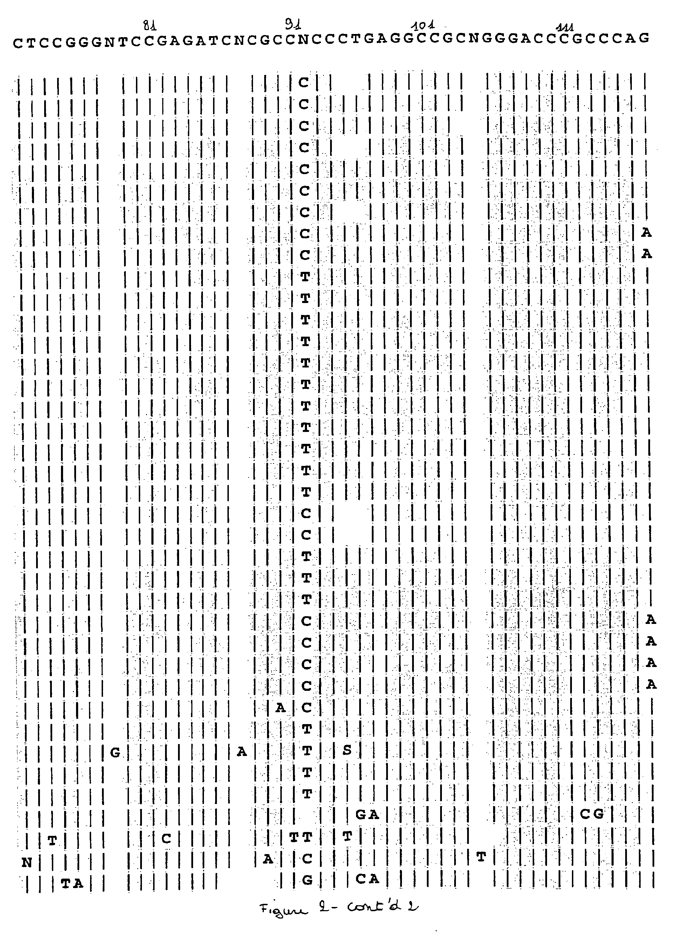

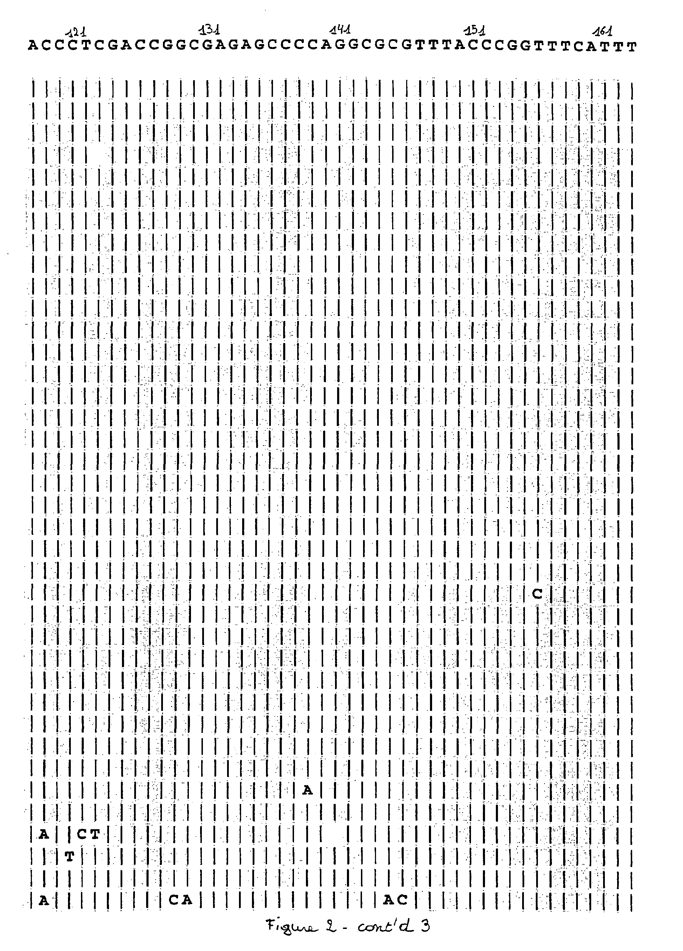

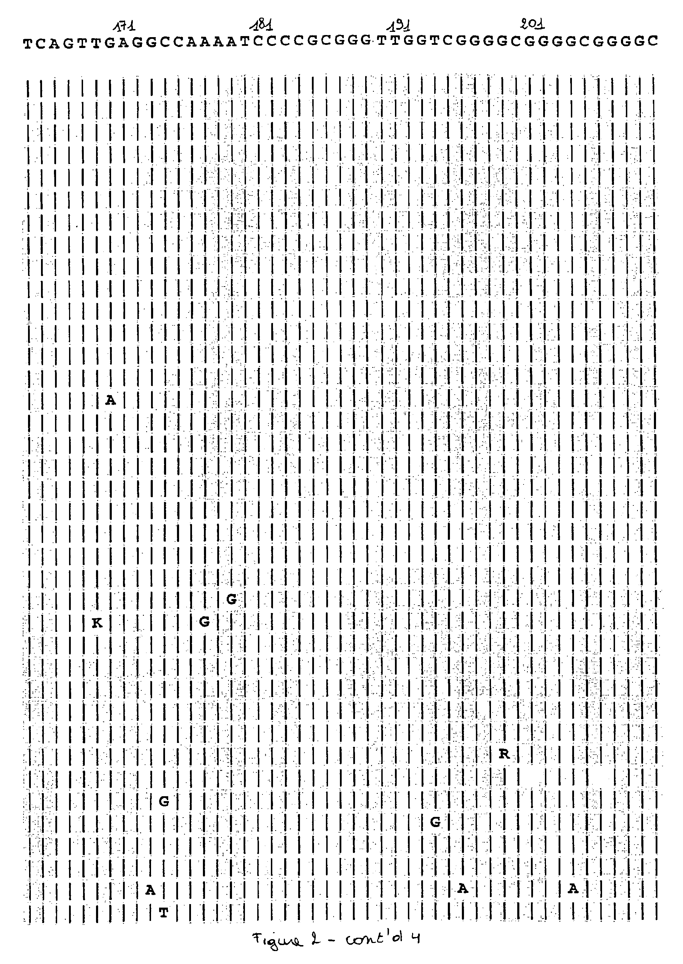

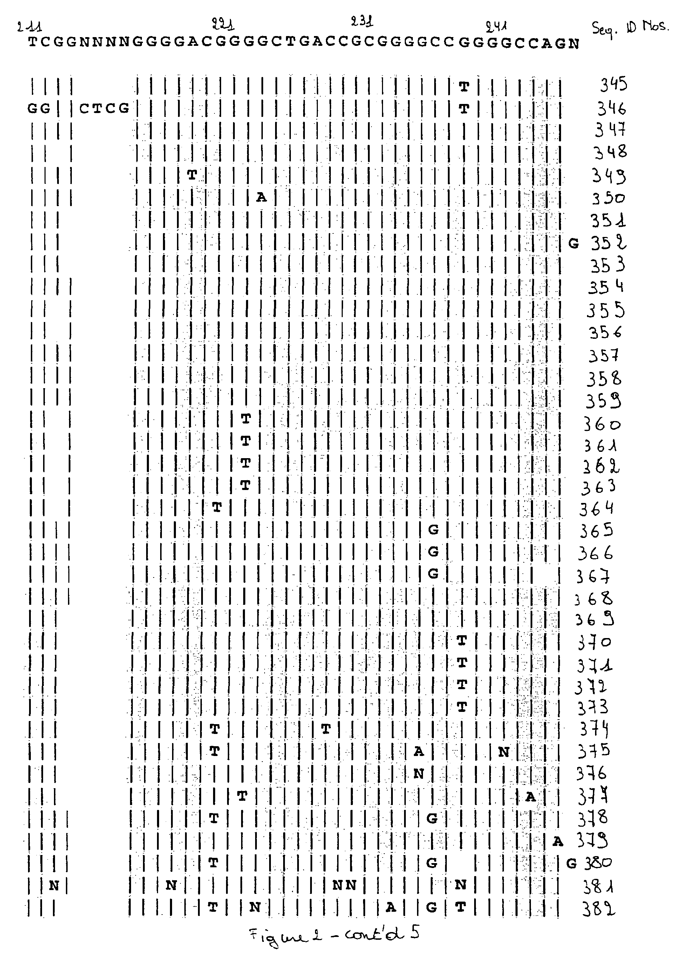

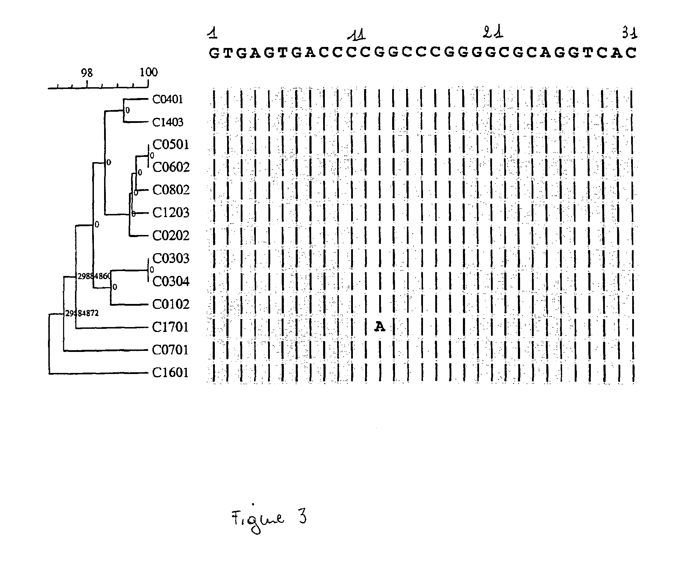

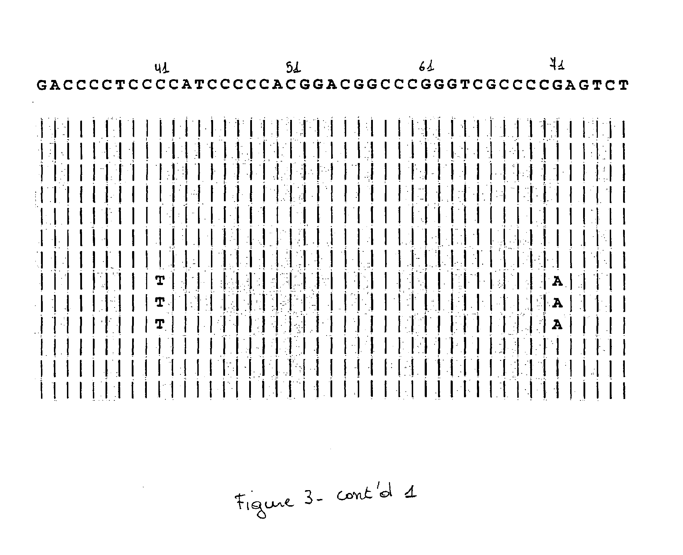

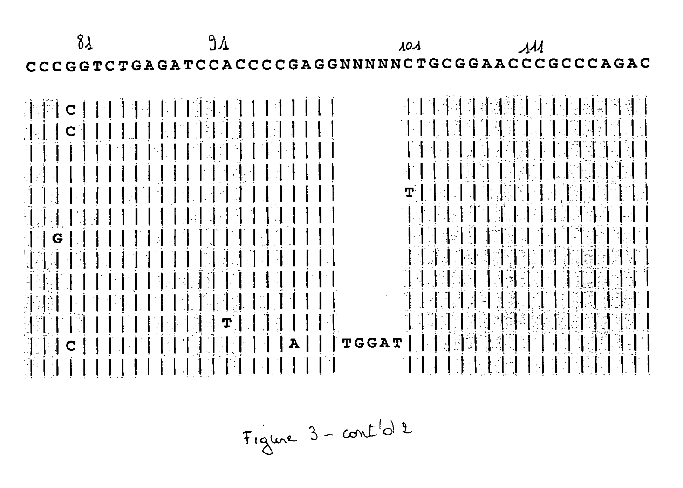

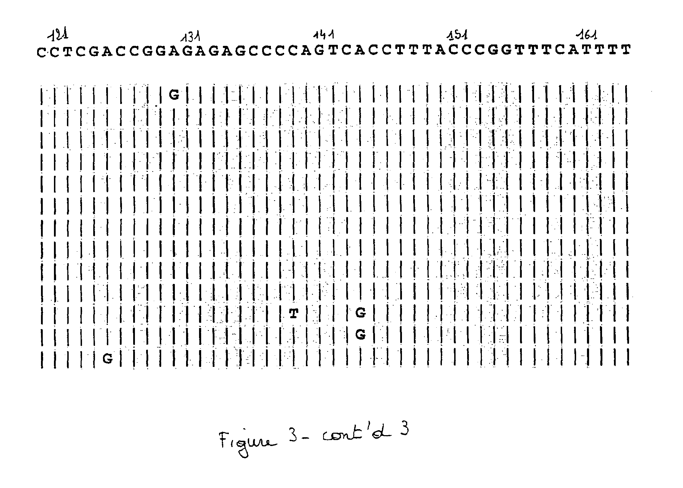

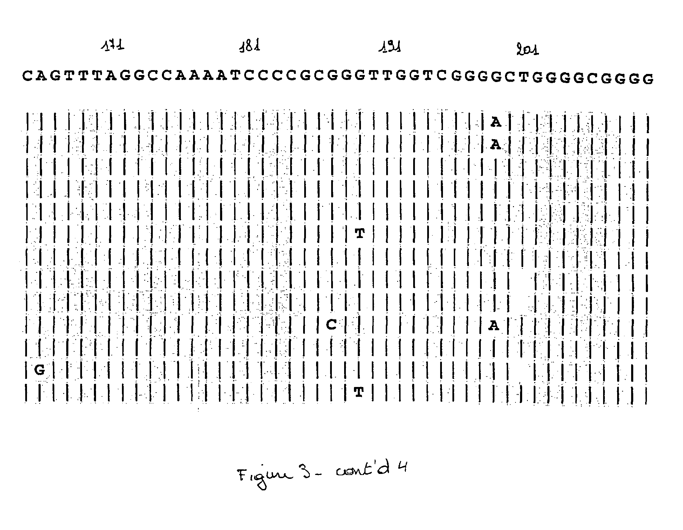

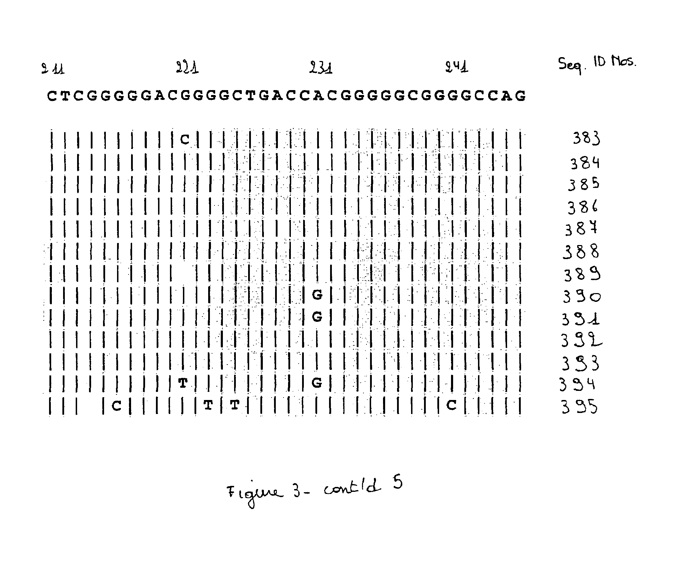

Further disclosed herein is a primer as described above, for use in the amplification of exon 2 of HLA-A, HLA-B or HLA-C alleles, said primer specifically hybridizing to a locus-specific target sequence in intron 2 of respectively HLA-A, HLA-B or HLA-C; more specifically, said primer specifically hybridizing to a locus-specific target sequence situated at position:
- 67, 96, 109, 110, 118, 123, 131 or 181 of the HLA-A intron 2 (Fig. 1); or
- 35 or 170 of the HLA-B intron 2 (Fig. 2); or
- 84. 107 or 142 of the HLA-C intron 2 (Fig. 3);
more specifically, said primer being chosen from table 1 (for the amplification of exon 2 of HLA-A), from table 5 (for the amplification of exon 2 of HLA-B) or from table 9 (for the amplification of exon 2 of HLA-C).

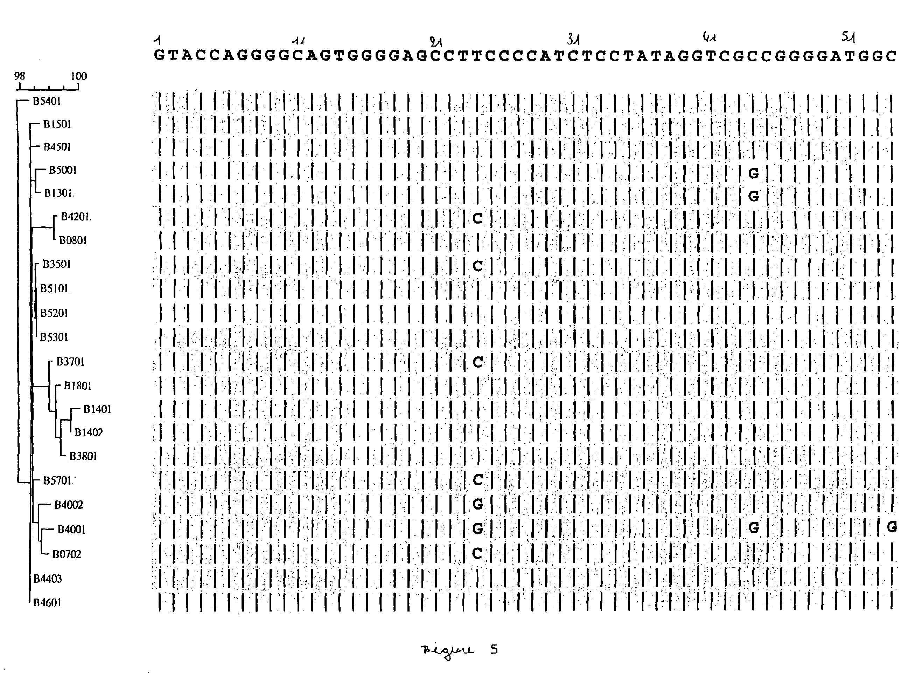

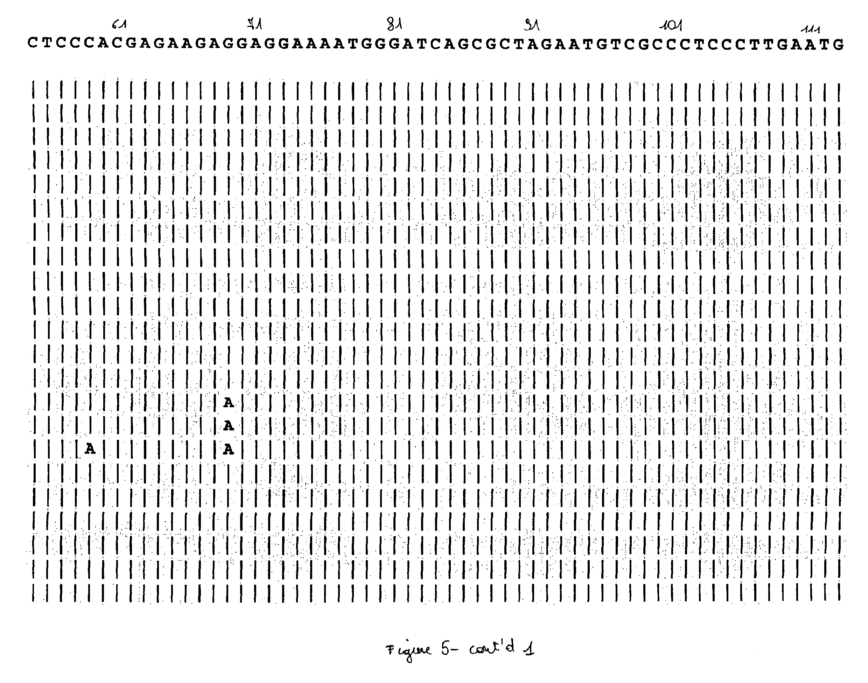

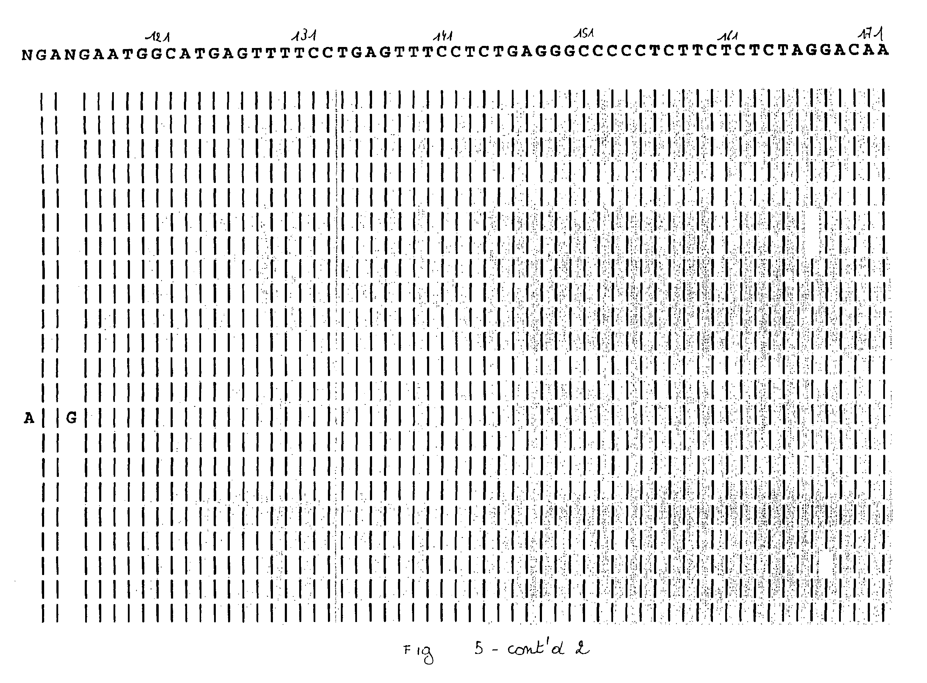

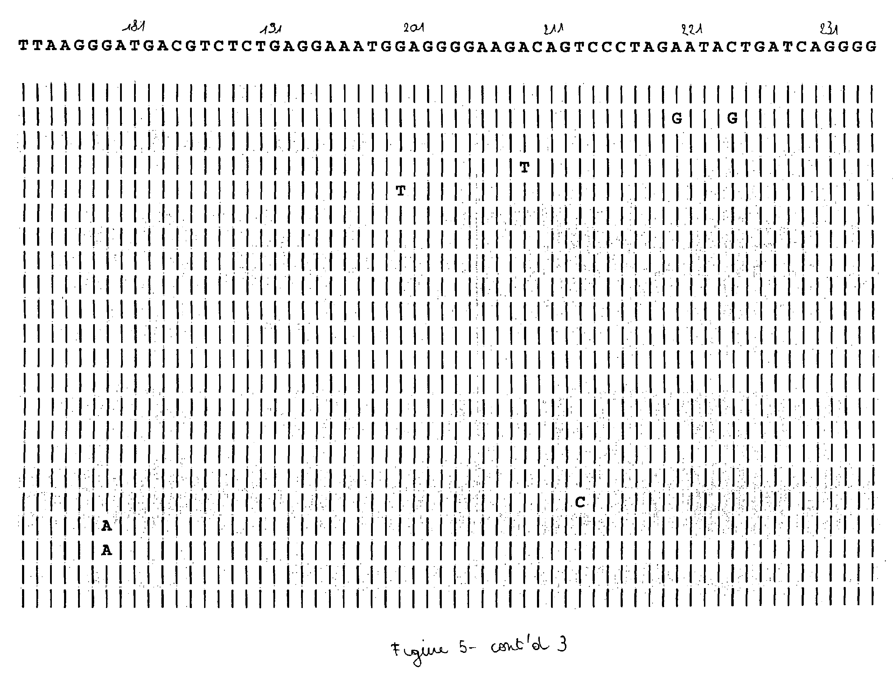

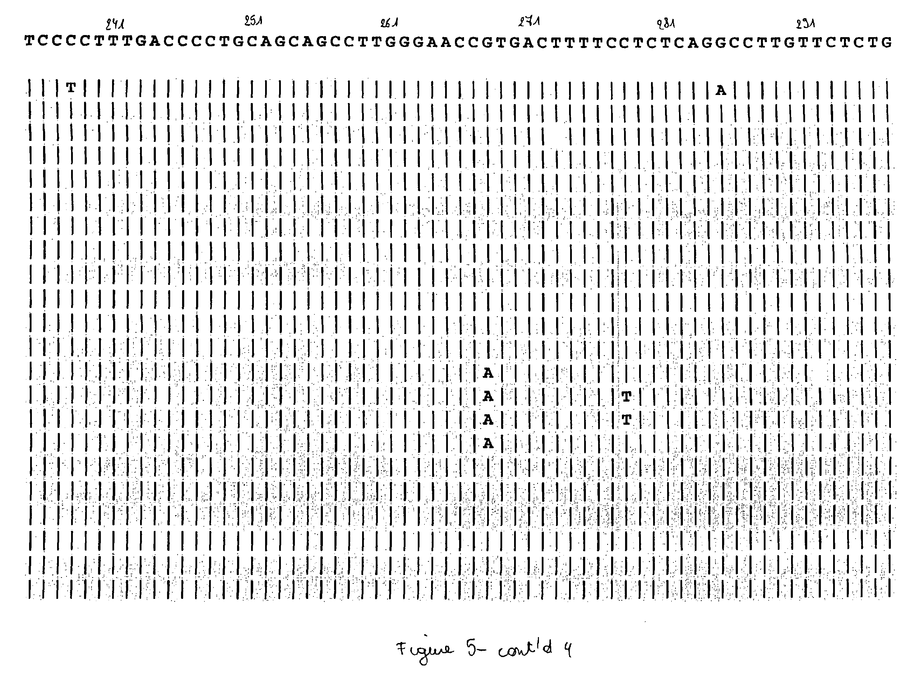

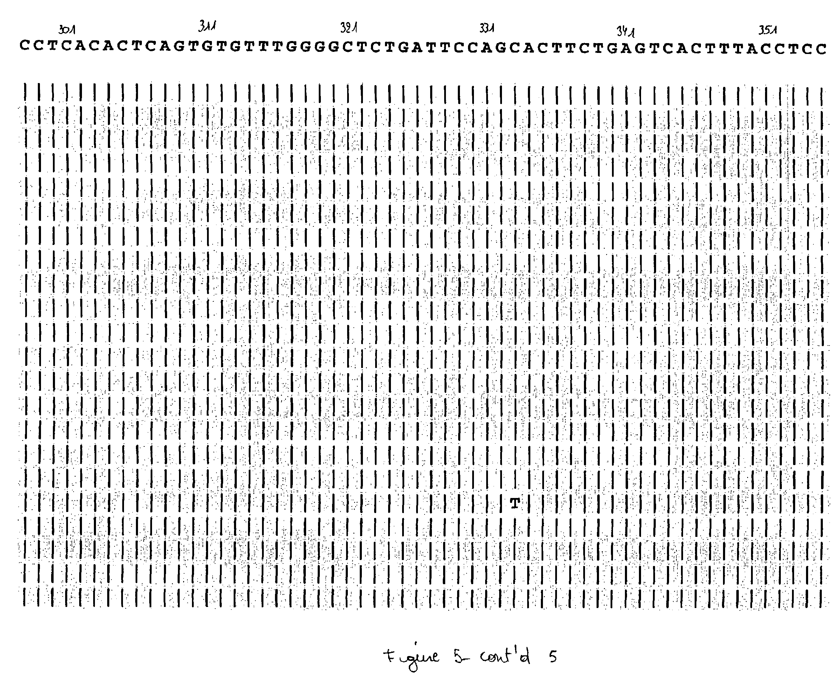

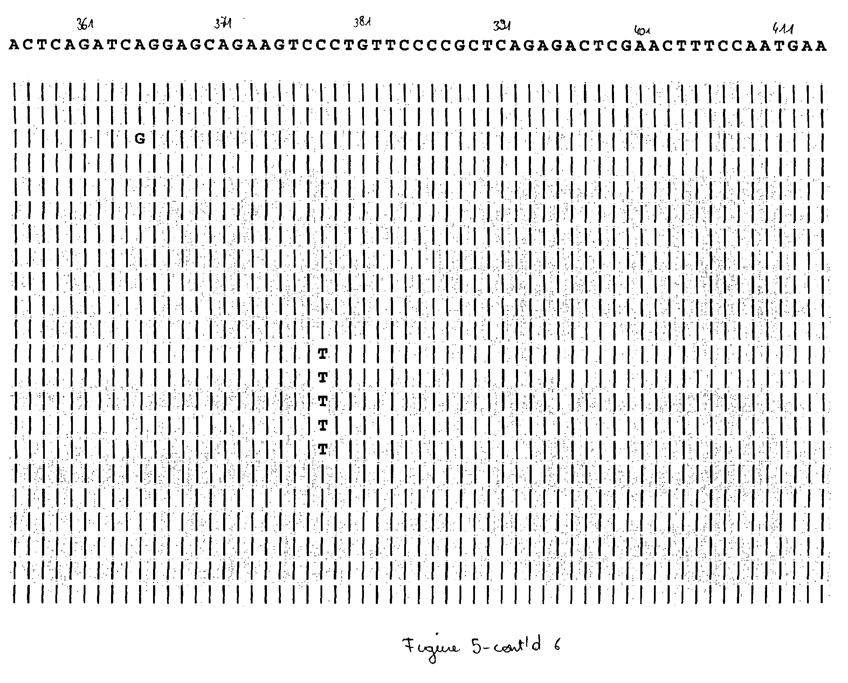

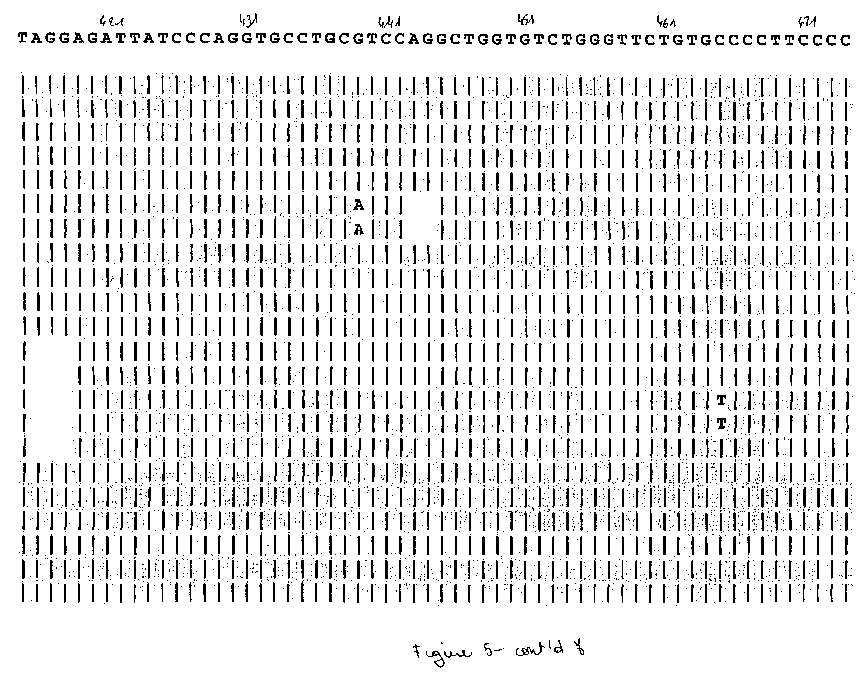

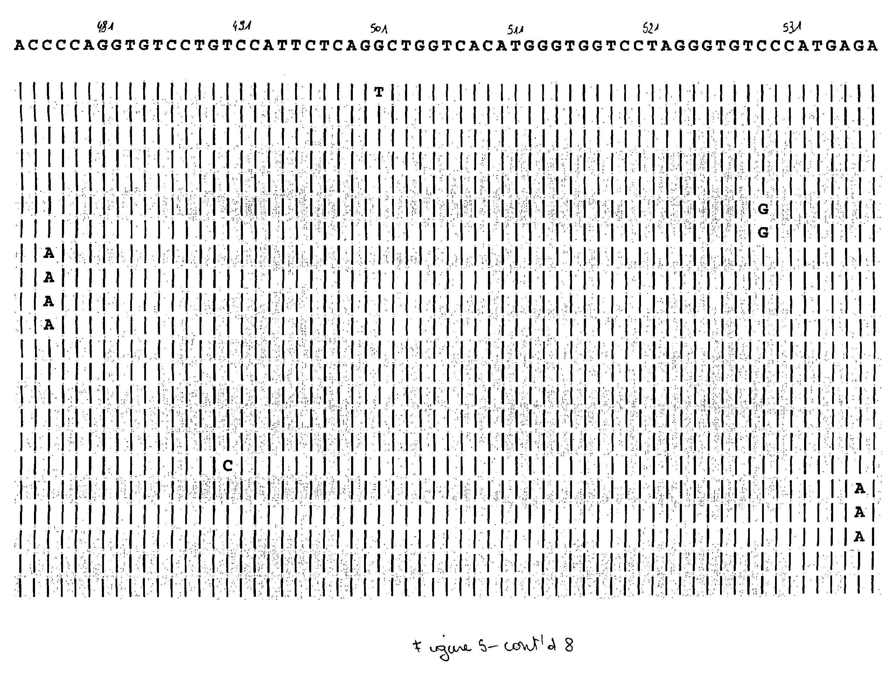

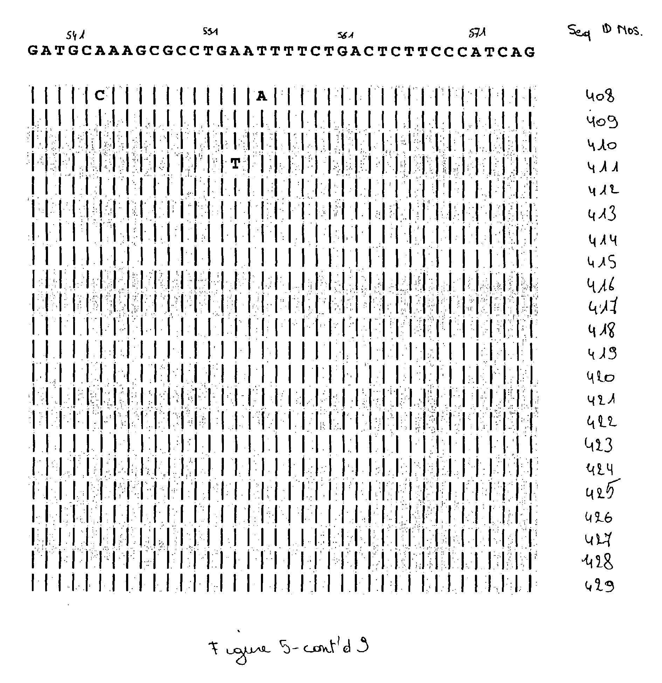

Further disclosed is a primer as described above, for use in the amplification of exon 3 of HLA-A, HLA-B or HLA-C alleles, said primer specifically hybridizing to a locus-specific target sequence in intron 2 (forward primer) or to a locus-specific target sequence in intron 3 (reverse primer) of respectively HLA-A, HLA-B or HLA-C; more specifically, said primer specifically hybridizing to a locus-specific target sequence situated at position:
- 67, 96, 109, 110, 118, 123, 131 or 181 of the HLA-A intron 2 (Fig. 1) or 32, 50, 62, 73, 83, 86, 118, 130, 150 of the HLA-A intron 3 (Fig. 4); or
- 35 or 170 of the HLA-B intron 2 (Fig. 2) or 42, 46, 65, 68, 96, of the HLA-B intron 3 (Fig. 5); or
- 84, 107 or 142 of the HLA-C intron 2 (Fig. 3).
more specifically, said primer being chosen from table 2 or table 3 (for the amplification of exon 3 of HLA-A) or from table 6 or table 7 (for the amplification of exon 3 of HLA-B).

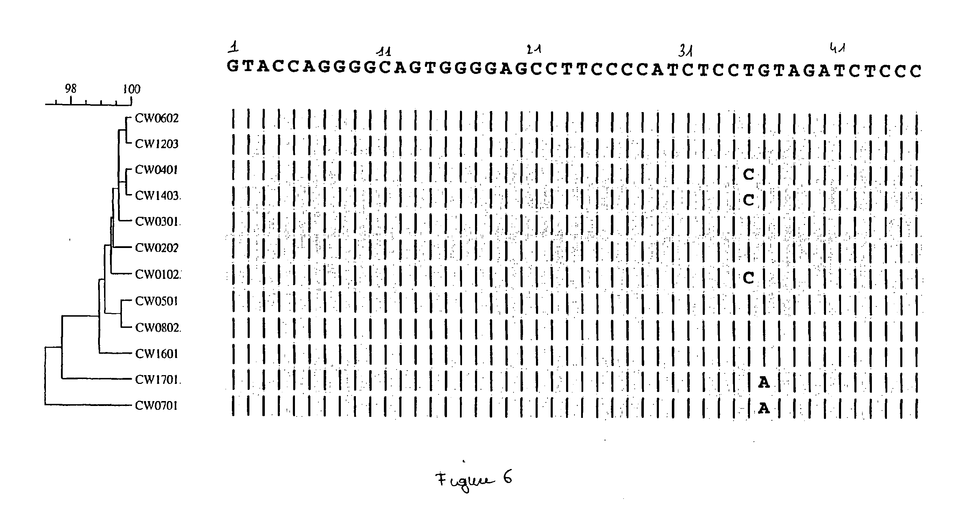

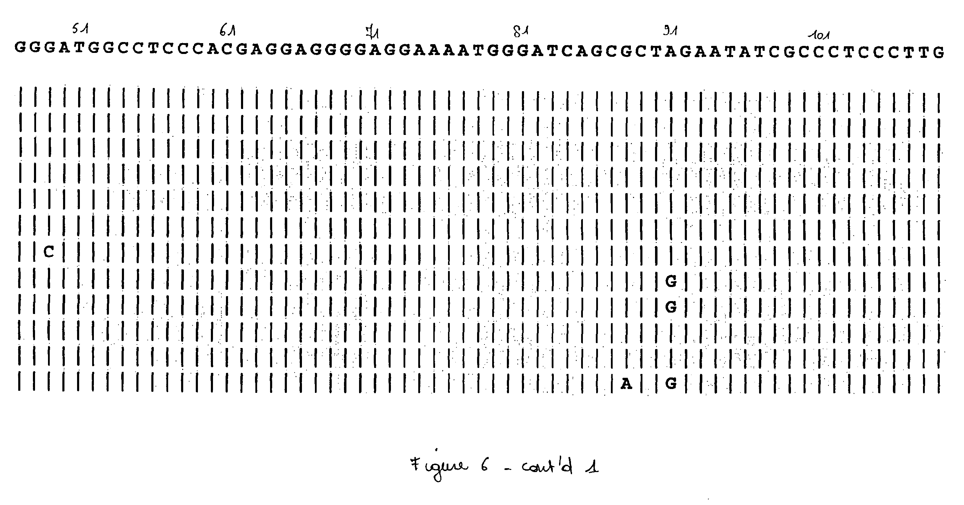

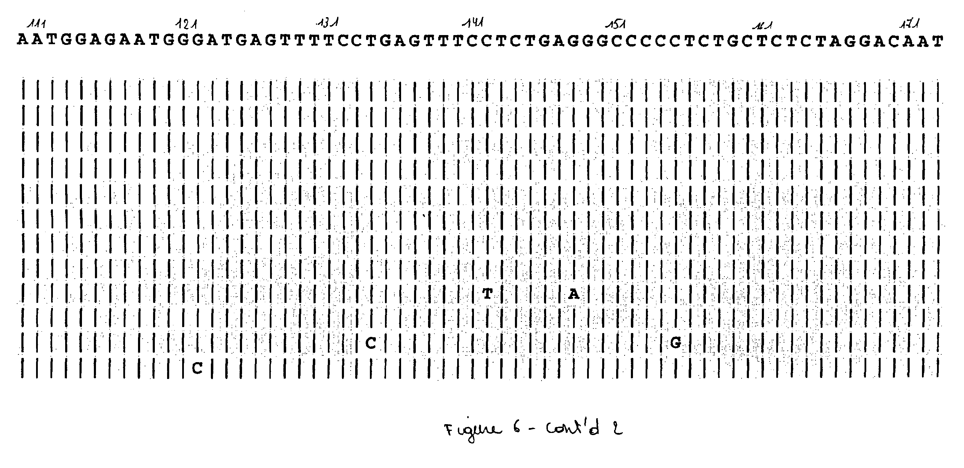

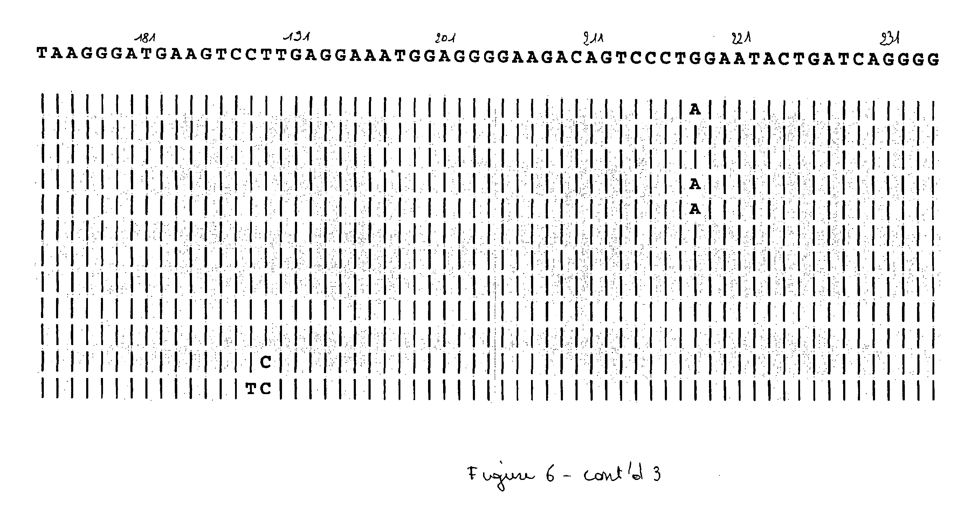

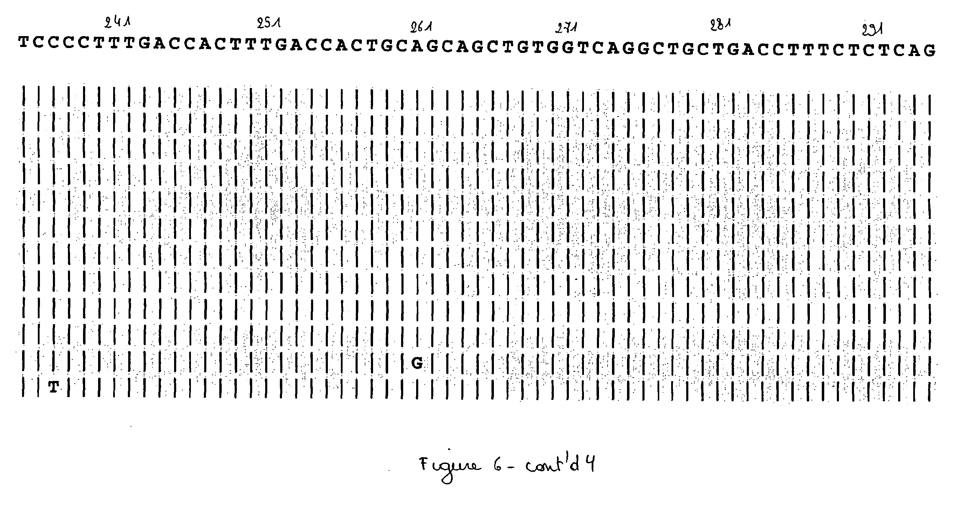

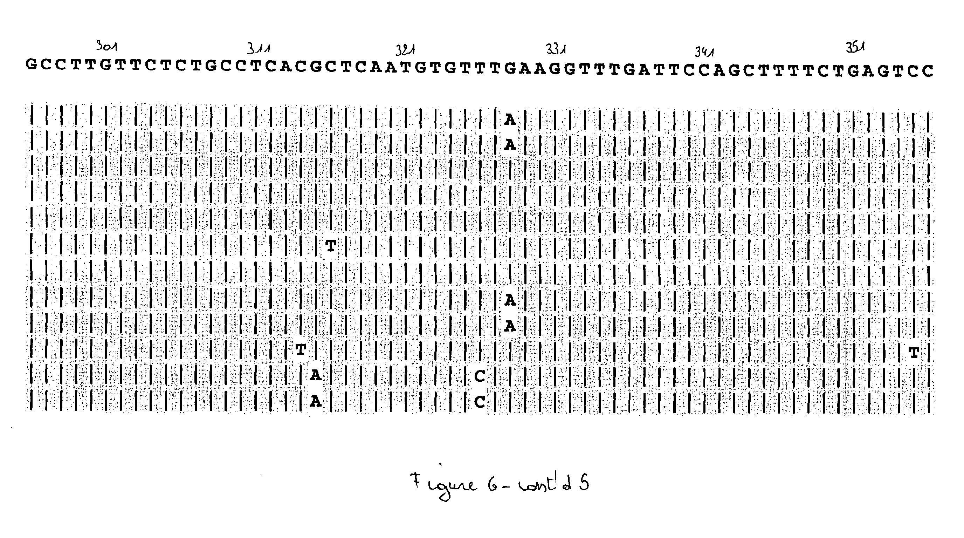

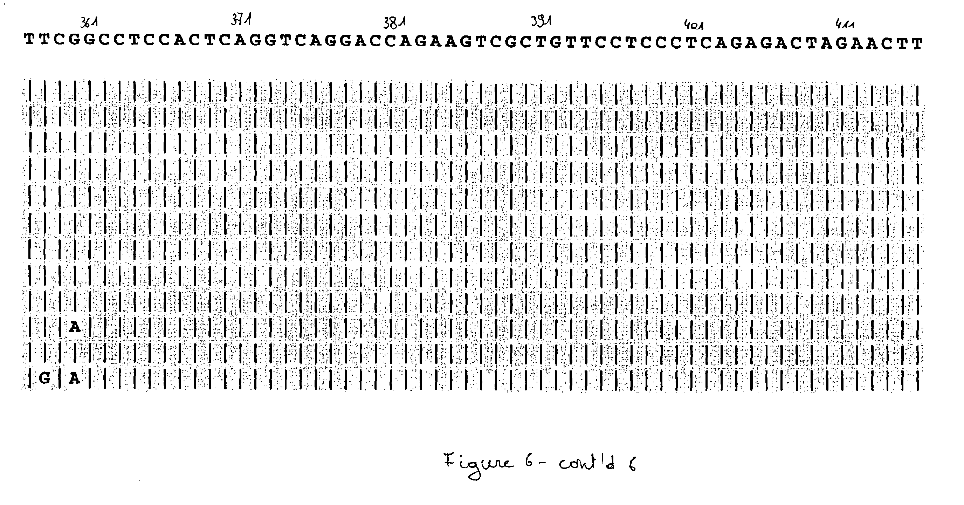

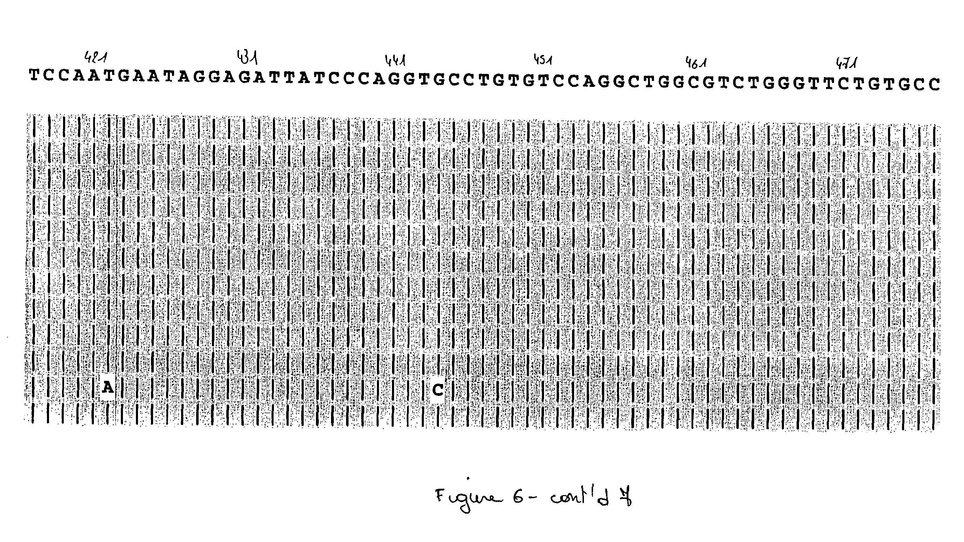

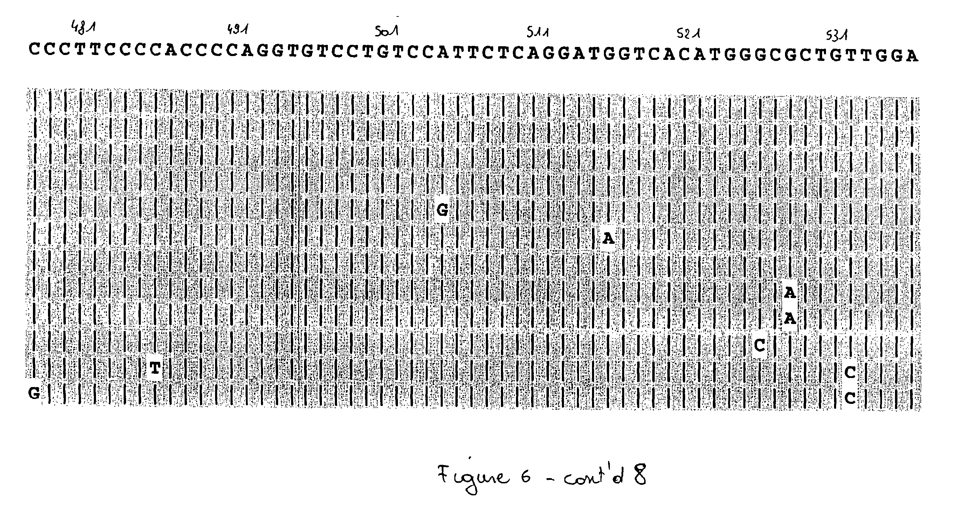

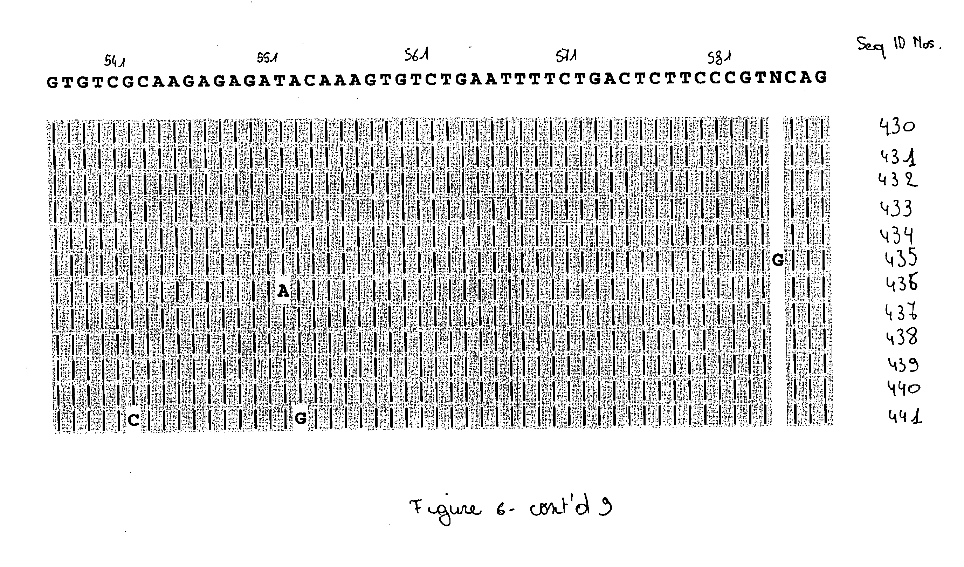

Further disclosed is a primer as described above, for use in the amplification of exon 4 of HLA-A, HLA-B or HLA-C alleles, said primer specifically hybridizing to a locus-specific target sequence in intron 3 of respectively HLA-A, HLA-B or HLA-C; more specifically, said primer specifically hybridizing to a locus-specific target sequence situated at position:
- 501, 525, 561 or 571 of the HLA-A intron 3 (Fig. 4); or
- 438, 502, 524, 547 or 571 of the HLA-B intron 3 (Fig. 5); or
- 461, 477, 527, 545 or 561 of the HLA-C intron 3 (Fig. 6).
more specifically, said primer being chosen from table 4 (for the amplification of exon 4 of HLA-A), from table 8 (for the amplification of exon 4 of HLA-B) or from table 11 (for the amplification of exon 4 of HLA-C).

Further disclosed is a primer set consisting of a combination of a forward and a reverse primer as defined above, for use in the amplification of exon 2 of HLA-A, HLA-B or HLA-C alleles. Specifically disclosed is the combination of the forward primer 5APBio (SEQ ID NO 144) for HLA-A, IBPin1 (SEQ ID NO 145) for HLA-B or 5CIN1 (SEQ ID NO 146) for HLA-C and a reverse primer specifically hybridizing to a locus-specific target sequence in intron 2 of respectively HLA-A, HLA-B or HLA-C; more specifically, said reverse primer specifically hybridizing to a locus-specific target sequence situated at position:
- 67, 96, 109, 110, 118, 123, 131 or 181 of the HLA-A intron 2; or
- 35 or 170 of the HLA-B intron 2; or
- 84, 107 or 142 of the HLA-C intron 2;
more specifically, said reverse primer being chosen from table 1 (for the amplification of exon 2 of HLA-A), from table 5 (for the amplification of exon 2 of HLA-B) or from table 9 (for the amplification of exon 2 of HLA-C).

Also disclosed is a primer set consisting of a combination of a forward and a reverse primer as described above, for use in the amplification of exon 3 of HLA-A, HLA-B or HLA-C alleles. Specifically disclosed is the combination of a forward primer specifically hybridizing to a locus-specific target sequence in intron 2 of respectively HLA-A, HLA-B or HLA-C; more specifically, said forward primer specifically hybridizing to a locus-specific target sequences situated at position:
- 67, 96, 109, 110, 118, 123, 131 or 181 of the HLA-A intron 2; or
- 35 or 170 of the HLA-B intron 2; or
- 84, 107 or 142 of the HLA-C intron 2;
more specifically, said forward primer being chosen from table 2 (for the amplification of exon 3 of HLA-A), from table 6 (for the amplification of exon 3 of HLA-B) or from table 10 (for the amplification of exon 3 of HLA-C) and a reverse primer specifically hybridizing to a locus-specific target sequence in intron 3 of respectively HLA-A, HLA-B or HLA-C: more specifically, said reverse primer specifically hybridizing to a locus-specific target sequences situated at position:
- 32, 50, 62, 73, 83, 86, 118, 130, 150 (Fig. 4) of the HLA-A intron 3: or
- 42, 46, 65, 68, 96, of the HLA-B intron 3 (Fig. 5);
more specifically, said reverse primer being chosen from table 3 (for the amplification of exon 3 of HLA-A), from table 7 (for the amplification of exon 3 of HLA-B) or from 3CIN3 (SEQ ID NO 149) (for the amplification of exon 3 of HLA-C).

Further disclosed is a primer set consisting of a combination of a forward and a reverse primer as described above, for use in the amplification of exon 4 of HLA-A, HLA-B or HLA-C alleles. Specifically disclosed is the combination of a forward primer specifically hybridizing to a locus-specific target sequence in intron 3 of respectively HLA-A, HLA-B or HLA-C; more specifically, said forward primer specifically hybridizing to a locus-specific target sequences situated at position:
- 501, 525, 561 or 571 of the HLA-A intron 3 (Fig. 4); or
- 438, 502, 524, 547 or 571 of the HLA-B intron 3 (Fig. 5); or
- 461, 477, 527, 545 or 561 of the HLA-C intron 3 (Fig. 6).
more specifically, said forward primer being chosen from table 4 (for the amplification of exon 4 of HLA-A), from table 8 (for the amplification of exon 4 of HLA-B) or from table 11 (for the amplification of exon 4 of HLA-C) and a reverse primer 3ex4APbio (SEQ ID NO 311) for HLA-A, 3ex4IBbio (SEQ ID NO 312) for HLA-B or 3ex4ICbio (SEQ ID NO 313) for HLA-C.

Specifically the primers are used in a mix that allows the separate amplification of exon 2 and exon 3. Accordingly, disclosed is a multiplex primer mix containing at least one primer pair as described above for the amplification of exon 2 and one primer pair as described above for the amplification of exon 3.

Specifically, the multiplex primer mix for the separate amplification of exon 2 and exon 3 comprises the following primer sets:
- for the separate amplification of exon 2 and exon 3 of HLA-A:

| **Amplicon** | **Primerset** | **Sequence (5'-3')** | **SEQ ID** |
|---|---|---|---|
| Exon 1-2 | 5APbio(5') | B-TTCTCCCCAGACGCCGAGGATGGCC | 144 |
| | 3ex2APbio (3') | B-ATCTCGGACCCGGAGACTGT | 1 |
| Exon 3 | 5ex3APbio(5') | B-CAGTTTAGGCCAAAAATCCCCC | 104 |
| | 3ex3APbio (3') | B-CCCTCCTTGTGGGAGGCCAG | 156 |

- for the separate amplification of exon 2 and exon 3 of HLA-B:

| **Amplicon** | **Primerset** | **Sequence (5'-3')** | **SEQ ID** |
|---|---|---|---|
| Exon 2 | IBPin1bio (5') | B-GGGAGGAGCGAGGGGACCSCAG | 145 |
| | IB3Pin2bio (3') | B-AACCCGCGGGGATTTTGGCCTC | 109 |
| Exon 3 | IB5Pin2bio (5') | B-CGCGTTTACCCGGTTTCATTTTCAGTTG | 224 |
| | IB3Pin3bio (3') | B-TCTTCTCGTKGGAGSCCATCCCC | 234 |

- for the separate amplification of exon 2 and exon 3 of HLA-C:

| **Amplicon** | **Primerset** | **Sequence (5'-3')** | **SEQ ID** |
|---|---|---|---|
| Exon 2 | 5CIN1(5') | B-AGCGAGGGGCCCGCCCGGCGA | 146 |
| | IC3Pin2bio(3') | B-GGTCGAGGGTCTGGGCGGGTT | 127 |
| Exon 3 | IC5Pin2bio(5') | B-TCGRCCGGRGAGAGCCCCAGT | 139 |
| | 3CIN3 (3') | B-GGAGATGGGGAAGGCTCCCCACT | 149 |

Specifically, the primers are used in a mix that allows the separate amplification of exon 2, exon 3 and exon 4. Accordingly, disclosed is a multiplex primer mix containing at least one primer pair as described above for amplification of exon 2, one primer pair as described above for the amplification of exon 3 and one primer pair as described above for the amplification of exon 4.

Specifically, the multiplex primer mix for the separate amplification of exon 2, exon 3 and exon 4 comprises the following primer sets:
- for the separate amplification of exon 2, exon 3 and exon 4 of HLA-A:

| **Amplicon** | **Primerset** | **Sequence (5'-3')** | **SEQ ID** |
|---|---|---|---|
| Exon 1-2 | 5APbio(5') | B-TTCTCCCCAGACGCCGAGGATGGCC | 144 |
| | 3ex2APbio (3') | B-ATCTCGGACCCGGAGACTGT | 1 |
| Exon 3 | 5ex3APbio(5') | B-CAGTTTAGGCCAAAAATCCCCC | 104 |
| | 3ex3APbio (3') | B-CCCTCCTTGTGGGAGGCCAG | 156 |
| Exon 4 | 5ex4APbio (5') | B-GTTCTGTGCTCYCTTCCCCAT | 205 |
| | 3ex4APbio (3') | B-TTGGGCAGACCCTCATGCTGC | 311 |

- for the separate amplification of exon 2, exon 3 and exon 4 of HLA-B:

| **Amplicon** | **Primerset** | **Sequence (5'-3')** | **SEQ ID** |
|---|---|---|---|
| Exon 2 | IBPinlbio (5') | B-GGGAGGAGCGAGGGGACCSCAG | 145 |
| | IB3Pin2bio (3') | B-AACCCGCGGGGATTTTGGCCTC | 109 |
| Exon 3 | IB5Pin2bio(5') | B-CGCGTTTACCCGGTTTCATTTTCAGTTG | 224 |
| | IB3Pin3bio (3') | B-TCTTCTCGTKGGAGSCCATCCCC | 234 |
| Exon 4 | 5ex4IBbio(5') | B-TCACATGGGTGGTCCTAGG | 267 |
| | 3ex4IBbio (3') | B-TCGGCAGCCCCTCATGCTGT | 312 |

- for the separate amplification of exon 2, exon 3 and exon 4 of HLA-C:

| **Amplicon** | **Primerset** | **Sequence (5'-3')** | **SEQ ID** |
|---|---|---|---|
| Exon 2 | 5CIN1(5') | B-AGCGAGGGGCCCGCCCGGCGA | 146 |
| | IC3Pin2bio(3') | B-GGTCGAGGGTCTGGGCGGGTT | 127 |
| Exon 3 | IC5Pin2bio(5') | B-TCGRCCGGRGAGAGCCCCAGT | 139 |
| | 3CIN3 (3') | B-GGAGATGGGGAAGGCTCCCCACT | 149 |
| Exon 4 | 5ex4ICbio(5') | B-TCTCAGGATRGTCACATGGSC | 299 |
| | 3ex4ICbio(3') | B-CATCTCAGGGTGMRGGGCTT | 313 |

The primers may be labeled. Labeling may be carried out by any method known to the person skilled in the art. The nature of the label may be isotopic (³²P, ³⁵S, etc.) or non-isotopic (biotin, digoxigenin, etc.).

The oligonucleotides used as primers may also comprise nucleotide analogues such as phosphorothiates (Matsukura et al., 1987), alkylphosphorothiates (Miller et al., 1979) or peptide nucleic acids (Nielsen et al., 1991; Nielsen et al., 1993) or may contain intercalating agents (Asseline et al., 1984). As most other variations or modifications introduced into the original DNA sequences, these variations will necessitate adaptions with respect to the conditions under which the oligonucleotide should be used to obtain the required specificity and sensitivity. However, the eventual results of hybridization will be essentially the same as those obtained with the unmodified oligonucleotides. The introduction of these modifications may be advantageous in order to positively influence characteristics such as hybridization kinetics, reversibility of the hybrid-formation, biological stability of the oligonucleotide molecules, etc.

Disclosed herein is the use of the primers, the primer sets and/or the primer mixes of the invention in a method for the locus-specific and separate amplification of exon 2, exon 3 and/or exon 4 of HLA-A, HLA-B or HLA-C.

The present invention also relates to a method for typing or subtyping of one or more HLA-A alleles in a sample comprising the following steps:
(i) if needed, release, isolation and/or concentration of the nucleic acids present in said sample;
(ii) amplification of the nucleic acids disclosed herein;
(iii) typing of the specific HLA-A alleles present in said sample.

Release, concentration and isolation of the nucleic acids from the sample can be done by any method known in the art. Currently, various commercial kits are available such as the QIAamp Blood Kit from Qiagen (Hilden, Germany) for the isolation of nucleic acids from blood samples or the 'High pure PCR Template Preparation Kit' (Roche Diagnostics, Brussels, Belgium). Other well-known procedures for the isolation of DNA or RNA from a biological sample are also available (Sambrook et al., 1989). The nucleic acids are subsequently amplified by the method of the invention described above. The products of this amplification step are then ideally suited for typing of the specific allele present in the sample. Currently 169 different alleles of HLA-A. 332 different alleles of HLA-B and 87 different alleles of HLA-C are known. Typing of these alleles can be done by any method known in the art, such as duplex analysis of the PCR products (Clay et al., 1994), single-stranded conformational polymorphism analysis of the PCR product (PCR-SSCP; Yoshida *et al.,* 1992), sequence-based typing (SBT; Santamaria et al., 1992 and 1993), the use of sequence specific primers in PCR reaction (PCR-SSP: Olerup and Zetterquist, 1991), the use of PCR in combination with sequence-specific oligonucleotide probing (PCR-SSOP; Saiki et al., 1986), conventional dot-blot, Southern blot, sandwich or probing by reverse dot-blot (Saiki et al., 1989). In order to obtain fast and easy results if a multitude of probes is involved, a reverse hybridization format may be convenient. Accordingly, in a preferred embodiment the selected probes are immobilized to certain locations on a solid support and the amplified polynucleic acids are labeled in order to enable the detection of the hybrids formed. The term "solid support" can refer to any substrate to which an oligonucleotide probe can be coupled, provided that it retains its hybridization characteristics and provided that the background level of hybridization remains low. Usually the solid substrate will be a microtiter plate (e.g. in the DEIA technique), a membrane (e.g. nylon or nitrocellulose) or a microsphere (bead) or a chip. Prior to application to the membrane or fixation, it may be convenient to modify the nucleic acid probe in order to facilitate fixation or improve the hybridization efficiency. Such modifications may encompass homopolymer tailing, coupling with different reactive groups such as aliphatic groups, NH₂ groups, SH groups, carboxylic groups, or coupling with biotin, haptens or proteins.

The present invention further relates to a diagnostic kit for the typing or subtyping of one or more HLA-A alleles in a sample comprising the following components:
(i) when appropriate, a means for releasing, isolating or concentrating the nucleic acids present in said sample;
(ii) a primer set or a primer mix as disclosed herein;
(iv) a means for typing of the specific HLA-A alleles present in said sample.

A specific and very user-friendly diagnostic kit is the a Line Probe Assay for the typing or subtyping of one or more HLA-A alleles in a sample comprising the following components:
(i) when appropriate, a means for releasing, isolating or concentrating the nucleic acids present in said sample;
(ii) a primer pair or a primer mix as disclosed herein;
(iii) at least one probe that specifically hybridizes with one of the HLA-A, alleles, fixed to a solid support;
(iv) a hybridization buffer, or components necessary for producing said buffer;
(v) a wash solution, or components necessary for producing said solution;
(vi) when appropriate, a means for detecting the hybrids resulting from the preceding hybridization.

In this embodiment, the selected set of probes is immobilized to a membrane strip in a line fashion. Said probes may be immobilized individually or as mixtures to the delineated locations. The amplified HLA-A polynucleic acids can be labelled with biotine, and the hybrid can then, via a biotine-streptavidine coupling, be detected with a non-radioactive colour developing system.

The term "hybridization buffer" means a buffer allowing a hybridization reaction between the probes and the polynucleic acids present in the sample, or the amplified products, under the appropriate stringency conditions.

The term "wash solution" means a solution enabling washing of the hybrids formed under the appropriate stringency conditions.

Also disclosed herein is the use of the primers, the primer sets and/or the primer mixes of the invention for the manufacturing of a diagnostic kit or Line Probe Assay for HLA Class I typing.

Throughout this specification and the claims which follow, unless the context requires otherwise, the word "comprise", and variations such as "comprises" and "comprising", will be understood to imply the inclusion of a stated integer or step or group of stated integers or steps but not to the exclusion of any other integer or step or group of integers or steps.

### FIGURE LEGENDS

**Figure 1****.** Alignment of 29 HLA-A intron 2 sequences. The consensus sequence is shown on top of the figure. Nucleotides conform to the consensus sequence are indicated with a vertical line. Nucleotides that differ from the consensus sequence are indicated. N indicates the absence of a nucleotide at that position. Sequencing was carried out according to example 1.
**Figure 2****.** Alignment of 38 HLA-B intron 2 sequences. The consensus sequence is shown on top of the figure. Nucleotides conform to the consensus sequence are indicated with a vertical line. Nucleotides that differ from the consensus sequence are indicated. N indicates the absence of a nucleotide at that position. Sequencing was carried out according to example 1.
**Figure 3****.** Alignment of 13 HLA-C intron 2 sequences. The consensus sequence is shown on top of the figure. Nucleotides conform to the consensus sequence are indicated with a vertical line. Nucleotides that differ from the consensus sequence are indicated. N indicates the absence of a nucleotide at that position. Sequencing was carried out according to example 1.
**Figure 4****.** Alignment of 12 HLA-A intron 3 sequences. The consensus sequence is shown on top of the figure. Nucleotides conform to the consensus sequence are indicated with a vertical line. Nucleotides that differ from the consensus sequence are indicated. N indicates the absence of a nucleotide at that position.
**Figure 5****.** Alignment of 22 HLA-B intron 3 sequences. The consensus sequence is shown on top of the figure. Nucleotides conform to the consensus sequence are indicated with a vertical line. Nucleotides that differ from the consensus sequence are indicated. N indicates the absence of a nucleotide at that position.
**Figure 6****.** Alignment of 12 HLA-C intron 3 sequences. The consensus sequence is shown on top of the figure. Nucleotides conform to the consensus sequence are indicated with a vertical line. Nucleotides that differ from the consensus sequence are indicated. N indicates the absence of a nucleotide at that position.

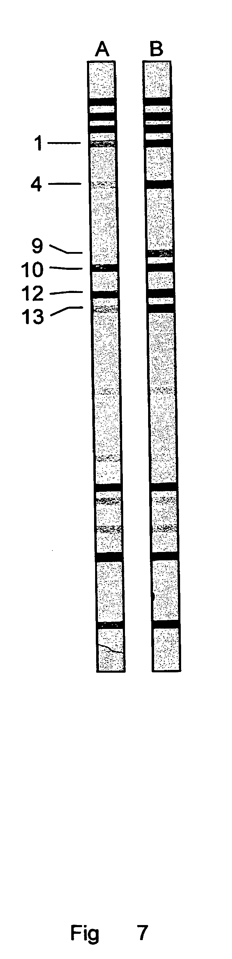

**Figure 7****.** Results of a Line Probe Assay for the typing of HLA-A as indicated in example 5. Nucleic acids were amplified by use of: (A) 5APBio (forward primer;
SEQ ID NO 144) and 3APBio (reverse primer; SEQ ID NO 147) for the amplification of exon 2 and exon 3 of HLA-A in one single amplicon; (B) the multiplex primer mix for the separate amplification of exon 2, exon 3 and exon 4 of HLA-A. The probes with numbers 1, 4, 9, 10, 12 and 13 cleary show a stronger signal after the hybridization with the amplicons obtained by use of the multiplex primer mix (strip B).

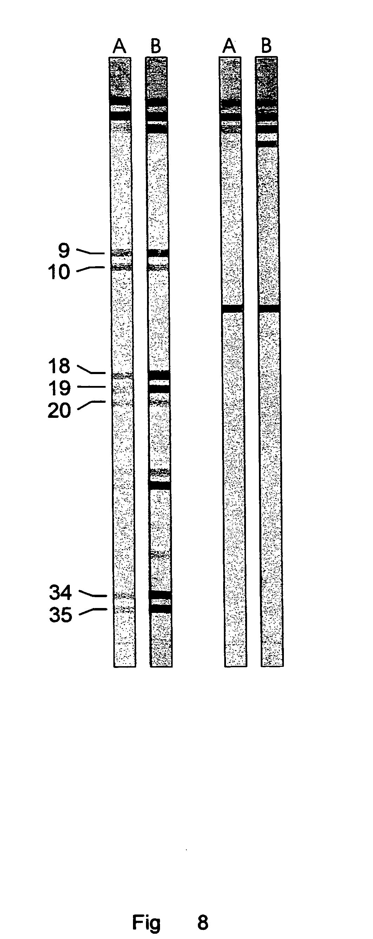

**Figure 8****.** Results of a Line Probe Assay for the typing of HLA-B as indicated in example 6. Nucleic acids were amplified by use of: (B) IBPin1 (forward primer; SEQ ID NO 145) and IBPin3 (reverse primer; SEQ ID NO 148) for the amplification of exon 2 and exon 3 of HLA-B in one single amplicon; (B) the multiplex primer mix for the separate amplification of exon 2, exon 3 and exon 4 of HLA-B. The probes with numbers 9, 10, 18, 19, 20, 34 and 35 cleary show a stronger signal after the hybridization with the amplicons obtained by use of the multiplex primer mix (strips B).

### TABLES

**Table 1. Reverse primers used for the amplification of HLA-A exon 2.**

| **3' position** **in intron 2** | **Sequence (5'-3')** | **SEQ ID NO** |
|---|---|---|
| 67 | ATCTCGGACCCGGAGACTGT | 1 |
| | GATCTCGGACCCGGAGACTGT | 2 |
| | GGATCTCGGACCCGGAGACTGT | 3 |
| | YGGATCTCGGACCCGGAGACTGT | 4 |
| | GYGGATCTCGGACCCGGAGACTGT | 5 |
| | GGYGGATCTCGGACCCGGAGACTGT | 6 |
| 96 | GGTCTCGGRGTCCCGCGGCT | 7 |
| | GGGTCTCGGRGTCCCGCGGCT | 8 |
| | AGGGTCTCGGRGTCCCGCGGCT | 9 |
| | AAGGGTCTCGGRGTCCCGCGGCT | 10 |
| | CAAGGGTCTCGGRGTCCCGCGGCT | 11 |
| 109 | CTCCCGGGDCAAGGGTCTCG | 12 |
| | TCTCCCGGGDCAAGGGTCTCG | 13 |
| | CTCTCCCGGGDCAAGGGTCTCG | 14 |
| | CCTCTCCCGGGDCAAGGGTCTCG | 15 |
| | GCCTCTCCCGGGDCAAGGGTCTCG | 16 |
| | GGCCTCTCCCGGGDCAAGGGTCTCG | 17 |
| 110 | TCTCCCGGGDCAAGGGTCTC | 18 |
| | CTCTCCCGGGDCAAGGGTCTC | 19 |
| | CCTCTCCCGGGDCAAGGGTCTC | 20 |
| | GCCTCTCCCGGGDCAAGGGTCTC | 21 |
| | GGCCTCTCCCGGGDCAAGGGTCTC | 22 |
| | GGGCCTCTCCCGGGDCAAGGGTCTC | 23 |
| 118 | CCTGGGCCTCTCCCGGGDCA | 30 |
| | GCCTGGGCCTCTCCCGGGDCA | 31 |
| | CGCCTGGGCCTCTCCCGGGDCA | 32 |
| | GCGCCTGGGCCTCTCCCGGGDCA | 33 |
| | GGCGCCTGGGCCTCTCCCGGGDCA | 34 |
| | AGGCGCCTGGGCCTCTCCCGGGDCA | 35 |
| 123 | AGGCGCCTGGGCCTCTCCCG | 36 |
| | AAGGCGCCTGGGCCTCTCCCG | 37 |
| | WAAGGCGCCTGGGCCTCTCCCG | 38 |
| | TWAAGGCGCCTGGGCCTCTCCCG | 39 |
| | GTWAAGGCGCCTGGGCCTCTCCCG | 40 |
| | GGTWAAGGCGCCTGGGCCTCTCCCG | 41 |
| 131 | CCGGGTWAAGGCGCCTGGGC | 42 |
| | ACCGGGTWAAGGCGCCTGGGC | 43 |
| | AACCGGGTWAAGGCGCCTGGGC | 44 |
| | AAACCGGGTWAAGGCGCCTGGGC | 45 |
| | GAAACCGGGTWAAGGCGCCTGGGC | 46 |
| | TGAAACCGGGTWAAGGCGCCTGGGC | 47 |
| 181 | YCCVGCCCCGACCAACCYGG | 48 |
| | GYCCVGCCCCGACCAACCYGG | 49 |
| | YGYCCVGCCCCGACCAACCYGG | 50 |
| | CYGYCCVGCCCCGACCAACCYGG | 51 |
| | CCYGYCCVGCCCCGACCAACCYGG | 52 |
| | CCCYGYCCVGCCCCGACCAACCYGG | 53 |

**Table 2. Forward primers used for the amplification of HLA-A exon 3.**

| **3' position** **in intron 2** | **Sequence (5'-3')** | **SEQ ID NO** |
|---|---|---|
| 67 | CGGACGGGCCRGGTSRCCCA | 54 |
| | ACGGACGGGCCRGGTSRCCCA | 55 |
| | CACGGACGGGCCRGGTSRCCCA | 56 |
| | CCACGGACGGGCCRGGTSRCCCA | 57 |
| | CCCACGGACGGGCCRGGTSRCCCA | 58 |
| | CCCCACGGACGGGCCRGGTSRCCCA | 59 |
| 96 | GGTCCGAGATCCRCCCCGAA | 60 |
| | GGGTCCGAGATCCRCCCCGAA | 61 |
| | CGGGTCCGAGATCCRCCCCGAA | 62 |
| | CCGGGTCCGAGATCCRCCCCGAA | 63 |
| | TCCGGGTCCGAGATCCRCCCCGAA | 64 |
| | CTCCGGGTCCGAGATCCRCCCCGAA | 65 |
| 109 | CCCCGAAGCCGCGGGACYCC | 66 |
| | RCCCCGAAGCCGCGGGACYCC | 67 |
| | CRCCCCGAAGCCGCGGGACYCC | 68 |
| | CCRCCCCGAAGCCGCGGGACYCC | 69 |
| | TCCRCCCCGAAGCCGCGGGACYCC | 70 |
| | ATCCRCCCCGAAGCCGCGGGACYCC | 71 |
| 110 | CCCGAAGCCGCGGGACYCCG | 72 |
| | CCCCGAAGCCGCGGGACYCCG | 73 |
| | RCCCCGAAGCCGCGGGACYCCG | 74 |
| | CRCCCCGAAGCCGCGGGACYCCG | 75 |
| | CCRCCCCGAAGCCGCGGGACYCCG | 76 |
| | TCCRCCCCGAAGCCGCGGGACYCCG | 77 |
| 118 | CGCGGGACYCCGAGACCCTT | 84 |
| | CCGCGGGACYCCGAGACCCTT | 85 |
| | GCCGCGGGACYCCGAGACCCTT | 86 |
| | AGCCGCGGGACYCCGAGACCCTT | 87 |
| | AAGCCGCGGGACYCCGAGACCCTT | 88 |
| | GAAGCCGCGGGACYCCGAGACCCTT | 89 |
| 123 | GACYCCGAGACCCTTGDCCC | 90 |
| | GGACYCCGAGACCCTTGDCCC | 91 |
| | GGGACYCCGAGACCCTTGDCCC | 92 |
| | CGGGACYCCGAGACCCTTGDCCC | 93 |
| | GCGGGACYCCGAGACCCTTGDCCC | 94 |
| | CGCGGGACYCCGAGACCCTTGDCCC | 95 |
| 131 | GACCCTTGDCCCGGGAGAGG | 96 |
| | AGACCCTTGDCCCGGGAGAGG | 97 |
| | GAGACCCTTGDCCCGGGAGAGG | 98 |
| | CGAGACCCTTGDCCCGGGAGAGG | 99 |
| | CCGAGACCCTTGDCCCGGGAGAGG | 100 |
| | YCCGAGACCCTTGDCCCGGGAGAGG | 101 |
| 181 | GTTTAGGCCAAAAATCCCCC | 102 |
| | AGTTTAGGCCAAAAATCCCCC | 103 |
| | CAGTTTAGGCCAAAAATCCCCC | 104 |
| | TCAGTTTAGGCCAAAAATCCCCC | 105 |
| | TTCAGTTTAGGCCAAAAATCCCCC | 106 |
| | TTTCAGTTTAGGCCAAAAATCCCCC | 107 |

**Table 3. Reverse primers used for the amplification of HLA-A exon 3.**

| **3' position** **in intron 3** | **Sequence (5'-3')** | **SEQ ID NO** |
|---|---|---|
| 32 | AGCCCGGGAGATCTAYAGGC | 150 |
| | CAGCCCGGGAGATCTAYAGGC | 151 |
| | CCAGCCCGGGAGATCTAYAGGC | 152 |
| | GCCAGCCCGGGAGATCTAYAGGC | 153 |
| | GGCCAGCCCGGGAGATCTAYAGGC | 154 |
| | AGGCCAGCCCGGGAGATCTAYAGGC | 155 |
| 50 | CCCTCCTTGTGGGAGGCCAG | 156 |
| | CCCCTCCTTGTGGGAGGCCAG | 157 |
| | TCCCCTCCTTGTGGGAGGCCAG | 158 |
| | CTCCCCTCCTTGTGGGAGGCCAG | 159 |
| | TCTCCCCTCCTTGTGGGAGGCCAG | 160 |
| | GTCTCCCCTCCTTGTGGGAGGCCAG | 161 |
| 62 | CCCAAWTGTCTCCCCTCCTT | 162 |
| | TCCCAAWTGTCTCCCCTCCTT | 163 |
| | GTCCCAAWTGTCTCCCCTCCTT | 164 |
| | GGTCCCAAWTGTCTCCCCTCCTT | 165 |
| | TGGTCCCAAWTGTCTCCCCTCCTT | 166 |
| | TTGGTCCCAAWTGTCTCCCCTCCTT | 167 |
| 73 | CTAGTGTTGGTCCCAAWTGT | 168 |
| | TCTAGTGTTGGTCCCAAWTGT | 169 |
| | TTCTAGTGTTGGTCCCAAWTGT | 170 |
| | ATTCTAGTGTTGGTCCCAAWTGT | 171 |
| | TATTCTAGTGTTGGTCCCAAWTGT | 172 |
| | ATATTCTAGTGTTGGTCCCAAWTGT | 173 |
| 83 | GGGYGATATTCTAGTGTTGG | 174 |
| | AGGGYGATATTCTAGTGTTGG | 175 |
| | GAGGGYGATATTCTAGTGTTGG | 176 |
| | GGAGGGYGATATTCTAGTGTTGG | 177 |
| | GGGAGGGYGATATTCTAGTGTTGG | 178 |
| | AGGGAGGGYGATATTCTAGTGTTGG | 179 |
| 86 | GGAGGGYGATATTCTAGTGT | 180 |
| | GGGAGGGYGATATTCTAGTGT | 181 |
| | AGGGAGGGYGATATTCTAGTGT | 182 |
| | GAGGGAGGGYGATATTCTAGTGT | 183 |
| | AGAGGGAGGGYGATATTCTAGTGT | 184 |
| | CAGAGGGAGGGYGATATTCTAGTGT | 185 |
| 118 | CCCAGGAGGAKTCCTCTCCC | 186 |
| | ACCCAGGAGGAKTCCTCTCCC | 187 |
| | AACCCAGGAGGAKTCCTCTCCC | 188 |
| | AAACCCAGGAGGAKTCCTCTCCC | 189 |
| | GAAACCCAGGAGGAKTCCTCTCCC | 190 |
| | GGAAACCCAGGAGGAKTCCTCTCCC | 191 |
| 130 | AGGATCTGGAAACCCAGGAG | 192 |
| | CAGGATCTGGAAACCCAGGAG | 193 |
| | ACAGGATCTGGAAACCCAGGAG | 194 |
| | TACAGGATCTGGAAACCCAGGAG | 195 |
| | GTACAGGATCTGGAAACCCAGGAG | 196 |
| | GGTACAGGATCTGGAAACCCAGGAG | 197 |
| 150 | TCAGAGTCACTCTCTGGTAC | 198 |
| | CTCAGAGTCACTCTCTGGTAC | 199 |
| | CCTCAGAGTCACTCTCTGGTAC | 200 |
| | ACCTCAGAGTCACTCTCTGGTAC | 201 |
| | AACCTCAGAGTCACTCTCTGGTAC | 202 |
| | GAACCTCAGAGTCACTCTCTGGTAC | 203 |

**Table 4. Forward primers used for the amplification of HLA-A exon 4.**

| **3' position** **in intron 3** | **Sequence (5'-3')** | **SEQ ID NO** |
|---|---|---|
| 501 | TTCTGTGCTCYCTTCCCCAT | 204 |
| | GTTCTGTGCTCYCTTCCCCAT | 205 |
| | GGTTCTGTGCTCYCTTCCCCAT | 206 |
| | GGGTTCTGTGCTCYCTTCCCCAT | 207 |
| | TGGGTTCTGTGCTCYCTTCCCCAT | 208 |
| | CTGGGTTCTGTGCTCYCTTCCCCAT | 209 |
| 525 | GGTGTCCTGTCCATTCTCAA | 24 |
| | RGGTGTCCTGTCCATTCTCAA | 25 |
| | CRGGTGTCCTGTCCATTCTCAA | 26 |
| | CCRGGTGTCCTGTCCATTCTCAA | 27 |
| | CCCRGGTGTCCTGTCCATTCTCAA | 28 |
| | TCCCRGGTGTCCTGTCCATTCTCAA | 29 |
| 561 | CTGGWGGAGTGTCCCATKAC | 201 |
| | GCTGGWGGAGTGTCCCATKAC | 211 |
| | TGCTGGWGGAGTGTCCCATKAC | 212 |
| | RTGCTGGWGGAGTGTCCCATKAC | 213 |
| | YRTGCTGGWGGAGTGTCCCATKAC | 214 |
| | GYRTGCTGGWGGAGTGTCCCATKAC | 215 |
| 571 | GTCCCATKACAGATRCMMAA | 216 |
| | TGTCCCATKACAGATRCMMAA | 217 |
| | GTGTCCCATKACAGATRCMMAA | 218 |
| | AGTGTCCCATKACAGATRCMMAA | 219 |
| | GAGTGTCCCATKACAGATRCMMAA | 220 |
| | GGAGTGTCCCATKACAGATRCMMAA | 221 |

**Table 5. Reverse primers used for the amplification of HLA-B exon 2.**

| **3' position** **in intron 2** | **Sequence (5'-3')** | **SEQ ID NO** |
|---|---|---|
| 170 | ACCCGCGGGGATTTTGGCCTC | 108 |
| | AACCCGCGGGGATTTTGGCCTC | 109 |
| | CAACCCGCGGGGATTTTGGCCTC | 110 |
| | CCAACCCGCGGGGATTTTGGCCTC | 111 |
| | MCCAACCCGCGGGGATTTTGGCCTC | 112 |
| | GMCCAACCCGCGGGGATTTTGGCCTC | 113 |
| | YGMCCAACCCGCGGGGATTTTGGCCTC | 314 |

**Table 6. Forward primers used for the amplification of HLA-B exon 3.**

| **3' position in intron 2** | **Sequence (5'-3')** | **SEQ ID NO** |
|---|---|---|
| 35 | CYGGGGCGSAGGTCACGACT | 114 |
| | CCYGGGGCGSAGGTCACGACT | 115 |
| | GCCYGGGGCGSAGGTCACGACT | 116 |
| | GGCCYGGGGCGSAGGTCACGACT | 117 |
| | CGGCCYGGGGCGSAGGTCACGACT | 118 |
| | CCGGCCYGGGGCGSAGGTCACGACT | 119 |
| 170 | CCCGGTTTCATTTTCAGTTG | 120 |
| | ACCCGGTTTCATTTTCAGTTG | 121 |
| | TACCCGGTTTCATTTTCAGTTG | 122 |
| | TTACCCGGTTTCATTTTCAGTTG | 123 |
| | TTTACCCGGTTTCATTTTCAGTTG | 124 |
| | GTTTACCCGGTTTCATTTTCAGTTG | 125 |
| | CGTTTACCCGGTTTCATTTTCAGTTG | 222 |
| | GCGTTTACCCGGTTTCATTTTCAGTTG | 223 |
| | CGCGTTTACCCGGTTTCATTTTCAGTTG | 224 |

**Table 7. Reverse primers used for the amplification of HLA-B exon 3.**

| **3' position** **in intron 3** | **Sequence (5'-3')** | **SEQ ID NO** |
|---|---|---|
| 42 | CGTKGGAGSCCATCCCCGSC | 225 |
| | TCGTKGGAGSCCATCCCCGSC | 226 |
| | CTCGTKGGAGSCCATCCCCGSC | 227 |
| | TCTCGTKGGAGSCCATCCCCGSC | 228 |
| | TTCTCGTKGGAGSCCATCCCCGSC | 229 |
| | CTTCTCGTKGGAGSCCATCCCCGSC | 230 |
| 46 | TCTCGTKGGAGSCCATCCCC | 231 |
| | TTCTCGTKGGAGSCCATCCCC | 232 |
| | CTTCTCGTKGGAGSCCATCCCC | 233 |
| | TCTTCTCGTKGGAGSCCATCCCC | 234 |
| | YTCTTCTCGTKGGAGSCCATCCCC | 235 |
| | CYTCTTCTCGTKGGAGSCCATCCCC | 236 |
| 65 | GATCCCATTTTCCTCYTCTT | 237 |
| | TGATCCCATTTTCCTCYTCTT | 238 |
| | CTGATCCCATTTTCCTCYTCTT | 239 |
| | GCTGATCCCATTTTCCTCYTCTT | 240 |
| | CGCTGATCCCATTTTCCTCYTCTT | 241 |
| | GCGCTGATCCCATTTTCCTCYTCTT | 242 |
| 68 | GCTGATCCCATTTTCCTCYT | 243 |
| | CGCTGATCCCATTTTCCTCYT | 244 |
| | GCGCTGATCCCATTTTCCTCYT | 245 |
| | AGCGCTGATCCCATTTTCCTCYT | 246 |
| | TAGCGCTGATCCCATTTTCCTCYT | 247 |
| | CTAGCGCTGATCCCATTTTCCTCYT | 248 |
| 96 | TCCATTCAAGGGAGGGCGAC | 249 |
| | CTCCATTCAAGGGAGGGCGAC | 250 |
| | TCTCCATTCAAGGGAGGGCGAC | 251 |
| | TTCTCCATTCAAGGGAGGGCGAC | 252 |
| | ATTCTCCATTCAAGGGAGGGCGAC | 253 |
| | CATTCTCCATTCAAGGGAGGGCGAC | 254 |

**Table 8. Forward primers used for the amplification of HLA-B exon 4.**

| **3' position** **in intron** | **Sequence (5'-3')** | **SEQ ID NO** |
|---|---|---|
| 438 | AGATTATCCCAGGTGCCTGC | 255 |
| | GAGATTATCCCAGGTGCCTGC | 256 |
| | GGAGATTATCCCAGGTGCCTGC | 257 |
| | AGGAGATTATCCCAGGTGCCTGC | 258 |
| | TAGGAGATTATCCCAGGTGCCTGC | 259 |
| | ATAGGAGATTATCCCAGGTGCCTGC | 260 |
| 502 | TGTCCTGYCCATTCTCAGKC | 261 |
| | GTGTCCTGYCCATTCTCAGKC | 262 |
| | GGTGTCCTGYCCATTCTCAGKC | 263 |
| | AGGTGTCCTGYCCATTCTCAGKC | 264 |
| | CAGGTGTCCTGYCCATTCTCAGKC | 265 |
| | CCAGGTGTCCTGYCCATTCTCAGKC | 266 |
| 524 | TCACATGGGTGGTCCTAGG | 267 |
| | GTCACATGGGTGGTCCTAGG | 268 |
| | GGTCACATGGGTGGTCCTAGG | 269 |
| | TGGTCACATGGGTGGTCCTAGG | 270 |
| | CTGGTCACATGGGTGGTCCTAGG | 271 |
| | KCTGGTCACATGGGTGGTCCTAGG | 272 |
| | GKCTGGTCACATGGGTGGTCCTAGG | 273 |
| 547 | TSCCATGARAGATGCMAAGC | 274 |
| | GTSCCATGARAGATGCMAAGC | 275 |
| | TGTSCCATGARAGATGCMAAGC | 276 |
| | GTGTSCCATGARAGATGCMAAGC | 277 |
| | GGTGTSCCATGARAGATGCMAAGC | 278 |
| | GGGTGTSCCATGARAGATGCMAAGC | 279 |
| 571 | GWAWTTTCTGACTCTTCCCA | 280 |
| | TGWAWTTTCTGACTCTTCCCA | 281 |
| | CTGWAWTTTCTGACTCTTCCCA | 282 |
| | CCTGWAWTTTCTGACTCTTCCCA | 283 |
| | GCCTGWAWTTTCTGACTCTTCCCA | 284 |
| | CGCCTGWAWTTTCTGACTCTTCCCA | 285 |

**Table 9. Reverse primers used for the amplification of HLA-C exon 2.**

| **3' position** **in intron 2** | **Sequence (5'-3')** | **SEQ ID NO** |
|---|---|---|
| 107 | GTCGAGGGTCTGGGCGGGTT | 126 |
| | GGTCGAGGGTCTGGGCGGGTT | 127 |
| | CGGTCGAGGGTCTGGGCGGGTT | 128 |
| | CCGGTCGAGGGTCTGGGCGGGTT | 129 |
| | YCCGGTCGAGGGTCTGGGCGGGTT | 130 |
| | CYCCGGTCGAGGGTCTGGGCGGGTT | 131 |

**Table 10. Forward primers used for the amplification of HLA-C exon 3.**

| **3' position** **in intron 2** | **Sequence (5'-3')** | **SEQ ID NO** |
|---|---|---|
| 84 | CGCCCCRAGTCTCCSSGTCT | 132 |
| | TCGCCCCRAGTCTCCSSGTCT | 133 |
| | GTCGCCCCRAGTCTCCSSGTCT | 134 |
| | GGTCGCCCCRAGTCTCCSSGTCT | 135 |
| | GGGTCGCCCCRAGTCTCCSSGTCT | 136 |
| | CGGGTCGCCCCRAGTCTCCSSGTCT | 137 |
| 142 | CGRCCGGRGAGAGCCCCAGT | 138 |
| | TCGRCCGGRGAGAGCCCCAGT | 139 |
| | CTCGRCCGGRGAGAGCCCCAGT | 140 |
| | CCTCGRCCGGRGAGAGCCCCAGT | 141 |
| | CCCTCGRCCGGRGAGAGCCCCAGT | 142 |
| | ACCCTCGRCCGGRGAGAGCCCCAGT | 143 |

**Table 11. Forward primers used for the amplification of HLA-C exon 4.**

| **3' position** **in intron 3** | **Sequence (5'-3')** | **SEQ ID NO** |
|---|---|---|
| 461 | GTGCCTGTGTCCAGGCTGGC | 286 |
| | GGTGCCTGTGTCCAGGCTGGC | 287 |
| | AGGTGCCTGTGTCCAGGCTGGC | 288 |
| | CAGGTGCCTGTGTCCAGGCTGGC | 289 |
| | CCAGGTGCCTGTGTCCAGGCTGGC | 290 |
| | CCCAGGTGCCTGTGTCCAGGCTGGC | 291 |
| 477 | TGGCGTCTGGGTTCTGTGCC | 292 |
| | CTGGCGTCTGGGTTCTGTGCC | 293 |
| | GCTGGCGTCTGGGTTCTGTGCC | 294 |
| | GGCTGGCGTCTGGGTTCTGTGCC | 295 |
| | AGGCTGGCGTCTGGGTTCTGTGCC | 296 |
| | CAGGCTGGCGTCTGGGTTCTGTGCC | 297 |
| 527 | CTCAGGATRGTCACATGGSC | 298 |
| | TCTCAGGATRGTCACATGGSC | 299 |
| | TTCTCAGGATRGTCACATGGSC | 300 |
| | RTTCTCAGGATRGTCACATGGSC | 301 |
| | CRTTCTCAGGATRGTCACATGGSC | 302 |
| | CCRTTCTCAGGATRGTCACATGGSC | 303 |
| 545 | GCGCTGTTGGAGTGTCGCAA | 78 |
| | GGCGCTGTTGGAGTGTCGCAA | 79 |
| | GGGCGCTGTTGGAGTGTCGCAA | 80 |
| | TGGGCGCTGTTGGAGTGTCGCAA | 81 |
| | ATGGGCGCTGTTGGAGTGTCGCAA | 82 |
| | CATGGGCGCTGTTGGAGTGTCGCAA | 83 |
| 561 | SCAAGAGAGAWRCAAAGTGT | 304 |
| | CSCAAGAGAGAWRCAAAGTGT | 305 |
| | TCSCAAGAGAGAWRCAAAGTGT | 306 |
| | GTCSCAAGAGAGAWRCAAAGTGT | 307 |
| | TGTCSCAAGAGAGAWRCAAAGTGT | 308 |
| | GTGTCSCAAGAGAGAWRCAAAGTGT | 309 |

### EXAMPLES

### Example 1: Sequence determination of intron 2 of various HLA-A, HLA-B and HLA-C alleles

Nucleic acids were prepared from different blood samples by use of the QIAamp Blood Kit (Qiagen, Hilden, Germany) according to the manufacturer's protocol.

Part of exon 1, intron 2, exon 2, intron 2 and exon 3 of HLA-A were amplified by use of the following primer set:

| **Primer** | **Sequence (5'-3')** | **SEQ ID NO** |
|---|---|---|
| 5APBio (5') | B-TTCTCCCCAGACGCCGAGGATGGCC | 144 |
| 3APBio (3') | B-CCGTGCGCTGCAGCGTCTCCTTCCCG | 147 |

| | | |
|---|---|---|
| B = biotine. | | |

Exon 2, intron 2 and exon 3 of HLA-B were amplified by use of the following primer set:

| **Primer** | **Sequence (5'-3')** | **SEQ ID NO** |
|---|---|---|
| IBPin1 (5') | B-GGGAGGAGCGAGGGGACCSCAG | 145 |
| IBPin3 (3') | B-GGAGGCCATCCCCGGCGACCTAT | 148 |

| | | |
|---|---|---|
| B = biotine. | | |

Exon 2, intron 2 and exon 3 of HLA-C were amplified by use of the following primer set:

| **Primer** | **Sequence (5'-3')** | **SEQ ID NO** |
|---|---|---|
| 5CIN1 (5') | B-AGCGAGGGGCCCGCCCGGCGA | 146 |
| 3CIN3 (3') | B-GGAGATGGGGAAGGCTCCCCACT | 149 |

| | | |
|---|---|---|
| B = biotine. | | |

The PCR reaction cycle was composed of the following steps:
1 min at 96°C;
5 times (30 s at 96°C; 50 s at 64°C; 50 s at 72°C);
5 times (30 s at 96°C; 50 s at 62°C; 50 s at 72°C);
10 times (30 s at 96°C; 50 s at 60°C; 50 s at 72°C);
15 times (30 s at 96°C; 50 s at 55°C; 50 s at 72°C);
5 min at 72°C.

The amplification reaction was carried out in 50 mM Tris-HCl pH 9.2, 16 mM (NH₄)₂SO₄, 200 µM dNTPs, 2.5 U Taq polymerase, 1.5 mM MgCl₂, 15 pmol of each primer and 0.1 to 0.5 µg DNA.

The resulting amplicon was cloned in the pGEMt-vector (Promega, Madison, WI, USA). Nucleotide sequence analysis was performed by use of an automated DNA sequencer Model 373A (Applied Biosystems, Foster City, CA, USA) with fluorescence-labelled dideoxy nucleotides (PrismTM Ready Reaction Dye Terminator Cycle Sequencing Kit; Applied Biosystems, Foster City, CA, USA). The primers used for the sequencing reaction were the same as for the amplification step. 29 intron 2 sequences were obtained for HLA-A, 38 for HLA-B and 13 for HLA-C. The sequences are shown in Figures 1, 2 and 3, respectively.

### Example 2: Amplification of exon 2 and exon 3 of HLA-A

Nucleic acids were prepared from different blood samples by use of the QIAamp Blood Kit (Qiagen, Hilden, Germany) according to the manufacturer's protocol. Based on the sequence alignment of the HLA-A intron 2 sequences (Figure 1), a reverse and a forward, locus-specific primer was designed for the specific amplification of the HLA-A exon 2 and exon 3, respectively. With these primers, a primer mix was constructed for the separate amplification of exon 2 and exon 3 of HLA-A consisting of the following 2 primer sets:
- for exon 2: 5APBio (SEQ ID NO 144) as forward primer and 5'ATCTCGGACCCGGAGACTGT3' (SEQ ID NO 1) as reverse primer;
- for exon 3: 5'CAGTTTAGGCCAAAAATCCCCC3' (SEQ ID NO 104) as forward primer and 3APBio (SEQ ID NO 147) as reverse primer.

The PCR reaction cycle was composed of the following steps:
5 min at 96°C;
35 times (30 s at 96°C; 20 s at 58°C; 30 s at 72°C);
10 min at 72°C.

The PCR reaction was carried out in 10 mM Tris.HCl pH 8.3, 50 mM KCl, 1.5 mM MgCl₂, 0.001% (w/v) gelatine, 200µM dNTP's (dATP, dGTP, dCTP, dTTP), 20 pmol of each primer and 1U AmpliTaq (Applied Biosystems, Foster City, CA, USA). The length of the obtained PCR products was verified on an agarose gel according to Sambrook et al. (1989).

### Example 3: Amplification of exon 2 and exon 3 of HLA-B

Nucleic acids were prepared from different blood samples by use of the QIAamp Blood Kit (Qiagen, Hilden, Germany) according to the manufacturer's protocol. Based on the sequence alignment of the HLA-B intron 2 sequences (Figure 2), a reverse and a forward, locus-specific primer was designed for the specific amplification of the HLA-B exon 2 and exon 3, respectively. With these primers, a primer mix was constructed for the separate amplification of exon 2 and exon 3 of HLA-B consisting of the following 2 primer sets:
- for exon 2: IBPin1 (SEQ ID NO 145) as forward primer and 5'ACCCGCGGGGATTTTTGGCCTC3' (SEQ ID NO 310) as reverse primer;
- for exon 3: 5'ACCCGGTTTCATTTTCAGTTG3' (SEQ ID NO 121) as forward primer and IBPin3 (SEQ ID NO 148) as reverse primer.

The PCR reaction cycle was composed of the following steps:
5 min at 96°C;
35 times (30 s at 96°C; 20 s at 58°C; 30 s at 72°C);
10 min at 72°C.

The PCR reaction was carried out in 10 mM Tris.HCl pH 8.3, 50 mM KCl, 1.5 mM MgCl₂, 0.001% (w/v) gelatine, 200µM dNTP's (dATP, dGTP, dCTP, dTTP), 20 pmol of each primer and 1U AmpliTaq (Applied Biosystems, Foster City, CA, USA). The length of the obtained PCR products was verified on an agarose gel according to Sambrook et al. (1989).

### Example 4: Amplification of exon 2 and exon 3 of HLA-C

Nucleic acids were prepared from different blood samples by use of the QIAamp Blood Kit (Qiagen, Hilden, Germany) according to the manufacturer's protocol. Based on the sequence alignment of the HLA-C intron 2 sequences (Figure 3), a reverse and a forward, locus-specific primer was designed for the specific amplification of the HLA-C exon 2 and exon 3, respectively. With these primers, a primer mix was constructed for the separate amplification of exon 2 and exon 3 of HLA-C consisting of the following 2 primer sets:
- for exon 2: 5CIN1 (SEQ ID NO 146) as forward primer and 5'GGTCGAGGGTCTGGGCGGGTT3' (SEQ ID NO 127) as reverse primer;
- for exon 3: 5'TCGRCCGGRGAGAGCCCCAGT3' (SEQ ID NO 139) as forward primer and 3CIN3 (SEQ ID NO 149) as reverse primer.

The PCR reaction cycle was composed of the following steps:
5 min at 96°C;
35 times (30 s at 96°C; 20 s at 58°C; 30 s at 72°C);
10 min at 72°C.

The PCR reaction was carried out in 10 mM Tris.HCl pH 8.3? 50 mM KCl, 1.5 mM MgCl₂, 0.001% (w/v) gelatine, 200µM DNTP's (dATP, dGTP, dCTP, dTTP), 20 pmol of each primer and 1U AmpliTaq (Applied Biosystems, Foster City, CA, USA). The length of the obtained PCR products was verified on an agarose gel according to Sambrook et al. (1989).

### Example 5: Amplification exon 2, exon 3 and exon 4 of HLA-A

Blood samples were collected from a Caucasian donor at B.A.R.C. (Gent, Belgium). Nucleic acids were prepared from the blood samples by use of the QIAamp Blood Kit (Qiagen. Hilden, Germany) according to the manufacturer's protocol.

### 5.1 Primer mix for the separate amplification of exon 2, exon 3 and exon 4 of HLA-A

A primer mix was used for the separate amplification of exon 2, exon 3 and exon 4 of HLA-A consisting of the following primer sets:

| **Amplicon** | **Primerset** | **Sequence (5'-3')** | **SEQ ID NO** |
|---|---|---|---|
| Exon 1-2 | 5APbio (5') | B-TTCTCCCCAGACGCCGAGGATGGCC | 144 |
| | 3ex2APbio (3') | B-ATCTCGGACCCGGAGACTGT | |
| Exon 3 | 5ex3APbio (5') | B-CAGTTTAGGCCAAAAATCCCCC | 104 |
| | 3ex3APbio (3') | B-CCCTCCTTGTGGGAGGCCAG | 156 |
| Exon 4 | 5ex4APbio (5') | B-GTTCTGTGCTCYCTTCCCCAT | 205 |
| | 3ex4APbio (3') | B-TTGGGCAGACCCTCATGCTGC | 311 |

| | | | |
|---|---|---|---|
| B = biotine. | | | |

The PCR reaction cycle was composed of the following steps:
1 min 96 °C;
5 times (30 s at 96 °C; 50 s at 64 °C; 50 s at 72 °C);
5 times (30 s at 96 °C; 50 s at 62 °C; 50 s at 72 °C);
10 times (30 s at 96 °C; 50 s at 60 °C; 50 s at 72 °C);
15 times (30 s at 96 °C; 50 s at 55 °C; 50 s at 72 °C);
5 min 72 °C;
4°C.

The PCR reaction was carried out in 50 mM Tris.HCl pH 9.2, 16 mM (NH₄)₂SO₄, 1.5 mM MgCl₂, 200µM dNTP's (dATP, dGTP, dCTP, dTTP), 2.5 U Taq polymerase (Perkin Elmer, Roche Molecular Systems, Branchburg, New Jersey, US), 20 pmol primer 5APbio, 20 pmol primer 3ex2APbio, 40 pmol primer 5ex3APbio, 40 pmol primer 3ex3APbio, 15 pmol primer 5ex4APbio, 15 pmol primer 3ex4APbio and 0.1-0.5 µg DNA.

The length of the obtained amplification products was verified on a 3% agarose gel according to Sambrook et al. (1989). 3 bands of different length were obtained relating respectively to exon 2 (559 bp), exon 3 (439 bp) and exon 4 (380 bp) of HLA-A. By use of this protocol, the HLA-A exons were amplified strong enough for subsequent typing in a hybridization assay.

### 5.2 Testing of the obtained amplification products in a HLA-A typing assay

The HLA-A amplicons were subsequently typed in a reverse hybridization assay based on the LiPA technology (Stuyver et al., 1993). After the amplification step as described above, the amplified nucleic acids were hybridized to a panel of 36 probes by use of the LiPA HLA-A kit (Innogenetics, Gent, Belgium) according to the manufacturer's instructions. Results of this reverse hybridization are shown in Figure 7.

For comparison, also an amplification product comprising exon 2 and exon 3 in one single amplicon was obtained by use of 5APBio (forward primer; SEQ ID NO 144) and 3APBio (reverse primer; SEQ ID NO 147). The hybridization of this larger amplicon to the probes on the LiPA strip is also shown in Figure 7. Figure 7 clearly illustrates that the amplicons obtained by separate amplification of exon 2 and exon 3 enable a more clear and prononounced typing then the larger amplicon obtained by amplification of exon 2 and exon 3 in one single amplicon.

### Example 6: Amplification exon 2, exon 3 and exon 4 of HLA-B

Blood samples were collected from a Caucasian donor at B.A.R.C. (Gent, Belgium). Nucleic acids were prepared from the blood samples by use of the QIAamp Blood Kit (Qiagen, Hilden, Germany) according to the manufacturer's protocol.

### 6.1 Primer mix for the separate amplification of exon 2, exon 3 and exon 4 of HLA-B

A primer mix was used for the separate amplification of exon 2, exon 3 and exon 4 of HLA-B consisting of the following primer sets:

| **Amplicon** | **Primerset** | **Sequence (5'-3')** | **SEQ ID NO** |
|---|---|---|---|
| Exon 2 | IBPin1bio (5') | B-GGGAGGAGCGAGGGGACCSCAG | 145 |
| | IB3Pin2bio (3') | B-AACCCGCGGGGATTTTGGCCTC | 109 |
| Exon 3 | IB5Pin2bio (5') | B-CGCGTTTACCCGGTTTCATTTTCAGTTG | 224 |
| | IB3Pin3bio (3') | B-TCTTCTCGTKGGAGSCCATCCCC | 234 |
| Exon 4 | 5ex4IBbio (5') | B-TCACATGGGTGGTCCTAGG | 267 |
| | 3ex4IBbio (3') | B-TCGGCAGCCCTCATGCTGT | 312 |

| | | | |
|---|---|---|---|
| B = biotine. | | | |

The PCR reaction cycle was composed of the following steps:
1 min 96°C;
5 times (30 s at 96 °C; 50 s at 64 °C; 50 s at 72 °C);
5 times (30 s at 96 °C; 50 s at 62°C; 50 s at 72 °C);
10 times (30 s at 96 °C; 50 s at 60 °C; 50 s at 72 °C);
15 times (30 s at 96 °C; 50 s at 55 °C; 50 s at 72 °C);
5 min 72°C;
4°C

The PCR reaction was carried out in 50 mM Tris.HCl pH 9.2, 16 mM (Nm₄)₂SO₄, 1.5 mM MgCl₂, 200µM dNTP's (dATP, dGTP, dCTP, dTTP), 2.5 U Taq polymerase (Perkin Elmer, Roche Molecular Systems, Branchburg, New Jersey, US), 35 pmol primer IBPinlbio, 35 pmol primer IBPin2bio, 50 pmol primer IB5Pin2bio, 50 pmol primer IB3Pin3bio, 10 pmol primer 5ex4IBbio, 10 pmol primer 3ex4IBbio and 0.1-0.5 µg DNA.

The length of the obtained amplification products was verified on a 3% agarose gel according to Sambrook et al. (1989). 3 bands of different length were obtained relating respectively to HLA-B exon 2 (555 bp), exon 3 (446 bp) and exon 4 (323 bp). By use of this protocol, the HLA-B exons were amplified strong enough for subsequent typing in a Line Probe Assay.

### 6.2 Testing of the obtained amplification products in a HLA-B typing assay

The HLA-B amplicons were subsequently typed in a reverse hybridization assay based on the LiPA technology (Stuyver et al., 1993). After the amplification step as described above, the amplified nucleic acids were hybridized to a panel of 60 probes by use of the LiPA HLA-B kit (Innogenetics, Gent, Belgium) according to the manufacturer's instructions. Results of this reverse hybridization are shown in Figure 8.

For comparison, also an amplification product comprising exon 2 and exon 3 in one single amplicon was obtained by use of IBPin1 (forward primer; SEQ ID NO 145) and IBPin3 (reverse primer; SEQ ID NO 148). The hybridization of this larger amplicon to the probes on the LiPA strip is also shown in Figure 8. Figure 8 clearly illustrates that the amplicons obtained by separate amplification of exon 2 and exon 3 enable a more clear and prononounced typing then the larger amplicon obtained by amplification of exon 2 and exon 3 in one single amplicon.

### Example 7: Amplification exon 2, exon 3 and exon 4 of HLA-C

Nucleic acids are prepared from the blood samples by use of the QIAamp Blood Kit (Qiagen, Hilden, Germany) according to the manufacturer's protocol. A primer mix is used for the separate amplification of exon 2, exon 3 and exon 4 of HLA-C consisting of the following primer sets:

| **Amplicon** | **Primerset** | **Sequence (5'-3')** | **SEQ ID NO** |
|---|---|---|---|
| Exon 2 | 5CIN1(5') | B-AGCGAGGGGCCCGCCCGGCGA | 146 |
| | IC3Pin2bio(3') | B-GGTCGAGGGTCTGGGCGGGTT | 127 |
| Exon 3 | IC5Pin2bio(5') | B-TCGRCCGGRGAGAGCCCCAGT | 139 |
| | 3CIN3 (3') | B-GGAGATGGGGAAGGCTCCCCACT | 149 |
| Exon 4 | 5ex4ICbio(5') | B-TCTCAGGATRGTCACATGGSC | 299 |
| | 3ex4ICbio(3') | B-CATCTCAGGGTGMRGGGCTT | 313 |

| | | | |
|---|---|---|---|
| B = biotine. | | | |

The PCR reaction cycle is composed of the following steps:
1 min 96 °C;
5 times (30 s at 96 °C; 50 s at 64 °C; 50 s at 72°C);
5 times (30 s at 96 °C; 50 s at 62 °C; 50 s at 72 °C);
10 times (30 s at 96 °C; 50 s at 60 °C; 50 s at 72 °C);
15 times (30 s at 96 °C; 50 s at 55 °C; 50 s at 72 °C);
5 min 72 °C;
4°C.

The PCR reaction is carried out in 50 mM Tris.HCl pH 9.2, 16 mM (NH₄)₂SO₄, 1.5 mM MgCl₂, 200µM dNTP's (dATP, dGTP, dCTP, dTTP), 2.5 U Taq polymerase (Perkin Elmer, Roche Molecular Systems, Branchburg, New Jersey, US), 20 pmol of each primer and 0.1-0.5 µg DNA.

The length of the obtained amplification products is verified on a 3% agarose gel according to Sambrook et al. (1989). 3 bands of different length are obtained relating respectively to HLA-C exon 2, exon 3 and exon 4. By use of this protocol, the HLA-C exons are amplified strong enough for subsequent typing in a Line Probe Assay.

### REFERENCES

Andersson G, Larhammar D, Widmark E, Servenius B, Peterson PA and Rask L (1987) Class II genes of the human major histocompatibility complex. Organization and evolutionary relationship of the DRbeta genes. J Biol Chem 262: 8748-8758.
Apple RJ and Erlich HA (1996) HLA classII genes: structure and diversity. Chapter 5 HLA and MHC: genes, molecules and function. BIOS Scientific Publishers Ltd., Oxford, UK.
Asseline U, Delarue M, Lancelot G, Toulme F and Thuong N (1984) Nucleic acid-binding molecules with high affinity and base sequence specificity: intercalating agents covalently linked to oligodeoxynucleotides. Proc Natl Acad Sci USA 81: 3297-3301.
Barany F (1991) The ligase chain reaction in a PCR world. PCR Methods Appl 1: 5-16.
Campbell RD and Trowsdale J (1993) Map of the human MHC. Immunology Today 14: 349-352.
Clay TM, Culpan D, Howell WM, Sage DA, Bradley BA and Bidwell JL (1994) UHG crossmatching. A comparison with PCR-SSO typing in the selection of HLA-DPB-compatible bone marrow donors. Transplantation 58: 200-207.
Compton J (1991) Nucleic acid sequence-based amplification. Nature 350: 91-92.
Duck P (1990) Probe amplifier system based on chimeric cycling oligonucleotides. Biotechniques 9: 142-147.
Fleischhauer K, Kernan NA, O'Reilly RJ, Dupont B and Yang SY (1990) Bone marrow-allograft rejection by T lymphocytes recognizing a single amino acid difference in HLA-B44. New Eng J Med 323: 1818-1822.
Guatelli J, Whitfield K, Kwoh D, Barringer K, Richman D and Gengeras T (1990) Isothermal, in vitro amplification of nucleic acids by a multienzyme reaction modeled after retroviral replication. Proc Natl Acad Sci USA 87: 1874-1878.
Hirschorn K, Bach F, Kolodny RL and Firschen IL (1963) Immune response and mitotis of human peripheral blood lymphocytes in vitro. Science 142: 1185-1187.
Kissmeyer NF, Svejgaard A and Hauge M (1969) The HLA system defined with lymphocytoyoxic and platelet antibodies in relation to kidney transplantation. Transplant Proc 1: 357-361.
Kwoh D, Davis G, Whitfield K, Chappelle H, Dimichele L and Gingeras T (1989) Transcription-based amplification system and detection of amplified human immunodeficiency virus type 1 with a bead-based sandwich hybridization format. Proc Natl Acad Sci USA 86: 1173-1177.
Kwok S, Kellogg DE, McKinney N, Spasic D, Goda L, Levenson C and Sninsky JJ (1990) Effects of primer-template mismatches on the polymerase chain reaction: human immunodeficiency virus type 1 model studies. Nucleic Acids Res. 18: 999-1005.
Landgren U, Kaiser R, Sanders J and Hood L (1988) A ligase-mediated gene detection technique. Science 241: 1077-1080.
Lomeli H, Tyagi S, Pritchard C, Lisardi P and Kramer F (1989) Quantitative assays based on the use of replicatable hybridization probes. Clin Chem 35: 1826-1831.
Matsukura M, Shinozuka K, Zon G, Mitsuya H, Reitz M, Cohen J and Broder S (1987) Phosphorothioate analogs of oligodeoxynucleotides: inhibitors of replication and cytopathic effects of human immunodeficiency virus. Proc Natl Acad Sci USA 84: 7706-7710.
Miller P, Yano J, Yano E, Carroll C, Jayaram K and Ts'o P (1979) Nonionic nucleic acid analogues. Synthesis and characterization of dideoxyribonucleoside methylphosphonates. Biochemistry 18: 5134-5143.
Mullis K, Faloona F, Scharf S, Saiki R, Horn G and Erlich H (1986) Specific enzymatic amplification of DNA in vitro: the polymerase chain reaction. Gold Spring Harb Symp Quant Biol 1: 263-273.
Mullis KB and Faloona FA (1987) Specific synthesis of DNA in vitro via a polymerase-catalyzed chain reaction. Methods Enzymol 155: 335-350.
Nielsen P, Egholm M, Berg R and Buchardt O (1991) Sequence-selective recognition of DNA by strand displacement with a thymine-substituted polyamide. Science 254: 1497-1500.
Nielsen P, Egholm M, Berg R and Buchardt O (1993) Sequence specific inhibition of DNA restriction enzyme cleavage by PNA. Nucleic-Acids-Res 21: 197-200.
Olerup O and Zetterquist H (1991) HLA-DRB1*01 subtyping by allele-specific PCR amplification: a sensitive, specific and rapid technique. Tissue Antigens 37: 197-204.
Saiki RK, Bugawan TL, Horn GT, Mullis KB and Erlich HA (1986) Analysis of enzymatically amplified beta-globin and HLA-DQ alpha DNA with allele-specific oligonucleotide probes. Nature 324: 163-166.
Saiki RK, Gelfland DH, Stoffel S, Scharf SJ, Higuchi R, Horn GT, Mullis KB and Erlich HA (1988) Primer-directed enzymatic amplification of DNA with a thermostable DNA polymerase. Science 239: 487-491.
Saiki RK, Walsh PS, Levenson CH and Erlich HA (1989) Genetic analysis of amplified DNA with immobilizes sequence-specific oligonucleotide probes. Proc Natl Acad Sci USA 86: 6230-6234.
Sambrook J, Fritsch E and Maniatis T (1989) Molecular cloning: a laboratory manual. Cold Spring Harbor Laboratory Press, Cold Spring Harbor, NY, USA.
Santamaria P, Boyce-Jacino MT, Lindstrom AL, Barbosa JJ, Faras AJ and Rich SS (1992) HLA class II "typing": direct sequencing of DRB, DQB and DQA genes. Hum Immunol 33: 69-81.
Santamaria P, Lindstrom AL, Boyce JM, Jacino MT, Myster SH, Barbosa JJ, Faras AJ and Rich SS (1993) HLA class I sequence-based typing. Hum Immunol 37: 39-50.
Sanchez-Pescador R, Stempien MS and Urdea MS (1988) Rapid chemiluminescent nucleic acid assays for detection of TEM-1 beta-lactamase-mediated penicillin resistance in Neisseria gonorrhoeae and other bacteria. J Clin Microbiol 26: 1934-1938.
Spencer WR and Parham P (1996) HLA class I genes: structure and diversity. Chapter 4. HLA and MHC: genes, molecules and function. BIOS Scientific Publishers Ltd., Oxford, UK.
Stuyver L, Rossau R, Wyseur A, Duhamel M, Vanderborght B, Van Heuverswyn H and Maertens G (1993) Typing of hepatitis C virus isolates and characterization of new subtypes using a line probe assay. J Gen Virol 74: 1093-1102.
Terasaki PH and McClelland JD (1964) Microdoplet assay of human serum cytotoxins. Nature 204: 998-1007.
Townsend A and Bodmer H (1989) Antigen recognition by Class I-restricted T lymphocytes. Annu Rev Immunol 7: 601-624.
Urdea MS, Horn T, Fultz TJ, Anderson M, Running JA, Hamren S, Ahle D and Chang CA (1991) Branched DNA amplification multimers for the sensitive, direct detection of human hepatitis viruses. Nucleic Acids Symp Ser 24:197-200.
Wu D and Wallace B (1989) The ligation amplification reaction (LAR) - amplification of specific DNA sequences using sequential rounds of template-dependent ligation. Genomics 4: 560-569.
Yang SY (1987) A standardised method for detection of HLA-A and HLA-B alleles by one-dimensional isoelectric focusing (IEF) gel electrophoresis. Immunobiology of HLA. Histocompatibility testing (ed. B Dupont). Springer-Verlag, New York, USA. pp. 332-335.
Yoshida M, Kimura A, Numano F and Sasazuki T (1992) Polymerase-chain-reaction-based analysis of polymorphism in the HLA-B gene. Hum Immunol 34: 257-266.

## Claims

1. Method for the typing or subtyping of one or more HLA-A alleles in a sample comprising the following steps:
(i) if needed, release, isolation and/or concentration of the nucleic acids present in said sample;
(ii) locus-specific, separate amplification of exon 2, exon 3 and exon 4 of HLA-A alleles, making use of at least three primer sets wherein
- for the amplification of exon 2, the reverse primer specifically hybridizes to a locus-specific target sequence in intron 2 of HLA-A,
- for the amplification of exon 3, the forward primer specifically hybridizes to a locus-specific target sequence in intron 2 of HLA-A and/or the reverse primer specifically hybridizes to a locus-specific target sequence in intron 3 of HLA-A, and
- for the amplification of exon 4, the forward primer specifically hybridizes to a locus-specific target sequence in intron 3 of HLA-A;
(iii) typing of the specific HLA-A allele present in said sample.

2. Method according to claim 1 further **characterized in that** the locus-specific target sequence is situated at position 67, 96, 109, 110, 118, 123, 131 or 181 of the HLA-A intron 2 (Fig. 1) and/or position 32, 50, 62, 73, 83, 86, 118, 130, 150, 501, 525, 561 or 571 of the HLA-A intron 3 (Fig. 4).

3. Method according to claim 2 further **characterized in that** said positions constitute the 3' end of the primer that is used for the amplification of exon 2, exon 3 or exon 4.

4. Method according to claim 3 further **characterized in that** the primer is chosen from the following list:
- for the amplification of exon 2 of HLA-A (table 1):
5'ATCTCGGACCCGGAGACTGT3' (SEQ ID NO 1)
5'GATCTCGGACCCGGAGACTGT3' (SEQ ID NO 2)
5'GGATCTCGGACCCGGAGACTGT3' (SEQ ID NO 3)
5'YGGATCTCGGACCCGGAGACTGT3' (SEQ ID NO 4)
5'GYGGATCTCGGACCCGGAGACTGT3' (SEQ ID NO 5)
5'GGYGGATCTCGGACCCGGAGACTGT3' (SEQ ID NO 6)
5'GGTCTCGGRGTCCCGCGGCT3' (SEQ ID NO 7)
5'GGGTCTCGGRGTCCCGCGGCT3' (SEQ ID NO 8)
5'AGGGTCTCGGRGTCCCGCGGCT3' (SEQ ID NO 9)
5'AAGGGTCTCGGRGTCCCGCGGCT3' (SEQ ID NO 10)
5'CAAGGGTCTCGGRGTCCCGCGGCT3' (SEQ ID NO 11)
5'CTCCCGGGDCAAGGGTCTCG3' (SEQ ID NO 12)
5'TCTCCCGGGDCAAGGGTCTCG3' (SEQ ID NO 13)
5'CTCTCCCGGGDCAAGGGTCTCG3' (SEQ ID NO 14)
5'CCTCTCCCGGGDCAAGGGTCTCG3' (SEQ ID NO 15)
5'GCCTCTCCCGGGDCAAGGGTCTCG3' (SEQ ID NO 16)
5'GGCCTCTCCCGGGDCAAGGGTCTCG3' (SEQ ID NO 17)
5'TCTCCCGGGDCAAGGGTCTC3' (SEQ ID NO 18)
5'CTCTCCCGGGDCAAGGGTCTC3' (SEQ ID NO 19)
5'CCTCTCCCGGGDCAAGGGTCTC3' (SEQ ID NO 20)
5'GCCTCTCCCGGGDCAAGGGTCTC3' (SEQ ID NO 21)
5'GGCCTCTCCCGGGDCAAGGGTCTC3' (SEQ ID NO 22)
5'GGGCCTCTCCCGGGDCAAGGGTCTC3' (SEQ ID NO 23)
5'CCTGGGCCTCTCCCGGGDCA3' (SEQ ID NO 30)
5'GCCTGGGCCTCTCCCGGGDCA3' (SEQ ID NO 31)
5'CGCCTGGGCCTCTCCCGGGDCA3' (SEQ ID NO 32)
5'GCGCCTGGGCCTCTCCCGGGDCA3' (SEQ ID NO 33)
5'GGCGCCTGGGCCTCTCCCGGGDCA3' (SEQ ID NO 34)
5'AGGCGCCTGGGCCTCTCCCGGGDCA3' (SEQ ID NO 35)
5'AGGCGCCTGGGCCTCTCCCG3' (SEQ ID NO 36)
5'AAGGCGCCTGGGCCTCTCCCG3' (SEQ ID NO 37)
5'WAAGGCGCCTGGGCCTCTCCCG3' (SEQ ID NO 38)
5'TWAAGGCGCCTGGGCCTCTCCCG3' (SEQ ID NO 39)
5'GTWAAGGCGCCTGGGCCTCTCCCG3' (SEQ ID NO 40)
5'GGTWAAGGCGCCTGGGCCTCTCCCG3' (SEQ ID NO 41)
5'CCGGGTWAAGGCGCCTGGGC3' (SEQ ID NO 42)
5'ACCGGGTWAAGGCGCCTGGGC3' (SEQ ID NO 43)
5'AACCGGGTWAAGGCGCCTGGGC3' (SEQ ID NO 44)
5'AAACCGGGTWAAGGCGCCTGGGC3' (SEQ ID NO 45)
5'GAAACCGGGTWAAGGCGCCTGGGC3' (SEQ ID NO 46)
5'TGAAACCGGGTWAAGGCGCCTGGGC3' (SEQ ID NO 47)
5'YCCVGCCCCGACCAACCYGG3' (SEQ ID NO 48)
5'GYCCVGCCCCGACCAACCYGG3' (SEQ ID NO 49)
5'YGYCCVGCCCCGACCAACCYGG3' (SEQ ID NO 50)
5'CYGYCCVGCCCCGACCAACCYGG3' (SEQ ID NO 51)
5'CCYGYCCVGCCCCGACCAACCYGG3' (SEQ ID NO 52)
5'CCCYGYCCVGCCCCGACCAACCYGG3' (SEQ ID NO 53)
- for the amplification of exon 3 of HLA-A (table 2; table 3):
5'CGGACGGGCCRGGTSRCCCA3' (SEQ ID NO 54)
5' ACGGACGGGCCRGGTSRCCCA3' (SEQ ID NO 55)
5'CACGGACGGGCCRGGTSRCCCA3' (SEQ ID NO 56)
5'CCACGGACGGGCCRGGTSRCCCA3' (SEQ ID NO 57)
5'CCCACGGACGGGCCRGGTSRCCCA3' (SEQ ID NO 58)
5'CCCCACGGACGGGCCRGGTSRCCCA3' (SEQ ID NO 59)
5'GGTCCGAGATCCRCCCCGAA3' (SEQ ID NO 60)
5'GGGTCCGAGATCCRCCCCGAA3' (SEQ ID NO 61)
5'CGGGTCCGAGATCCRCCCCGAA3' (SEQ ID NO 62)
5'CCGGGTCCGAGATCCRCCCCGAA3' (SEQ ID NO 63)
5'TCCGGGTCCGAGATCCRCCCCGAA3' (SEQ ID NO 64)
5'CTCCGGGTCCGAGATCCRCCCCGAA3' (SEQ ID NO 65)
5'CCCCGAAGCCGCGGGACYCC3' (SEQ ID NO 66)
5'RCCCCGAAGCCGCGGGACYCC3' (SEQ ID NO 67)
5'CRCCCCGAAGCCGCGGGACYCC3' (SEQ ID NO 68)
5'CCRCCCCGAAGCCGCGGGACYCC3' (SEQ ID NO 69)
5'TCCRCCCCGAAGCCGCGGGACYCC3' (SEQ ID NO 70)
5'ATCCRCCCCGAAGCCGCGGGACYCC3' (SEQ ID NO 71)
5'CCCGAAGCCGCGGGACYCCG3' (SEQ ID NO 72)
5'CCCCGAAGCCGCGGGACYCCG3' (SEQ ID NO 73)
5'RCCCCGAAGCCGCGGGACYCCG3' (SEQ ID NO 74)
5'CRCCCCGAAGCCGCGGGACYCCG3' (SEQ ID NO 75)
5'CCRCCCCGAAGCCGCGGGACYCCG3' (SEQ ID NO 76)
5'TCCRCCCCGAAGCCGCGGGACYCCG3' (SEQ ID NO 77)
5'CGCGGGACYCCGAGACCCTT3' (SEQ ID NO 84)
5'CCGCGGGACYCCGAGACCCTT3' (SEQ ID NO 85)
5'GCCGCGGGACYCCGAGACCCTT3' (SEQ ID NO 86)
5'AGCCGCGGGACYCCGAGACCCTT3' (SEQ ID NO 87)
5'AAGCCGCGGGACYCCGAGACCCTT3' (SEQ ID NO 88)
5'GAAGCCGCGGGACYCCGAGACCCTT3' (SEQ ID NO 89)
5'GACYCCGAGACCCTTGDCCC3' (SEQ ID NO 90)
5'GGACYCCGAGACCCTTGDCCC3' (SEQ ID NO 91)
5'GGGACYCCGAGACCCTTGDCCC3' (SEQ ID NO 92)
5'CGGGACYCCGAGACCCTTGDCCC3' (SEQ ID NO 93)
5'GCGGGACYCCGAGACCCTTGDCCC3' (SEQ ID NO 94)
5'CGCGGGACYCCGAGACCCTTGDCCC3' (SEQ ID NO 95)
5'GACCCTTGDCCCGGGAGAGG3' (SEQ ID NO 96)
5'AGACCCTTGDCCCGGGAGAGG3' (SEQ ID NO 97)
5'GAGACCCTTGDCCCGGGAGAGG3' (SEQ ID NO 98)
5'CGAGACCCTTGDCCCGGGAGAGG3' (SEQ ID NO 99)
5'CCGAGACCCTTGDCCCGGGAGAGG3' (SEQ ID NO 100)
5'YCCGAGACCCTTGDCCCGGGAGAGG3' (SEQ ID NO 101)
5'GTTTAGGCCAAAAATCCCCC3' (SEQ ID NO 102)
5'AGTTTAGGCCAAAAATCCCCC3' (SEQ ID NO 103)
5'CAGTTTAGGCCAAAAATCCCCC3' (SEQ ID NO 104)
5'TCAGTTTAGGCCAAAAATCCCCC3' (SEQ ID NO 105)
5'TTCAGTTTAGGCCAAAAATCCCCC3' (SEQ ID NO 106)
5'TTTCAGTTTAGGCCAAAAATCCCCC3' (SEQ ID NO 107)
5'AGCCCGGGAGATCTAYAGGC3' (SEQ ID NO 150)
5'CAGCCCGGGAGATCTAYAGGC3' (SEQ ID NO 151)
5'CCAGCCCGGGAGATCTAYAGGC3' (SEQ ID NO 152)
5'GCCAGCCCGGGAGATCTAYAGGC3' (SEQ ID NO 153)
5'GGCCAGCCCGGGAGATCTAYAGGC3' (SEQ ID NO 154)
5'AGGCCAGCCCGGGAGATCTAYAGGC3' (SEQ ID NO 155)
5'CCCTCCTTGTGGGAGGCCAG3' (SEQ ID NO 156)
5'CCCCTCCTTGTGGGAGGCCAG3' (SEQ ID NO 157)
5'TCCCCTCCTTGTGGGAGGCCAG3' (SEQ ID NO 158)
5'CTCCCCTCCTTGTGGGAGGCCAG3' (SEQ ID NO 159)
5'TCTCCCCTCCTTGTGGGAGGCCAG3' (SEQ ID NO 160)
5'GTCTCCCCTCCTTGTGGGAGGCCAG3' (SEQ ID NO 161)
5'CCCAAWTGTCTCCCCTCCTT3' (SEQ ID NO 162)
5'TCCCAAWTGTCTCCCCTCCTT3' (SEQ ID NO 163)
5'GTCCCAAWTGTCTCCCCTCCTT3' (SEQ ID NO 164)
5'GGTCCCAAWTGTCTCCCCTCCTT3' (SEQ ID NO 165)
5'TGGTCCCAAWTGTCTCCCCTCCTT3' (SEQ ID NO 166)
5'TTGGTCCCAAWTGTCTCCCCTCCTT3' (SEQ ID NO 167)
5'CTAGTGTTGGTCCCAAWTGT3' (SEQ ID NO 168)
5'TCTAGTGTTGGTCCCAAWTGT3' (SEQ ID NO 169)
5'TTCTAGTGTTGGTCCCAAWTGT3' (SEQ ID NO 170)
5'ATTCTAGTGTTGGTCCCAAWTGT3' (SEQ ID NO 171)
5'TATTCTAGTGTTGGTCCCAAWTGT3' (SEQ ID NO 172)
5' ATATTCTAGTGTTGGTCCCAAWTGT3' (SEQ ID NO 173)
5'GGGYGATATTCTAGTGTTGG3' (SEQ ID NO 174)
5'AGGGYGATATTCTAGTGTTGG3' (SEQ ID NO 175)
5'GAGGGYGATATTCTAGTGTTGG3' (SEQ ID NO 176)
5'GGAGGGYGATATTCTAGTGTTGG3' (SEQ ID NO 177)
5'GGGAGGGYGATATTCTAGTGTTGG3' (SEQ ID NO 178)
5'AGGGAGGGYGATATTCTAGTGTTGG3' (SEQ ID NO 179)
5'GGAGGGYGATATTCTAGTGT3' (SEQ ID NO 180)
5'GGGAGGGYGATATTCTAGTGT3' (SEQ ID NO 181)
5'AGGGAGGGYGATATTCTAGTGT3' (SEQ ID NO 182)
5'GAGGGAGGGYGATATTCTAGTGT3' (SEQ ID NO 183)
5' AGAGGGAGGGYGATATTCTAGTGT3' (SEQ ID NO 184)
5'CAGAGGGAGGGYGATATTCTAGTGT3' (SEQ ID NO 185)
5'CCCAGGAGGAKTCCTCTCCC3' (SEQ ID NO 186)
5'ACCCAGGAGGAKTCCTCTCCC3' (SEQ ID NO 187)
5'AACCCAGGAGGAKTCCTCTCCC3' (SEQ ID NO 188)
5'AAACCCAGGAGGAKTCCTCTCCC3' (SEQ ID NO 189)
5'GAAACCCAGGAGGAKTCCTCTCCC3' (SEQ ID NO 190)
5'GGAAACCCAGGAGGAKTCCTCTCCC3' (SEQ ID NO 191)
5'AGGATCTGGAAACCCAGGAG3' (SEQ ID NO 192)
5'CAGGATCTGGAAACCCAGGAG3' (SEQ ID NO 193)
5'ACAGGATCTGGAAACCCAGGAG3' (SEQ ID NO 194)
5'TACAGGATCTGGAAACCCAGGAG3' (SEQ ID NO 195)
5'GTACAGGATCTGGAAACCCAGGAG3' (SEQ ID NO 196)
5'GGTACAGGATCTGGAAACCCAGGAG3' (SEQ ID NO 197)
5'TCAGAGTCACTCTCTGGTAC3' (SEQ ID NO 198)
5'CTCAGAGTCACTCTCTGGTAC3' (SEQ ID NO 199)
5'CCTCAGAGTCACTCTCTGGTAC3' (SEQ ID NO 200)
5'ACCTCAGAGTCACTCTCTGGTAC3' (SEQ ID NO 201)
5'AACCTCAGAGTCACTCTCTGGTAC3' (SEQ ID NO 202)
5'GAACCTCAGAGTCACTCTCTGGTAC3' (SEQ ID NO 203)
- for the amplification of exon 4 of HLA-A (table 4):
5'TTCTGTGCTCYCTTCCCCAT3' (SEQ ID NO 204)
5'GTTCTGTGCTCYCTTCCCCAT3'(SEQ ID NO 205)
5'GGTTCTGTGCTCYCTTCCCCAT3' (SEQ ID NO 206)
5'GGGTTCTGTGCTCYCTTCCCCAT3' (SEQ ID NO 207)
5'TGGGTTCTGTGCTCYCTTCCCCAT3' (SEQ ID NO 208)
5'CTGGGTTCTGTGCTCYCTTCCCCAT3' (SEQ ID NO 209)
5'GGTGTCCTGTCCATTCTCAA3' (SEQ ID NO 24)
5'RGGTGTCCTGTCCATTCTCAA3' (SEQ ID NO 25)
5'CRGGTGTCCTGTCCATTCTCAA3' (SEQ ID NO 26)
5'CCRGGTGTCCTGTCCATTCTCAA3' (SEQ ID NO 27)
5'CCCRGGTGTCCTGTCCATTCTCAA3' (SEQ ID NO 28)
5'TCCCRGGTGTCCTGTCCATTCTCAA3' (SEQ ID NO 29)
5'CTGGWGGAGTGTCCCATKAC3' (SEQ ID NO 210)
5'GCTGGWGGAGTGTCCCATKAC3' (SEQ ID NO 211)
5'TGCTGGWGGAGTGTCCCATKAC3' (SEQ ID NO 212)
5'RTGCTGGWGGAGTGTCCCATKAC3' (SEQ ID NO 213)
5'YRTGCTGGWGGAGTGTCCCATKAC3' (SEQ ID NO 214)
5'GYRTGCTGGWGGAGTGTCCCATKAC3' (SEQ ID NO 215)
5'GTCCCATKACAGATRCMMAA3' (SEQ ID NO 216)
5'TGTCCCATKACAGATRCMMAA3' (SEQ ID NO 217)
5'GTGTCCCATKACAGATRCMMAA3' (SEQ ID NO 218)
5'AGTGTCCCATKACAGATRCMMAA3' (SEQ ID NO 219)
5'GAGTGTCCCATKACAGATRCMMAA3' (SEQ ID NO 220)
5'GGAGTGTCCCATKACAGATRCMMAA3' (SEQ ID NO 221)

5. Method according to any of claims 1 to 4 further **characterized in that** the amplification of exon 2 is carried out with the following forward primer:
• 5'TTCTCCCCAGACGCCGAGGATGGCC3' (SEQ ID NO 144).

6. Method according to any of claims 1 to 5 further **characterized in that** the amplification of exon 4 is carried out with the following reverse primer:
• 5'TTGGGCAGACCCTCATGCTGC3' (SEQ ID NO 311).

7. Method according to any of claims 1 to 6 further **characterized in that**:
- the amplification of exon 2 is carried out with the following primer set:
• 5'TTCTCCCCAGACGCCGAGGATGGCC3' (SEQ ID NO 144) and 5'ATCTCGGACCCGGAGACTGT3' (SEQ ID NO 1);
- the amplification of exon 3 is carried out with the following primer set:
• 5'CAGTTTAGGCCAAAAATCCCCC3' (SEQ ID NO 104) and 5'CCCTCCTTGTGGGAGGCCAG3' (SEQ ID NO 156);
- the amplification of exon 4 is carried out with the following primer set:
• 5'GTTCTGTGCTCYCTTCCCCAT3' (SEQ ID NO 205) and 5'TTGGGCAGACCCTCATGCTGC3' (SEQ ID NO 311).

8. Method according to any of claims 1 to 7 further **characterized in that** both exon 2 and exon 3 of HLA-A are amplified by use of a multiplex primer mix containing at least one primer pair for the amplification of exon 2 and at least one primer pair for the amplification of exon 3.

9. Method according to any of claims 1 to 8 further **characterized in that** all three exons, exon 2, exon 3 and exon 4, of HLA-A are amplified by use of a multiplex primer mix containing at least one primer pair for the amplification of exon 2, at least one primer pair for the amplification of exon 3 and at least one primer pair for the amplification of exon 4.

10. Method according to any of claims 1 to 9 further **characterized in that** the typing step is carried out by hybridization with one or more suitable probes.

11. A diagnostic kit for the typing or subtyping of one or more HLA-A alleles in a sample comprising the following components:
(i) when appropriate, a means for releasing, isolating or concentrating the nucleic acids present in said sample;
(ii) a primer mix containing at least
- one primer set for the amplification of exon 2 consisting of a combination of a forward and a reverse primer as defined in any of claims 1 to 7 for use in the amplification of exon 2 of HLA-A alleles,
- one primer set for the amplification of exon 3 consisting of a combination of a forward and a reverse primer as defined in any of claims 1 to 7 for use in the amplification of exon 3 of HLA-A alleles, and
- one primer set for the amplification of exon 4 consisting of a combination of a forward and a reverse primer as defined in any of claims 1 to 7 for use in the amplification of exon 4 of HLA-A alleles;
(iii) a means for the typing of the specific HLA-A allele present in said sample.

12. A line probe assay kit for the typing or subtyping of one or more HLA-A alleles in a sample comprising the following components:
(i) when appropriate, a means for releasing, isolating or concentrating the nucleic acids present in said sample;
(ii) a primer mix containing at least
- one primer set for the amplification of exon 2 consisting of a combination of a forward and a reverse primer as defined in any of claims 1 to 7 for use in the amplification of exon 2 of HLA-A alleles,
- one primer set for the amplification of exon 3 consisting of a combination of a forward and a reverse primer as defined in any of claims 1 to 7 for use in the amplification of exon 3 of HLA-A alleles, and
- one primer set for the amplification of exon 4 consisting of a combination of a forward and a reverse primer as defined in any of claims 1 to 7 for use in the amplification of exon 4 of HLA-A alleles;
(iii) at least one probe that specifically hybridizes with exon 2, exon 3 or exon 4 of HLA-A, fixed to a solid support;
(iv) a hybridization buffer, or components necessary for producing said buffer;
(v) a wash solution, or components necessary for producing said solution;
(vi) when appropriate, a means for detecting the hybrids resulting from the preceding hybridization.

## Patentansprüche

1. Methode zur Typisierung und Subtypisierung eines oder mehrerer HLA-A-Allele in einer Probe, mit den folgenden Schritten:
(i) nötigenfalls Freisetzung, Isolierung und/oder Aufkonzentrierung der in der Probe vorhandenen Nukleinsäuren;
(ii) Locus-spezifische, getrennte Amplifikation von Exon 2, Exon 3 und Exon 4 der HLA-A-Allele unter Verwendung von wenigstens drei Primersätzen, wobei
- zur Amplifikation von Exon 2 der Rückwärtsprimer spezifisch an eine Locus-spezifische Zielsequenz in Intron 2 von HLA-A hybridisiert,
- zur Amplifikation von Exon 3 der Vorwärtsprimer spezifisch an eine Locus-spezifische Zielsequenz in Intron 2 von HLA-A und/oder der Rückwärtsprimer spezifisch an eine Locus-spezifische Zielsequenz in Intron 3 von HLA-A hybridisiert und
- zur Amplifikation von Exon 4 der Vorwärtsprimer spezifisch an eine Locus-spezifische Zielsequenz in Intron 3 von HLA-A hybridisiert;
(iii) Typisierung des in der Probe vorhandenen spezifischen HLA-A-Allels.

2. Methode nach Anspruch 1, ferner **dadurch gekennzeichnet, dass** die Locus-spezifische Zielsequenz bei Position 67, 96, 109, 110, 118, 123, 131 oder 181 des HLA-A-Introns 2 (Fig. 1) und/oder Position 32, 50, 62, 73, 83, 86, 118, 130, 150, 501, 525, 561 oder 571 des HLA-A-Introns 3 (Fig. 4) liegt.

3. Methode nach Anspruch 2, ferner **dadurch gekennzeichnet, dass** die Positionen das 3'-Ende des Primers, der für die Amplifikation von Exon 2, Exon 3 oder Exon 4 verwendet wird, bilden.

4. Methode nach Anspruch 3, ferner **dadurch gekennzeichnet, dass** der Primer aus der folgenden Liste gewählt wird:
- für die Amplifikation von Exon 2 von HLA-A (Tabelle 1):
5'ATCTCGGACCCGGAGACTGT3' (SEQ ID NO 1)
5'GATCTCGGACCCGGAGACTGT3' (SEQ ID NO 2)
5'GGATCTCGGACCCGGAGACTGT3' (SEQ ID NO 3)
5'-YGGATCTCGGACCCGGAGACTGT3' (SEQ ID NO 4)
5'GYGGATCTCGGACCCGGAGACTGT3' (SEQ ID NO 5)
5'GGYGGATCTCGGACCCGGAGACTGT3' (SEQ ID NO 6)
5'GGTCTCGGRGTCCCGCGGCT3' (SEQ ID NO 7)
5'GGGTCTCGGRGTCCCGCGGCT3' (SEQ ID NO 8)
5'AGGGTCTCGGRGTCCCGCGGCT3' (SEQ ID NO 9)
5'AAGGGTCTCGGRGTCCCGCGGCT3' (SEQ ID NO 10)
5'CAAGGGTCTCGGRGTCCCGCGGCT3' (SEQ ID NO 11)
5'CTCCCGGGDCAAGGGTCTCG3' (SEQ ID NO 12)
5'TCTCCCGGGDCAAGGGTCTCG3' (SEQ ID NO 13)
5'CTCTCCCGGGDCAAGGGTCTCG3' (SEQ ID NO 14)
5'CCTCTCCCGGGDCAAGGGTCTCG3' (SEQ ID NO 15)
5'GCCTCTCCCGGGDCAAGGGTCTCG3' (SEQ ID NO 16)
5'GGCCTCTCCCGGGDCAAGGGTCTCG3' (SEQ ID NO 17)
5'TCTCCCGGGDCAAGGGTCTC3' (SEQ ID NO 18)
5'CTCTCCCGGGDCAAGGGTCTC3' (SEQ ID NO 19)
5'CCTCTCCCGGGDCAAGGGTCTC3' (SEQ ID NO 20)
5'GCCTCTCCCGGGDCAAGGGTCTC3' (SEQ ID NO 21)
5'GGCCTCTCCCGGGDCAAGGGTCTC3' (SEQ ID NO 22)
5'GGGCCTCTCCCGGGDCAAGGGTCTC3' (SEQ ID NO 23)
5'CCTGGGCCTCTCCCGGGDCA3' (SEQ ID NO 30)
5'GCCTGGGCCTCTCCCGGGDCA3' (SEQ ID NO 31)
5'CGCCTGGGCCTCTCCCGGGDCA3' (SEQ ID NO 32)
5'-GCGCCTGGGCCTCTCCCGGGDCA3' (SEQ ID NO 33)
5'GGCGCCTGGGCCTCTCCCGGGDCA3' (SEQ ID NO 34)
5'AGGCGCCTGGGCCTCTCCCGGGDCA3' (SEQ ID NO 35)
5'AGGCGCCTGGGCCTCTCCCG3' (SEQ ID NO 36)
5'AAGGCGCCTGGGCCTCTCCCG3' (SEQ ID NO 37)
5' WAAGGCGCCTGGGCCTCTCCCG3' (SEQ ID NO 38)
5'TWAAGGCGCCTGGGCCTCTCCCG3' (SEQ ID NO 39)
5'GTWAAGGCGCCTGGGCCTCTCCCG3' (SEQ ID NO 40)
5'GGTWAAGGCGCCTGGGCCTCTCCCG3' (SEQ ID NO 41)
5'CCGGGTWAAGGCGCCTGGGC3' (SEQ ID NO 42)
5'ACCGGGTWAAGGCGCCTGGGC3' (SEQ ID NO 43)
5'AACCGGGTWAAGGCGCCTGGGC3' (SEQ ID NO 44)
5'AAACCGGGTWAAGGCGCCTGGGC3' (SEQ ID NO 45)
5'GAAACCGGGTWAAGGCGCCTGGGC3' (SEQ ID NO 46)
5'TGAAACCGGGTWAAGGCGCCTGGGC3' (SEQ ID NO 47)
5'YCCVGCCCCGACCAACCYGG3' (SEQ ID NO 48)
5'GYCCVGCCCCGACCAACCYGG3' (SEQ ID NO 49)
5'YGYCCVGCCCCGACCAACCYGG3' (SEQ ID NO 50)
5'CYGYCCVGCCCCGACCAACCYGG3' (SEQ ID NO 51)
5'CCYGYCCVGCCCCGACCAACCYGG3' (SEQ ID NO 52)
5'CCCYGYCCVGCCCCGACCAACCYGG3' (SEQ ID NO 53)
- für die Amplifikation von Exon 3 von HLA-A (Tabelle 2; Tabelle 3):
5'CGGACGGGCCRGGTSRCCCA3' (SEQ ID NO 54)
5'ACGGACGGGCCRGGTSRCCCA3' (SEQ ID NO 55)
5'CACGGACGGGCCRGGTSRCCCA3' (SEQ ID NO 56)
5'CCACGGACGGGCCRGGTSRCCCA3' (SEQ ID NO 57)
5'CCCACGGACGGGCCRGGTSRCCCA3' (SEQ ID NO 58)
5'CCCCACGGACGGGCCRGGTSRCCCA3' (SEQ ID NO 59)
5'GGTCCGAGATCCRCCCCGAA3' (SEQ ID NO 60)
5'GGGTCCGAGATCCRCCCCGAA3' (SEQ ID NO 61)
5'CGGGTCCGAGATCCRCCCCGAA3' (SEQ ID NO 62)
5'CCGGGTCCGAGATCCRCCCCGAA3' (SEQ ID NO 63)
5'TCCGGGTCCGAGATCCRCCCCGAA3' (SEQ ID NO 64)
5'CTCCGGGTCCGAGATCCRCCCCGAA3' (SEQ ID NO 65)
5'CCCCGAAGCCGCGGGACYCC3' (SEQ ID NO 66)
5'RCCCCGAAGCCGCGGGACYCC3' (SEQ ID NO 67)
5'CRCCCCGAAGCCGCGGGACYCC3' (SEQ ID NO 68)
5'CCRCCCCGAAGCCGCGGGACYCC3' (SEQ ID NO 69)
5'TCCRCCCCGAAGCCGCGGGACYCC3' (SEQ ID NO 70)
5'ATCCRCCCCGAAGCCGCGGGACYCC3' (SEQ ID NO 71)
5'CCCGAAGCCGCGGGACYCCG3' (SEQ ID NO 72)
5'CCCCGAAGCCGCGGGACYCCG3' (SEQ ID NO 73)
5'RCCCCGAAGCCGCGGGACYCCG3' (SEQ ID NO 74)
5'CRCCCCGAAGCCGCGGGACYCCG3' (SEQ ID NO 75)
5'CCRCCCCGAAGCCGCGGGACYCCG3' (SEQ ID NO 76)
5'TCCRCCCCGAAGCCGCGGGACYCCG3' (SEQ ID NO 77)
5'CGCGGGACYCCGAGACCCTT3' (SEQ ID NO 84)
5'CCGCGGGACYCCGAGACCCTT3' (SEQ ID NO 85)
5'GCCGCGGGACYCCGAGACCCTT3' (SEQ ID NO 86)
5'AGCCGCGGGACYCCGAGACCCTT3' (SEQ ID NO 87)
5'AAGCCGCGGGACYCCGAGACCCTT3' (SEQ ID NO 88)
5'GAAGCCGCGGGACYCCGAGACCCTT3' (SEQ ID NO 89)
5'GACYCCGAGACCCTTGDCCC3' (SEQ ID NO 90)
5'GGACYCCGAGACCCTTGDCCC3' (SEQ ID NO 91)
5'GGGACYCCGAGACCCTTGDCCC3' (SEQ ID NO 92)
5'CGGGACYCCGAGACCCTTGDCCC3' (SEQ ID NO 93)
5'GCGGGACYCCGAGACCCTTGDCCC3' (SEQ ID NO 94)
5'CGCGGGACYCCGAGACCCTTGDCCC3' (SEQ ID NO 95)
5'GACCCTTGDCCCGGGAGAGG3' (SEQ ID NO 96)
5'AGACCCTTGDCCCGGGAGAGG3' (SEQ ID NO 97)
5'GAGACCCTTGDCCCGGGAGAGG3' (SEQ ID NO 98)
5'CGAGACCCTTGDCCCGGGAGAGG3' (SEQ ID NO 99)
5'CCGAGACCCTTGDCCCGGGAGAGG3' (SEQ ID NO 100)
5'YCCGAGACCCTTGDCCCGGGAGAGG3' (SEQ ID NO 101)
5'GTTTAGGCCAAAAATCCCCC3' (SEQ ID NO 102)
5'AGTTTAGGCCAAAAATCCCCC3' (SEQ ID NO 103)
5'CAGTTTAGGCCAAAAATCCCCC3' (SEQ ID NO 104)
5'TCAGTTTAGGCCAAAAATCCCCC3' (SEQ ID NO 105)
5'TTCAGTTTAGGCCAAAAATCCCCC3' (SEQ ID NO 106)
5'TTTCAGTTTAGGCCAAAAATCCCCC3' (SEQ ID NO 107)
5'AGCCCGGGAGATCTAYAGGC3' (SEQ ID NO 150)
5'CAGCCCGGGAGATCTAYAGGC3' (SEQ ID NO 151)
5'CCAGCCCGGGAGATCTAYAGGC3' (SEQ ID NO 152)
5'GCCAGCCCGGGAGATCTAYAGGC3' (SEQ ID NO 153)
5'GGCCAGCCCGGGAGATCTAYAGGC3' (SEQ ID NO 154)
5'AGGCCAGCCCGGGAGATCTAYAGGC3' (SEQ ID NO 155)
5'CCCTCCTTGTGGGAGGCCAG3' (SEQ ID NO 156)
5'CCCCTCCTTGTGGGAGGCCAG3' (SEQ ID NO 157)
5'TCCCCTCCTTGTGGGAGGCCAG3' (SEQ ID NO 158)
5'CTCCCCTCCTTGTGGGAGGCCAG3' (SEQ ID NO 159)
5'TCTCCCCTCCTTGTGGGAGGCCAG3' (SEQ ID NO 160)
5'GTCTCCCCTCCTTGTGGGAGGCCAG3' (SEQ ID NO 161)
5'CCCAAWTGTCTCCCCTCCTT3' (SEQ ID NO 162)
5'TCCCAAWTGTCTCCCCTCCTT3' (SEQ ID NO 163)
5'GTCCCAAWTGTCTCCCCTCCTT3' (SEQ ID NO 164)
5'GGTCCCAAWTGTCTCCCCTCCTT3' (SEQ ID NO 165)
5'TGGTCCCAAWTGTCTCCCCTCCTT3' (SEQ ID NO 166)
5'TTGGTCCCAAWTGTCTCCCCTCCTT3' (SEQ ID NO 167)
5'CTAGTGTTGGTCCCAAWTGT3' (SEQ ID NO 168)
5'TCTAGTGTTGGTCCCAAWTGT3' (SEQ ID NO 169)
5'TTCTAGTGTTGGTCCCAAWTGT3' (SEQ ID NO 170)
5'ATTCTAGTGTTGGTCCCAAWTGT3' (SEQ ID NO 171)
5'TATTCTAGTGTTGGTCCCAAWTGT3' (SEQ ID NO 172)
5'ATATTCTAGTGTTGGTCCCAAWTGT3' (SEQ ID NO 173)
5'GGGYGATATTCTAGTGTTGG3' (SEQ ID NO 174)
5'AGGGYGATATTCTAGTGTTGG3' (SEQ ID NO 175)
5'GAGGGYGATATTCTAGTGTTGG3' (SEQ ID NO 176)
5'GGAGGGYGATATTCTAGTGTTGG3' (SEQ ID NO 177)
5'GGGAGGGYGATATTCTAGTGTTGG3' (SEQ ID NO 178)
5'AGGGAGGGYGATATTCTAGTGTTGG3' (SEQ ID NO 179)
5'GGAGGGYGATATTCTAGTGT3' (SEQ ID NO 180)
5'GGGAGGGYGATATTCTAGTGT3' (SEQ ID NO 181)
5'AGGGAGGGYGATATTCTAGTGT3' (SEQ ID NO 182)
5'GAGGGAGGGYGATATTCTAGTGT3' (SEQ ID NO 183)
5'AGAGGGAGGGYGATATTCTAGTGT3' (SEQ ID NO 184)
5'CAGAGGGAGGGYGATATTCTAGTGT3' (SEQ ID NO 185)
5'CCCAGGAGGAKTCCTCTCCC3' (SEQ ID NO 186)
5'ACCCAGGAGGAKTCCTCTCCC3' (SEQ ID NO 187)
5'AACCCAGGAGGAKTCCTCTCCC3' (SEQ ID NO 188)
5'AAACCCAGGAGGAKTCCTCTCCC3' (SEQ ID NO 189)
5'GAAACCCAGGAGGAKTCCTCTCCC3' (SEQ ID NO 190)
5'GGAAACCCAGGAGGAKTCCTCTCCC3' SEQ ID NO 191)
5'AGGATCTGGAAACCCAGGAG3' (SEQ ID NO 192)
5'CAGGATCTGGAAACCCAGGAG3' (SEQ ID NO 193)
5'ACAGGATCTGGAAACCCAGGAG3' (SEQ ID NO 194)
5'TACAGGATCTGGAAACCCAGGAG3' (SEQ ID NO 195)
5'GTACAGGATCTGGAAACCCAGGAG3' (SEQ ID NO 196)
5'GGTACAGGATCTGGAAACCCAGGAG3' (SEQ ID NO 197)
5'TCAGAGTCACTCTCTGGTAC3' (SEQ ID NO 198)
5-CTCAGAGTCACTCTCTGGTAC3' (SEQ ID NO 199)
5'CCTCAGAGTCACTCTCTGGTAC3' (SEQ ID NO 200)
5'ACCTCAGAGTCACTCTCTGGTAC3' (SEQ ID NO 201)
5'AACCTCAGAGTCACTCTCTGGTAC3' (SEQ ID NO 202)
5'GAACCTCAGAGTCACTCTCTGGTAC3' (SEQ ID NO 203)
- für die Amplifikation von Exon 4 von HLA-A (Tabelle 4):
5'TTCTGTGCTCYCTTCCCCAT3' (SEQ ID NO 204)
5'GTTCTGTGCTCYCTTCCCCAT3'(SEQ ID NO 205)
5'GGTTCTGTGCTCYCTTCCCCAT3' (SEQ IN NO 206)
5'GGGTTCTGTGCTCYCTTCCCCAT3' (SEQ ID NO 207)
5'TGGGTTCTGTGCTCYCTTCCCCAT3' (SEQ ID NO 208)
5'CTGGGTTCTGTGCTCYCTTCCCCAT3' (SEQ ID NO 209)
5'GGTGTCCTGTCCATTCTCAA3' (SEQ ID NO 24)
5'RGGTGTCCTGTCCATTCTCAA3' (SEQ ID NO 25)
5'CRGGTGTCCTGTCCATTCTCAA3' (SEQ ID NO 26)
5'CCRGGTGTCCTGTCCATTCTCAA3' (SEQ ID NO 27)
5'CCCRGGTGTCCTGTCCATTCTCAA3' (SEQ ID NO 28)
5'TCCCRGGTGTCCTGTCCATTCTCAA3' (SEQ ID NO 29)
5'CTGGWGGAGTGTCCCATKAC3' (SEQ ID NO 210)
5'GCTGGWGGAGTGTCCCATKAC3' (SEQ ID NO 211)
5'TGCTGGWGGAGTGTCCCATKAC3' (SEQ ID NO 212)
5'RTGCTGGWGGAGTGTCCCATKAC3' (SEQ ID NO 213)
5'YRTGCTGGWGGAGTGTCCCATKAC3' (SEQ ID NO 214)
5'GYRTGCTGGWGGAGTGTCCCATKAC3' (SEQ ID NO 215)
5'GTCCCATKACAGATRCMMAA3' (SEQ ID NO 216)
5'TGTCCCATKACAGATRCMMAA3' (SEQ ID NO 217)
5'GTGTCCCATKACAGATRCMMAA3' (SEQ ID NO 218)
5'AGTGTCCCATKACAGATRCMMAA3' (SEQ ID NO 219)
5'GAGTGTCCCATKACAGATRCMMAA3' (SEQ ID NO 220)
5'GGAGTGTCCCATKACAGATRCMMAA3' (SEQ ID NO 221)

5. Methode nach einem der Ansprüche 1 bis 4, ferner **dadurch gekennzeichnet, dass** die Amplifikation von Exon 2 mit dem folgenden Vorwärtsprimer durchgeführt wird:
• 5'TTCTCCCCAGACGCCGAGGATGGCC3' (SEQ ID NO 144)

6. Methode nach einem der Ansprüche 1 bis 5, ferner **dadurch gekennzeichnet, dass** die Amplifikation von Exon 4 mit dem folgenden Rückwärtsprimer durchgeführt wird:
• 5'TTGGGCAGACCCTCATGCTGC3' (SEQ ID NO 311)

7. Methode nach einem der Ansprüche 1 bis 6, ferner **dadurch gekennzeichnet, dass**:
- die Amplifikation von Exon 2 mit dem folgenden Primersatz durchgeführt wird:
• 5'TTCTCCCCAGACGCCGAGGATGGCC3' (SEQ ID NO 144) und 5'ATCTCGGACCCGGAGACTGT3' (SEQ ID NO 1) ;
- die Amplifikation von Exon 3 mit dem folgenden Primersatz durchgeführt wird:
• 5'CAGTTTAGGCCAAAAATCCCCC3' (SEQ ID NO 104) und 5'CCCTCCTTGTGGGAGGCCAG3' (SEQ ID NO 156);
- die Amplifikation von Exon 4 mit dem folgenden Primersatz durchgeführt wird:
• 5'GTTCTGTGCTCYCTTCCCCAT3' (SEQ ID NO 205) und 5'TTGGGCAGACCCTCATGCTGC3' (SEQ ID NO 311).

8. Methode nach einem der Ansprüche 1 bis 7, ferner **dadurch gekennzeichnet, dass** sowohl Exon 2 als auch Exon 3 von HLA-A durch Verwendung eines Multiplex-Primer-Mix amplifiziert werden, der wenigstens ein Primerpaar für die Amplifikation von Exon 2 und wenigstens ein Primerpaar für die Amplifikation von Exon 3 enthält.

9. Methode nach einem der Ansprüche 1 bis 8, ferner **dadurch gekennzeichnet, dass** alle drei Exons von HLA-A, Exon 2, Exon 3 und Exon 4, durch Verwendung eines Multiplex-Primer-Mix amplifiziert werden, der wenigstens ein Primerpaar für die Amplifikation von Exon 2, wenigstens ein Primerpaar für die Amplifikation von Exon 3 und wenigstens ein Primerpaar für die Amplifikation von Exon 4 enthält.

10. Methode nach einem der Ansprüche 1 bis 9, ferner **dadurch gekennzeichnet, dass** der Typisierungsschritt durch Hybridisierung mit einer oder mehreren geeigneten Sonden durchgeführt wird.

11. Diagnostischer Kit zur Typisierung und Subtypisierung eines oder mehrerer HLA-A-Allele in einer Probe, mit den folgenden Komponenten:
(i) gegebenenfalls einem Mittel zur Freisetzung, Isolierung oder Aufkonzentrierung der in der Probe vorhandenen Nukleinsäuren;
(ii) einem Primer-Mix mit wenigstens
- einem Primersatz zur Amplifikation von Exon 2, bestehend aus einer Kombination eines Vorwärts- und eines Rückwärtsprimers mit der in einem der Ansprüche 1 bis 7 angegebenen Bedeutung, zur Verwendung bei der Amplifikation von Exon 2 von HLA-A-Allelen,
- einem Primersatz zur Amplifikation von Exon 3, bestehend aus einer Kombination eines Vorwärts- und eines Rückwärtsprimers mit der in einem der Ansprüche 1 bis 7 angegebenen Bedeutung, zur Verwendung bei der Amplifikation von Exon 3 von HLA-A-Allelen und
- einem Primersatz zur Amplifikation von Exon 4, bestehend aus einer Kombination eines Vorwärts- und eines Rückwärtsprimers mit der in einem der Ansprüche 1 bis 7 angegebenen Bedeutung, zur Verwendung bei der Amplifikation von Exon 4 von HLA-A-Allelen;
(iii) einem Mittel zur Typisierung des in der Probe vorhandenen spezifischen HLA-A-Allels.

12. Line-Probe-Assay-Kit zur Typisierung und Subtypisierung eines oder mehrerer HLA-A-Allele in einer Probe, mit den folgenden Komponenten:
(i) gegebenenfalls einem Mittel zur Freisetzung, Isolierung oder Aufkonzentrierung der in der Probe vorhandenen Nukleinsäuren;
(ii) einem Primer-Mix mit wenigstens
- einem Primersatz zur Amplifikation von Exon 2, bestehend aus einer Kombination eines Vorwärts- und eines Rückwärtsprimers mit der in einem der Ansprüche 1 bis 7 angegebenen Bedeutung, zur Verwendung bei der Amplifikation von Exon 2 von HLA-A-Allelen,
- einem Primersatz zur Amplifikation von Exon 3, bestehend aus einer Kombination eines Vorwärts- und eines Rückwärtsprimers mit der in einem der Ansprüche 1 bis 7 angegebenen Bedeutung, zur Verwendung bei der Amplifikation von Exon 3 von HLA-A-Allelen und
- einem Primersatz zur Amplifikation von Exon 4, bestehend aus einer Kombination eines Vorwärts- und eines Rückwärtsprimers mit der in einem der Ansprüche 1 bis 7 angegebenen Bedeutung, zur Verwendung bei der Amplifikation von Exon 4 von HLA-A-Allelen;
(iii) wenigstens einer Sonde, die spezifisch mit Exon 2, Exon 3 oder Exon 4 von HLA-A hybridisiert, fixiert an einem festen Träger;
(iv) einem Hybridisierungspuffer oder zur Herstellung des Puffers notwendigen Komponenten;
(v) einer Waschlösung oder zur Herstellung der Lösung notwendigen Komponenten;
(vi) gegebenenfalls einem Mittel zum Nachweisen der sich aus der vorhergehenden Hybridisierung ergebenden Hybride.

## Revendications

1. Procédé pour le typage ou le sous-typage d'un ou plusieurs allèles HLA-A dans un échantillon comprenant les étapes suivantes :
(i) si besoin, la libération, l'isolement et/ou la concentration des acides nucléiques présents dans ledit échantillon ;
(ii) l'amplification séparée, spécifique d'un locus, de l'exon 2, de l'exon 3 et de l'exon 4 d'allèles HLA-A, à l'aide d'au moins trois ensembles d'amorces dans lesquels
- pour l'amplification de l'exon 2, l'amorce antisens s'hybride spécifiquement à une séquence cible spécifique d'un locus dans l'intron 2 d'HLA-A,
- pour l'amplification de l'exon 3, l'amorce sens s'hybride spécifiquement à une séquence cible spécifique d'un locus dans l'intron 2 d'HLA-A et/ou l'amorce antisens s'hybride spécifiquement à une séquence cible spécifique d'un locus dans l'intron 3 d'HLA-A et
- pour l'amplification de l'exon 4, l'amorce sens s'hybride spécifiquement à une séquence cible spécifique d'un locus dans l'intron 3 d'HLA-A ;
(iii) le typage de l'allèle HLA-A spécifique présent dans ledit échantillon.

2. Procédé selon la revendication 1 **caractérisé en outre en ce que** la séquence cible spécifique d'un locus est située à la position 67, 96, 109, 110, 118, 123, 131 ou 181 de l'intron 2 d'HLA-A (Fig. 1) et/ou à la position 32, 50, 62, 73, 83, 86, 118, 130, 150, 501, 525, 561 ou 571 de l'intron 3 d'HLA-A (Fig. 4).

3. Procédé selon la revendication 2 **caractérisé en outre en ce que** lesdites positions constituent l'extrémité 3' de l'amorce qui est utilisée pour l'amplification de l'exon 2, l'exon 3 ou l'exon 4.

4. Procédé selon la revendication 3 **caractérisé en outre en ce que** l'amorce est choisie parmi la liste suivante :
- pour l'amplification de l'exon 2 d'HLA-A (tableau 1) :
5'ATCTCGGACCCGGAGACTGT3' (SEQ ID NO 1)
5'GATCTCGGACCCGGAGACTGT3' (SEQ ID NO 2)
5'GGATCTCGGACCCGGAGACTGT3' (SEQ ID NO 3)
5'YGGATCTCGGACCCGGAGACTGT3' (SEQ ID NO 4)
5'GYGGATCTCGGACCCGGAGACTGT3' (SEQ ID NO 5)
5'GGYGGATCTCGGACCCGGAGACTGT3' (SEQ ID NO 6)
5'GGTCTCGGRGTCCCGCGGCT3' (SEQ ID NO 7)
5'GGGTCTCGGRGTCCCGCGGCT3' (SEQ ID NO 8)
5'AGGGTCTCGGRGTCCCGCGGCT3' (SEQ ID NO 9)
5'AAGGGTCTCGGRGTCCCGCGGCT3' (SEQ ID NO 10)
5'CAAGGGTCTCGGRGTCCCGCGGCT3' (SEQ ID NO 11)
5'CTCCCGGGDCAAGGGTCTCG3' (SEQ ID NO 12)
5'TCTCCCGGGDCAAGGGTCTCG3' (SEQ 10 NO 13)
5'CTCTCCCGGGDCAAGGGTCTCG3' (SEQ ID NO 14)
5'CCTCTCCCGGGDCAAGGGTCTCG3' (SEQ ID NO 15)
5'GCCTCTCCCGGGDCAAGGGTCTCG3' (SEQ ID NO 16)
5'GGCCTCTCCCGGGDCAAGGGTCTCG3' (SEQ ID NO 17)
5'TCTCCCGGGDCAAGGGTCTC3' (SEQ ID NO 18)
5'CTCTCCCGGGDCAAGGGTCTC3' (SEQ 10 NO 19)
5'CCTCTCCCGGGDCAAGGGTCTC3' (SEQ ID NO 20)
5'GCCTCTCCCGGGDCAAGGGTCTC3' (SEQ ID NO 21)
5'GGCCTCTCCCGGGDCAAGGGTCTC3' (SEQ ID NO 22)
5'GGGCCTCTCCCGGGDCAAGGGTCTC3' (SEQ ID NO 23)
5'CCTGGGCCTCTCCCGGGDCA3' (SEQ ID NO 30)
5'GCCTGGGCCTCTCCCGGGDCA3' (SEQ ID NO 31)
5'CGCCTGGGCCTCTCCCGGGDCA3' (SEQ ID NO 32)
5'GCGCCTGGGCCTCTCCCGGGDCA3' (SEQ ID NO 33)
5'GGCGCCTGGGCCTCTCCCGGGDCA3' (SEQ ID NO 34)
5'AGGCGCCTGGGCCTCTCCCGGGDCA3' (SEQ ID NO 35)
5'AGGCGCCTGGGCCTCTCCCG3' (SEQ ID NO 36)
5'AAGGCGCCTGGGCCTCTCCCG3' (SEQ ID NO 37)
5'WAAGGCGCCTGGGCCTCTCCCG3' (SEQ ID NO 38)
5'TWAAGGCGCCTGGGCCTCTCCCG3' (SEQ ID NO 39)
5'GTWAAGGCGCCTGGGCCTCTCCCG3' (SEQ ID NO 40)
5'GGTWAAGGCGCCTGGGCCTCTCCCG3' (SEQ ID NO 41)
5'CCGGGTWAAGGCGCCTGGGC3' (SEQ ID NO 42)
5'ACCGGGTWAAGGCGCCTGGGC3' (SEQ ID NO 43)
5'AACCGGGTWAAGGCGCCTGGGC3' (SEQ ID NO 44)
5'AAACCGGGTWAAGGCGCCTGGGC3' (SEQ ID NO 45)
5'GAAACCGGGTWAAGGCGCCTGGGC3' (SEQ ID NO 46)
5'TGAAACCGGGTWAAGGCGCCTGGGC3' (SEQ ID NO 47)
5'YCCVGCCCCGACCAACCYGG3' (SEQ ID NO 48)
5'GYCCVGCCCCGACCAACCYGG3' (SEQ ID NO 49)
5'YGYCCVGCCCCGACCAACCYGG3' (SEQ ID NO 50)
5'CYGYCCVGCCCCGACCAACCYGG3' (SEQ ID NO 51)
5'CCYGYCCVGCCCCGACCAACCYGG3' (SEQ ID NO 52)
5'CCCYGYCCVGCCCCGACCAACCYGG3' (SEQ ID NO 53)
- pour l'amplification de l'exon 3 d'HLA-A (tableau 2 ; tableau 3) :
5'CGGACGGGCCRGGTSRCCCA3' (SEQ ID NO 54)
5'ACGGACGGGCCRGGTSRCCCA3' (SEQ ID NO 55)
5'CACGGACGGGCCRGGTSRCCCA3' (SEQ ID NO 56)
5'CCACGGACGGGCCRGGTSRCCCA3' (SEQ ID NO 57)
5'CCCACGGACGGGCCRGGTSRCCCA3' (SEQ ID NO 58)
5'CCCCACGGACGGGCCRGGTSRCCCA3' (SEQ ID NO 59)
5'GGTCCGAGATCCRCCCCGAA3' (SEQ ID NO 60)
5'GGGTCCGAGATCCRCCCCGAA3' (SEQ ID NO 61)
5'CGGGTCCGAGATCCRCCCCGAA3' (SEQ ID NO 62)
5'CCGGGTCCGAGATCCRCCCCGAA3' (SEQ ID NO 63)
5'TCCGGGTCCGAGATCCRCCCCGAA3' (SEQ ID NO 64)
5'CTCCGGGTCCGAGATCCRCCCCGAA3' (SEQ ID NO 65)
5'CCCCGAAGCCGCGGGACYCC3' (SEQ ID NO 66)
5'RCCCCGAAGCCGCGGGACYCC3' (SEQ ID NO 67)
5'CRCCCCGAAGCCGCGGGACYCC3' (SEQ ID NO 68)
5'CCRCCCCGAAGCCGCGGGACYCC3' (SEQ ID NO 69)
5'TCCRCCCCGAAGCCGCGGGACYCC3' (SEQ ID NO 70)
5'ATCCRCCCCGAAGCCGCGGGACYCC3' (SEQ ID NO 71)
5'CCCGAAGCCGCGGGACYCCG3' (SEQ ID NO 72)
5'CCCCGAAGCCGCGGGACYCCG3' (SEQ ID NO 73)
5'RCCCCGAAGCCGCGGGACYCCG3' (SEQ ID NO 74)
5'CRCCCCGAAGCCGCGGGACYCCG3' (SEQ ID NO 75)
5'CCRCCCCGAAGCCGCGGGACYCCG3' (SEQ ID NO 76)
5'TCCRCCCCGAAGCCGCGGGACYCCG3' (SEQ ID NO 77)
5'CGCGGGACYCCGAGACCCTT3' (SEQ ID NO 84)
5'CCGCGGGACYCCGAGACCCTT3' (SEQ ID NO 85)
5'GCCGCGGGACYCCGAGACCCTT3' (SEQ ID NO 86)
5'AGCCGCGGGACYCCGAGACCCTT3' (SEQ ID NO 87)
5'AAGCCGCGGGACYCCGAGACCCTT3' (SEQ ID NO 88)
5'GAAGCCGCGGGACYCCGAGACCCTT3' (SEQ ID NO 89)
5'GACYCCGAGACCCTTGDCCC3' (SEQ ID NO 90)
5'GGACYCCGAGACCCTTGDCCC3' (SEQ ID NO 91)
5'GGGACYCCGAGACCCTTGDCCC3' (SEQ ID NO 92)
5'CGGGACYCCGAGACCCTTGDCCC3' (SEQ ID NO 93)
5'GCGGGACYCCGAGACCCTTGDCCC3' (SEQ ID NO 94)
5'CGCGGGACYCCGAGACCCTTGDCCC3' (SEQ ID NO 95)
5'GACCCTTGDCCCGGGAGAGG3' (SEQ ID NO 96)
5'AGACCCTTGDCCCGGGAGAGG3' (SEQ ID NO 97)
5'GAGACCCTTGDCCCGGGAGAGG3' (SEQ ID NO 98)
5'CGAGACCCTTGDCCCGGGAGAGG3' (SEQ ID NO 99)
5'CCGAGACCCTTGDCCCGGGAGAGG3' (SEQ ID NO 100)
5'YCCGAGACCCTTGDCCCGGGAGAGG3' (SEQ ID NO 101)
5'GTTTAGGCCAAAAATCCCCC3' (SEQ ID NO 102)
5'AGTTTAGGCCAAAAATCCCCC3' (SEQ ID NO 103)
5'CAGTTTAGGCCAAAAATCCCCC3' (SEQ ID NO 104)
5'TCAGTTTAGGCCAAAAATCCCCC3' (SEQ ID NO 105)
5'TTCAGTTTAGGCCAAAAATCCCCC3' (SEQ ID NO 106)
5'TTTCAGTTTAGGCCAAAAATCCCCC3' (SEQ ID NO 107)
5'AGCCCGGGAGATCTAYAGGC3' (SEQ ID NO 150)
5'CAGCCCGGGAGATCTAYAGGC3' (SEQ ID NO 151)
5'CCAGCCCGGGAGATCTAYAGGC3' (SEQ ID NO 152)
5'GCCAGCCCGGGAGATCTAYAGGC3' (SEQ ID NO 153)
5'GGCCAGCCCGGGAGATCTAYAGGC3' (SEQ ID NO 154)
5'AGGCCAGCCCGGGAGATCTAYAGGC3' (SEQ ID NO 155)
5'CCCTCCTTGTGGGAGGCCAG3' (SEQ ID NO 156)
5'CCCCTCCTTGTGGGAGGCCAG3' (SEQ ID NO 157)
5'TCCCCTCCTTGTGGGAGGCCAG3' (SEQ 1D NO 158)
5'CTCCCCTCCTTGTGGGAGGCCAG3' (SEQ ID NO 159)
5'TCTCCCCTCCTTGTGGGAGGCCAG3' (SEQ ID NO 160)
5'GTCTCCCCTCCTTGTGGGAGGCCAG3' (SEQ ID NO 161)
5'CCCAAWTGTCTCCCCTCCTT3' (SEQ ID NO 162)
5'TCCCAAWTGTCTCCCCTCCTT3' (SEQ ID NO 163)
5'GTCCCAAWTGTCTCCCCTCCTT3' (SEQ ID NO 164)
5'GGTCCCAAWTGTCTCCCCTCCTT3' (SEQ ID NO 165)
5'TGGTCCCAAWTGTCTCCCCTCCTT3' (SEQ ID NO 166)
5'TTGGTCCCAAWTGTCTCCCCTCCTT3' (SEQ ID NO 167)
5'CTAGTGTTGGTCCCAAWTGT3' (SEQ 1D NO 168)
5'TCTAGTGTTGGTCCCAAWTGT3' (SEQ ID NO 169)
5'TTCTAGTGTTGGTCCCAAWTGT3' (SEQ ID NO 170)
5'ATTCTAGTGTTGGTCCCAAWTGT3' (SEQ ID NO 171)
5'TATTCTAGTGTTGGTCCCAAWTGT3' (SEQ ID NO 172)
5'ATATTCTAGTGTTGGTCCCAAWTGT3' (SEQ ID NO 173)
5'GGGYGATATTCTAGTGTTGG3' (SEQ ID NO 174)
5'AGGGYGATATTCTAGTGTTGG3' (SEQ ID NO 175)
5'GAGGGYGATATTCTAGTGTTGG3' (SEQ ID NO 176)
5'GGAGGGYGATATTCTAGTGTTGG3' (SEQ ID NO 177)
5'GGGAGGGYGATATTCTAGTGTTGG3' (SEQ ID NO 178)
5'AGGGAGGGYGATATTCTAGTGTTGG3' (SEQ ID NO 179)
5'GGAGGGYGATATTCTAGTGT3' (SEQ ID NO 180)
5'GGGAGGGYGATATTCTAGTGT3' (SEQ ID NO 181)
5'AGGGAGGGYGATATTCTAGTGT3' (SEQ ID NO 182)
5'GAGGGAGGGYGATATTCTAGTGT3' (SEQ ID NO 183)
5'AGAGGGAGGGYGATATTCTAGTGT3' (SEQ ID NO 184)
5'CAGAGGGAGGGYGATATTCTAGTGT3' (SEQ ID NO 185)
5'CCCAGGAGGAKTCCTCTCCC3' (SEQ ID NO 186)
5'ACCCAGGAGGAKTCCTCTCCC3' (SEQ ID NO 187)
5'AACCCAGGAGGAKTCCTCTCCC3' (SEQ ID NO 188)
5'AAACCCAGGAGGAKTCCTCTCCC3' (SEQ ID NO 189)
5'GAAACCCAGGAGGAKTCCTCTCCC3' (SEQ ID NO 190)
5'GGAAACCCAGGAGGAKTCCTCTCCC3' (SEQ ID NO 191)
5'AGGATCTGGAAACCCAGGAG3' (SEQ ID NO 192)
5'CAGGATCTGGAAACCCAGGAG3' (SEQ ID NO 193)
5'ACAGGATCTGGAAACCCAGGAG3' (SEQ ID NO 194)
5'TACAGGATCTGGAAACCCAGGAG3' (SEQ ID NO 195)
5'GTACAGGATCTGGAAACCCAGGAG3' (SEQ ID NO 196)
5'GGTACAGGATCTGGAAACCCAGGAG3' (SEQ ID NO 197)
5'TCAGAGTCACTCTCTGGTAC3' (SEQ ID NO 198)
5'CTCAGAGTCACTCTCTGGTAC3' (SEQ ID NO 199)
5'CCTCAGAGTCACTCTCTGGTAC3' (SEQ ID NO 200)
5'ACCTCAGAGTCACTCTCTGGTAC3' (SEQ ID NO 201)
5'AACCTCAGAGTCACTCTCTGGTAC3' (SEQ 1D NO 202)
5'GAACCTCAGAGTCACTCTCTGGTAC3' (SEQ ID NO 203)
- pour l'amplification de l'exon 4 d'HLA-A (tableau 4) :
5'TTCTGTGCTCYCTTCCCCAT3' (SEQ ID NO 204)
5'GTTCTGTGCTCYCTTCCCCAT3'(SEQ ID NO 205)
5'GGTTCTGTGCTCYCTTCCCCAT3' (SEQ ID NO 206)
5'GGGTTCTGTGCTCYCTTCCCCAT3' (SEQ ID NO 207)
5'TGGGTTCTGTGCTCYCTTCCCCAT3' (SEQ ID NO 208)
5'CTGGGTTCTGTGCTCYCTTCCCCAT3' (SEQ ID NO 209)
5'GGTGTCCTGTCCATTCTCAA3' (SEQ ID NO 24)
5'RGGTGTCCTGTCCATTCTCAA3' (SEQ ID NO 25)
5'CRGGTGTCCTGTCCATTCTCAA3' (SEQ ID NO 26)
5'CCRGGTGTCCTGTCCATTCTCAA3' (SEQ ID NO 27)
5'CCCRGGTGTCCTGTCCATTCTCAA3' (SEQ ID NO 28)
5'TCCCRGGTGTCCTGTCCATTCTCAA3' (SEQ ID NO 29)
5'CTGGWGGAGTGTCCCATKAC3' (SEQ ID NO 210)
5'GCTGGWGGAGTGTCCCATKAC3' (SEQ ID NO 211)
5'TGCTGGWGGAGTGTCCCATKAC3' (SEQ 10 NO 212)
5'RTGCTGGWGGAGTGTCCCATKAC3' (SEQ ID NO 213)
5'YRTGCTGGWGGAGTGTCCCATKAC3' (SEQ ID NO 214)
5'GYRTGCTGGWGGAGTGTCCCATKAC3' (SEQ ID NO 215)
5'GTCCCATKACAGATRCMMAA3' (SEQ ID NO 216)
5'TGTCCCATKACAGATRCMMAA3' (SEQ ID NO 217)
5'GTGTCCCATKACAGATRCMMAA3' (SEQ ID NO 218)
5'AGTGTCCCATKACAGATRCMMAA3' (SEQ ID NO 219)
5'GAGTGTCCCATKACAGATRCMMAA3' (SEQ ID NO 220)
5'GGAGTGTCCCATKACAGATRCMMAA3' (SEQ ID NO 221).

5. Procédé selon l'une quelconque des revendications 1 à 4 **caractérisé en outre en ce que** l'amplification de l'exon 2 est effectuée avec l'amorce sens suivante :
• 5'TTCTCCCCAGACGCCGAGGATGGCC3' (SEQ ID NO 144).

6. Procédé selon l'une quelconque des revendications 1 à 5 **caractérisé en outre en ce que** l'amplification de l'exon 4 est effectuée avec l'amorce antisens suivante :
• 5'TTGGGCAGACCCTCATGCTGC3' (SEQ ID NO 311).

7. Procédé selon l'une quelconque des revendications 1 à 6 **caractérisé en outre en ce que** :
- l'amplification de l'exon 2 est effectuée avec l'ensemble d'amorces suivant :
• 5'TTCTCCCCAGACGCCGAGGATGGCC3' (SEQ ID NO 144) et 5'ATCTCGGACCCGGAGACTGT3' (SEQ ID NO 1) ;
- l'amplification de l'exon 3 est effectuée avec l'ensemble d'amorces suivant :
• 5'CAGTTTAGGCCAAAAATCCCCC3' (SEQ ID NO 104) et 5'CCCTCCTTGTGGGAGGCCAG3' (SEQ ID NO 156) ;
- l'amplification de l'exon 4 est effectuée avec l'ensemble d'amorces suivant :
• 5'GTTCTGTGCTCYCTTCCCCAT3' (SEQ ID NO 205) et 5'TTGGGCAGACCCTCATGCTGC3' (SEQ ID NO 311).

8. Procédé selon l'une quelconque des revendications 1 à 7 **caractérisé en outre en ce que** l'exon 2 et l'exon 3 d'HLA-A sont tous deux amplifiés à l'aide d'un mélange en multiplex d'amorces contenant au moins une paire d'amorces pour l'amplification de l'exon 2 et au moins une paire d'amorces pour l'amplification de l'exon 3.

9. Procédé selon l'une quelconque des revendications 1 à 8 **caractérisé en outre en ce que** les exons, l'exon 2, l'exon 3 et l'exon 4, d'HLA-A sont tous trois amplifiés à l'aide d'un mélange en multiplex d'amorces contenant au moins une paire d'amorces pour l'amplification de l'exon 2, au moins une paire d'amorces pour l'amplification de l'exon 3 et au moins une paire d'amorces pour l'amplification de l'exon 4.

10. Procédé selon l'une quelconque des revendications 1 à 9 **caractérisé en outre en ce que** l'étape de typage est effectuée par hybridation avec une ou plusieurs sondes appropriées.

11. Kit de diagnostic pour le typage ou le sous-typage d'un ou plusieurs allèles HLA-A dans un échantillon comprenant les composants suivants :
(i) lorsque c'est approprié, un moyen pour la libération, l'isolement ou la concentration des acides nucléiques présents dans ledit échantillon ;
(ii) un mélange d'amorces comprenant au moins
- un ensemble d'amorces pour l'amplification de l'exon 2 constitué d'une combinaison d'une amorce sens et d'une amorce antisens telles que définies dans l'une quelconque des revendications 1 à 7 destinées à être utilisées dans l'amplification de l'exon 2 d'allèles HLA-A,
- un ensemble d'amorces pour l'amplification de l'exon 3 constitué d'une combinaison d'une amorce sens et d'une amorce antisens telles que définies dans l'une quelconque des revendications 1 à 7 destinées à être utilisées dans l'amplification de l'exon 3 d'allèles HLA-A et
- un ensemble d'amorces pour l'amplification de l'exon 4 constitué d'une combinaison d'une amorce sens et d'une amorce antisens telles que définies dans l'une quelconque des revendications 1 à 7 destinées à être utilisées dans l'amplification de l'exon 4 d'allèles HLA-A ;
(iii) un moyen pour le typage de l'allèle HLA-A spécifique présent dans ledit échantillon.

12. Kit d'essai en ligne avec sonde pour le typage ou le sous-typage d'un ou plusieurs allèles HLA-A dans un échantillon comprenant les composants suivants :
(i) lorsque c'est approprié, un moyen pour la libération, l'isolement ou la concentration des acides nucléiques présents dans ledit échantillon ;
(ii) un mélange d'amorces comprenant au moins
- un ensemble d'amorces pour l'amplification de l'exon 2 constitué d'une combinaison d'une amorce sens et d'une amorce antisens telles que définies dans l'une quelconque des revendications 1 à 7 destinées à être utilisées dans l'amplification de l'exon 2 d'allèles HLA-A,
- un ensemble d'amorces pour l'amplification de l'exon 3 constitué d'une combinaison d'une amorce sens et d'une amorce antisens telles que définies dans l'une quelconque des revendications 1 à 7 destinées à être utilisées dans l'amplification de l'exon 3 d'allèles HLA-A et
- un ensemble d'amorces pour l'amplification de l'exon 4 constitué d'une combinaison d'une amorce sens et d'une amorce antisens telles que définies dans l'une quelconque des revendications 1 à 7 destinées à être utilisées dans l'amplification de l'exon 4 d'allèles HLA-A ;
(iii) au moins une sonde qui s'hybride spécifiquement avec l'exon 2, l'exon 3 ou l'exon 4 d'HLA-A, fixée à un support solide ;
(iv) un tampon d'hybridation, ou des composants nécessaires pour la production dudit tampon ;
(v) une solution de lavage, ou des composants nécessaires pour la production de ladite solution ;
(vi) lorsque c'est approprié, un moyen pour la détection des hybrides résultants de l'hybridation précédente.
